(19) **Europäisches Patentamt / European Patent Office / Office européen des brevets**

(11) **EP 3 332 023 B1**

(12) ## EUROPEAN PATENT SPECIFICATION

(45) Date of publication and mention
of the grant of the patent:
**21.06.2023 Bulletin 2023/25**

(21) Application number: **15750348.3**

(22) Date of filing: **06.08.2015**

(51) International Patent Classification (IPC):
**C12Q 1/689** (2018.01)   **C12Q 1/6869** (2018.01)

(52) Cooperative Patent Classification (CPC):
(C-Sets available)
**C12Q 1/689; C12Q 1/6869;** C12Q 2600/106;
C12Q 2600/156          (Cont.)

(86) International application number:
**PCT/EP2015/068188**

(87) International publication number:
**WO 2017/020967 (09.02.2017 Gazette 2017/06)**

(54) **GENETIC TESTING FOR ALIGNMENT-FREE PREDICTING RESISTANCE OF MICROORGANISMS AGAINST ANTIMICROBIAL AGENTS**

GENETISCHER TEST ZUR AUSRICHTUNGSFREIEN VORHERSAGE DER RESISTENZ VON MIKROORGANISMEN GEGEN ANTIMIKROBIELLE MITTEL

TEST GÉNÉTIQUE POUR LA PRÉDICTION SANS ALIGNEMENT DE LA RÉSISTANCE MICRO-ORGANISMES CONTRE DES AGENTS ANTIMICROBIENS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**13.06.2018 Bulletin 2018/24**

(73) Proprietor: **Ares Genetics GmbH**
**1230 Vienna (AT)**

(72) Inventors:
• **KELLER, Andreas**
  **66346 Püttlingen (DE)**
• **SCHMOLKE, Susanne**
  **81447 München (DE)**
• **STÄHLER, Cord Friedrich**
  **69493 Hirschberg an der Bergstraße (DE)**
• **BACKES, Christina**
  **66125 Saarbrücken (DE)**
• **GALATA, Valentina**
  **66123 Saarbrücken (DE)**

(74) Representative: **Schnappauf, Georg**
**ZSP Patentanwälte PartG mbB**
**Hansastraße 32**
**80686 München (DE)**

(56) References cited:
**WO-A2-2012/027302    US-B1- 7 135 283**

• **BENJAMIN P. HOWDEN ET AL: "Evolution of Multidrug Resistance during Staphylococcus aureus Infection Involves Mutation of the Essential Two Component Regulator WalKR", PLOS PATHOGENS, vol. 7, no. 11, 10 November 2011 (2011-11-10), pages 1-15, XP055169475, DOI: 10.1371/journal.ppat.1002359**
• **YU GONGXIN: "GenHtr: a tool for comparative assessment of genetic heterogeneity in microbial genomes generated by massive short-read sequencing", BMC BIOINFORMATICS, BIOMED CENTRAL, LONDON, GB, vol. 11, no. 1, 12 October 2010 (2010-10-12), page 508, XP021071835, ISSN: 1471-2105, DOI: 10.1186/1471-2105-11-508**
• **B. P. HOWDEN ET AL: "Genomic Analysis Reveals a Point Mutation in the Two-Component Sensor Gene graS That Leads to Intermediate Vancomycin Resistance in Clinical Staphylococcus aureus", ANTIMICROBIAL AGENTS AND CHEMOTHERAPY, vol. 52, no. 10, 21 July 2008 (2008-07-21) , pages 3755-3762, XP055228904, US ISSN: 0066-4804, DOI: 10.1128/AAC.01613-07**

- A. D. KENNEDY ET AL: "Epidemic community-associated methicillin-resistant Staphylococcus aureus: Recent clonal expansion and diversification", PROCEEDINGS OF THE NATIONAL ACADEMY OF SCIENCES, vol. 105, no. 4, 29 January 2008 (2008-01-29), pages 1327-1332, XP055228900, US ISSN: 0027-8424, DOI: 10.1073/pnas.0710217105
- MICHAL WOZNIAK ET AL: "An approach to identifying drug resistance associated mutations in bacterial strains", BMC GENOMICS, BIOMED CENTRAL LTD, LONDON, UK, vol. 13, no. Suppl 7, 7 December 2012 (2012-12-07), page S23, XP021127984, ISSN: 1471-2164, DOI: 10.1186/1471-2164-13-S7-S23
- M.W. SYRMIS ET AL: "Comparison of a multiplexed MassARRAY system with real-time allele-specific PCR technology for genotyping of methicillin-resistant Staphylococcus aureus", CLINICAL MICROBIOLOGY AND INFECTION., vol. 17, no. 12, 1 December 2011 (2011-12-01), pages 1804-1810, XP055229274, United Kingdom, Switzerland ISSN: 1198-743X, DOI: 10.1111/j.1469-0691.2011.03521.x
- ARNOUD H. M. VAN VLIET ET AL: "Use of Alignment-Free Phylogenetics for Rapid Genome Sequence-Based Typing of Helicobacter pylori Virulence Markers and Antibiotic Susceptibility", JOURNAL OF CLINICAL MICROBIOLOGY, vol. 53, no. 9, 1 July 2015 (2015-07-01), pages 2877-2888, XP055588077, US ISSN: 0095-1137, DOI: 10.1128/JCM.01357-15
- Shea N. Gardner ET AL: "When Whole-Genome Alignments Just Won't Work: kSNP v2 Software for Alignment-Free SNP Discovery and Phylogenetics of Hundreds of Microbial Genomes", PLoS ONE, vol. 8, no. 12, 9 December 2013 (2013-12-09), page e81760, XP055588104, DOI: 10.1371/journal.pone.0081760
- Zamin Iqbal ET AL: "De novo assembly and genotyping of variants using colored de Bruijn graphs", NATURE GENETICS, vol. 44, no. 2, 1 February 2012 (2012-02-01), pages 226-232, XP055334060, NEW YORK, US ISSN: 1061-4036, DOI: 10.1038/ng.1028

Remarks:

The complete document including Reference Table(s) and the Sequence Listing(s) can be downloaded from the EPO website

(52) Cooperative Patent Classification (CPC): (Cont.)

C-Sets
C12Q 1/6869, C12Q 2535/122

**Description**

The present teaching relates to a method of determining an infection of a patient with at least one microorganism, particularly a bacterial microorganism, potentially resistant to antimicrobial drug treatment, a method of selecting a treatment of a patient suffering from an infection with at least one microorganism, particularly bacterial microorganism, and a method of determining an antimicrobial drug, e.g. antibiotic, resistance profile for at least one microorganism, particularly bacterial microorganism, as well as computer program products used in these methods.

Antibiotic resistance is a form of drug resistance whereby a sub-population of a microorganism, e.g. a strain of a bacterial species, can survive and multiply despite exposure to an antibiotic drug. It is a serious health concern for the individual patient as well as a major public health issue. Timely treatment of a bacterial infection requires the analysis of clinical isolates obtained from patients with regard to antibiotic resistance, in order to select an efficacious therapy. Generally, for this purpose an association of the identified resistance with a certain microorganism (i.e. ID) is necessary.

Antibacterial drug resistance (ADR) represents a major health burden. According to the World Health Organization's antimicrobial resistance global report on surveillance, ADR leads to 25,000 deaths per year in Europe and 23,000 deaths per year in the US. In Europe, 2.5 million extra hospital days lead to societal cost of 1.5 billion euro. In the US, the direct cost of 2 million illnesses leads to 20 billion dollar direct cost. The overall cost is estimated to be substantially higher, reducing the gross domestic product (GDP) by up to 1.6%.

Staphylococcus is a genus of Gram-positive, facultative anaerobe bacteria of the family Staphylococcaceae, which are spherical, immobile and form grape-like clusters. The genus includes at least 40 species.

Staphylococcus aureus is the most common species of staphylococcus to cause Staph infections. It is frequently found in the human respiratory tract and on the skin. Although Staphylococcus aureus is not always pathogenic, it is a common cause of skin infections (e.g. boils), respiratory disease (e.g. sinusitis), and food poisoning as well as life-threatening diseases such as pneumonia, meningitis, os-teomyelitis, endocarditis, toxic shock syndrome (TSS), bacteremia, and sepsis. Staphylococcus aureus can survive from hours to weeks, or even months, on dry environmental surfaces, depending on strain. The position of S. aureus as one of the most important opportunistic human pathogens is largely attributable to the combination of its virulence potential and its ubiquitous occurrence as a colonizer in humans, domestic animals, and livestock.

Staphylococcus aureus is the second most common overall cause of healthcare-associated infections reported to the National Healthcare Safety Network (NHSN). And current estimates suggest that 49-65% of healthcare-associated Staphylococcus aureus infections reported to NHSN are caused by methicillin-resistant Staphylococcus aureus (MRSA). MRSA is troublesome in hospitals, prisons, and nursing homes, where patients with open wounds, invasive devices, and weakened immune systems are at greater risk of nosocomial infection than the general public. MRSA began as a hospital-acquired infection, but has developed limited endemic status and is now sometimes community-acquired. Healthcare-associated MRSA (HA-MRSA) is related to prolonged length of hospital stay and is currently one of the most frequently identified pathogens in hospitals in many parts of the world. Furthermore, community acquired MRSA (CAMRSA) has demonstrated increasing trends, hence guidelines for prevention and surveillance have been issued by several healthcare officials.

In 2011 the CDC estimates 80,461 invasive MRSA infections and 11,285 related deaths occurred in the US. An unknown but much higher number of less severe infections occurred in both the community and in healthcare settings. MRSA is difficult to treat because of its resistance to most antibiotics. Treatment with vancomycin, a glycopeptide antibiotic often considered a last line of defense against MRSA, has led to the emergence of vancomycin-resistant Staphylococcus aureus (VRSA), against which few agents are effective. In addition, the use of teicoplanin, an antibiotic derived from vancomycin, has given rise to teicoplanin-resistant MRSA strains. There are other agents available to treat MRSA infection, though many have limited therapeutic benefit, primarily because of severe side effects.

In general the mechanisms for resistance of bacteria against antimicrobial treatments rely to a very substantial part on the organism's genetics. The respective genes or molecular mechanisms are either encoded in the genome of the bacteria or on plasmids that can be interchanged between different bacteria. The most common resistance mechanisms include:

1) Efflux pumps are high-affinity reverse transport systems located in the membrane that transports the antibiotic out of the cell, e.g. resistance to tetracycline.
2) Specific enzymes modify the antibiotic in a way that it loses its activity. In the case of streptomycin, the antibiotic is chemically modified so that it will no longer bind to the ribosome to block protein synthesis.
3) An enzyme is produced that degrades the antibiotic, thereby inactivating it. For example, the penicillinases are a group of beta-lactamase enzymes that cleave the beta lactam ring of the penicillin molecule.

In addition, some pathogens show natural resistance against drugs. For example, an organism can lack a transport system for an antibiotic or the target of the antibiotic molecule is not present in the organism.

Resistance to methicillin and related drugs is conferred by the mecA gene, which codes for an altered penicillin-binding protein (PBP2a or PBP2') that has a lower affinity for binding β-lactams (penicillins, cephalosporins, and carbapenems). This allows for resistance to all β-lactam antibiotics, and obviates their clinical use during MRSA infections. As such, the glycopeptide vancomycin is often deployed against MRSA.

Pathogens that are in principle susceptible to drugs can become resistant by modification of existing genetic material (e.g. spontaneous mutations for antibiotic resistance, happening in a frequency of one in about 100 mio bacteria in an infection) or the acquisition of new genetic material from another source. One example is horizontal gene transfer, a process where genetic material contained in small packets of DNA can be transferred between individual bacteria of the same species or even between different species. Horizontal gene transfer may happen by transduction, transformation or conjugation.

Generally, testing for susceptibility/resistance to antimicrobial agents is performed by culturing organisms in different concentration of these agents.

In brief, agar plates are inoculated with patient sample (e.g. urine, sputum, blood, stool) overnight. On the next day individual colonies are used for identification of organisms, either by culturing or using mass spectroscopy. Based on the identity of organisms new plates containing increasing concentration of drugs used for the treatment of these organisms are inoculated and grown for additional 12 - 24 hours. The lowest drug concentration which inhibits growth (minimal inhibitory concentration - MIC) is used to determine susceptibility/resistance for tested drugs. The process takes at least 2 to 3 working days during which the patient is treated empirically. Automated systems exist from several companies, e.g. Biomeriux (Vitek), Beckman Coulter (Microscan). A significant reduction of time-to-result is needed especially in patients with life-threatening disease and to overcome the widespread misuse of antibiotics.

Recent developments include PCR based test kits for fast bacterial identification (e.g. Biomerieux Biofire Tests, Curetis Unyvero Tests). With these test the detection of selected resistance loci is possible for a very limited number of drugs, but no correlation to culture based AST is given. Mass spectroscopy is increasingly used for identification of pathogens in clinical samples (e.g. Bruker Biotyper), and research is ongoing to establish methods for the detection of susceptibility/resistance against antibiotics.

The use of molecular techniques for direct detection of MRSA has become more commonplace especially for screening purposes. Resistance to methicillin is mediated via the mec operon which is part of the staphylococcal cassette chromosome mec (SCCmec). Recently PCR tests were introduced that are based on the detection of the right extremity sequence of the SCCmec in combination with S. aureus specific marker. Initial reports exist that describe culture based susceptibility reports despite detection of the presence of a resistance conferring gene.

For some drugs such it is known that at least two targets are addressed, e.g. in case of Ciprofloxacin (drug bank ID 00537; http://www.drugbank.ca/drugs/DB00537) targets include DNA Topoisomerase IV, DNA Topoisomerase II and DNA Gyrase. It can be expected that this is also the case for other drugs although the respective secondary targets have not been identified yet. In case of a common regulation, both relevant genetic sites would naturally show a co-correlation or redundancy.

It is known that drug resistance can be associated with genetic polymorphisms. This holds for viruses, where resistance testing is established clinical practice (e.g. HIV genotyping). More recently, it has been shown that resistance has also genetic causes in bacteria and even higher organisms, such as humans where tumors resistance against certain cytostatic agents can be linked to genomic mutations.

Wozniak et al. (BMC Genomics 2012, 13(Suppl 7):S23) disclose genetic determinants of drug resistance in Staphylococcus aureus based on genotype and phenotype data. Stoesser et al. disclose prediction of antimicrobial susceptibilities for Escherichia coli and Klebsiella pneumoniae isolates using whole genomic sequence data (J Antimicrob Chemother 2013; 68: 2234-2244).

Chewapreecha et al (Chewapreecha et al (2014) Comprehensive Identification of single nucleotid polymorphisms associated with beta-lactam resistance within pneumococcal mosaic genes. PLoS Genet 10(8): e1004547) used a comparable approach to identify mutations in gram-positive Streptococcus Pneumonia.

Gordon et al (Gordon et al (2014), Prediction of Staphylococcus aureus Antimicrobial Resistance by Whole-Genome Sequencing, J. Clin.Microbiol. 2014, 52(4):1182) and Dordel et al (Dordel, J., Kim, C. et al (2014). Novel Determinants of Antibiotic Resistance: Identification of Mutated Loci in Highly Methicillin-Resistant Subpopulations of Methicillin-Resistant Staphylococcus aureus. mBio, 5(2), e01000-13; doi:10.1128/mBio.01000-13) focused in their identification of new markers on already known genes.

The fast and accurate detection of infections with microorganisms, particularly microbial species, e.g. Staphylococcus aureus, and the prediction of response to anti-microbial therapy, particularly also without reference to known genes, represent a high unmet clinical need.

This need is addressed by the present invention.

Summary

The present inventors addressed this need by carrying out whole genome sequencing of a large cohort of microorganisms, particularly bacterial microorganisms, particularly Staphylococcus aureus clinical isolates, and comparing the genetic mutation profile to resistant phenotypes of isolates and/or classical culture based antimicrobial susceptibility testing with the goal to develop a test which can be used to detect bacterial susceptibility/resistance against antimicrobial drugs using molecular testing.

The inventors performed extensive studies on the genome of bacterial species, particularly Staphylococcus species, particularly Staphylococcus aureus, either susceptible or resistant to antimicrobial, e.g. antibiotic, drugs, particularly being susceptible or resistant to methicillin and related drugs. Based on this information, it is now possible to provide a detailed analysis on the resistance pattern of Staphylococcus, particularly Staphylococcus aureus, strains based on individual mutations on a nucleotide level. This analysis involves the identification of a resistance against individual antimicrobial, e.g. antibiotic, drugs as well as clusters of them. This allows not only for the determination of a resistance to a single antimicrobial, e.g. antibiotic, drug, but also to groups of antimicrobial drugs, e.g. antibiotics such as lactam or quinolone antibiotics, or even to all relevant antibiotic drugs.

Therefore, the present invention will considerably facilitate the selection of an appropriate antimicrobial, e.g. antibiotic, drug for the treatment of a microbial, e.g. Staphylococcus, particularly Staphylococcus aureus, infection in a patient and thus will largely improve the quality of diagnosis and treatment.

The present approach is based on the use of reference free SNP calling and association testing to cover the different sources of genetic resistance as well as the different ways of how bacteria can become resistant. This way the detection of resistances is not limited to reference genomes anymore

In contrast to other works (e.g. Gordon et al, Dordel et al, see above) the identification of new markers was not focused on already known genes, but a reference free SNP calling was performed, and the annotation is based on several reference genomes.

The present invention is defined by the appended claims. The present disclosure provides teachings which in some respects go beyond the disclosure of the invention as such, which is defined exclusively by the appended claims. The teachings are provided to place the actual invention in a broader technical context and to illustrate possible related technical developments. Such additional technical information which does not fall within the scope of the appended claims is not part of the invention. In particular, the terms "embodiment", "invention", and "aspect" are not to be construed as necessarily referring to the claimed invention, unless the subject-matter in question falls within the scope of the claims.

According to a first aspect, the present teaching relates to a method of determining an antimicrobial drug, e.g. antibiotic, resistance profile for a microorganism, particularly a bacterial microorganism, comprising:

> obtaining or providing a first data set of gene sequences of a plurality of clinical isolates of the microorganism;
> wherein at least a part of the gene sequences of the first data set are assembled;
> analyzing the gene sequences of the first data set for genetic variants to obtain a third data set of genetic variants;
> providing a second data set of antimicrobial drug, e.g. antibiotic, resistance and/or susceptibility of the plurality of clinical isolates of the microorganism;
> correlating the third data set with the second data set and statistically analyzing the correlation; and
> determining the genetic sites in the genome of the microorganism with antimicrobial drug, e.g. antibiotic, resistance.

In a second aspect the present teaching discloses a diagnostic method of determining an infection of a patient with a microorganism, particularly a bacterial microorganism potentially resistant to antimicrobial drug treatment, comprising the steps of:

> a) obtaining or providing a sample containing or suspected of containing a microorganism, particularly a bacterial microorganism, from the patient;
> b) determining the presence of at least one genetic variant in at least one position of the microorganism, particularly the bacterial microorganism, as determined by the method of the first aspect, wherein the presence of said at least one genetic variant is indicative of an infection with an antimicrobial drug resistant microorganism in said patient.

A third aspect of the present teaching relates to a method of selecting a treatment of a patient suffering from an infection with a potentially resistant microorganism, particularly bacterial microorganism, comprising the steps of:

> a) obtaining or providing a sample containing or suspected of containing a microorganism, particularly a bacterial microorganism, from the patient;
> b) determining the presence of at least one genetic variant in at least one position of the microorganism, particularly bacterial microorganism, as determined by the method of the first aspect, wherein the presence of said at least one genetic variant is indicative of a resistance to one or more antimicrobial drugs;
c) identifying said at least one or more antimicrobial drugs; and
d) selecting one or more antimicrobial drugs different from the ones identified in step c) and being suitable for the treatment of the infection with the microorganism, particularly the bacterial microorganism.

A fourth aspect of the present teaching relates to a method of acquiring, respectively determining, an antimicrobial drug, e.g. antibiotic, resistance profile for a clinical isolate of a microorganism, particularly a bacterial microorganism, comprising:

obtaining or providing at least one gene sequence of the clinical isolate of the microorganism, particularly the bacterial microorganism; and
determining the presence of genetic variants in the at least one gene sequence of the clinical isolate of the microorganism, particularly bacterial microorganism, as determined by the method of the first aspect of the present teaching.

Furthermore, a computer program product comprising computer executable instructions which, when executed, perform a method according to any one of the first to third aspects of the present teaching is disclosed in a fifth aspect of the present teaching.

In addition, a sixth aspect of the present teaching relates to a diagnostic method of determining an infection of a patient with a Staphylococcus species, particularly Staphylococcus aureus, potentially resistant to antimicrobial drug, e.g. antibiotic, treatment, comprising the steps of:

a) obtaining or providing a sample containing or suspected of containing at least one Staphylococcus species, particularly Staphylococcus aureus, from the patient;
b) determining the presence of at least one genetic variation in at least two positions from the group of positions annotated with Nos. 1 - 50 with regard to the reference genomes with the genome names given in Table 1 below, wherein the presence of said at least two genetic variations is indicative of an infection with an antimicrobial drug, e.g. antibiotic, resistant Staphylococcus, particularly Staphylococcus aureus, strain in said patient, wherein for some positions more than one position in different reference genomes is annotated.

Table 1: List of positions with Nos. 1 - 50

| No. | position | reference genome | genome name |
|---|---|---|---|
| 1 | 534953 F<br>543821 F | NC_017340.1<br>NC_010079.1 | 04_02981<br>USA300_TCH1516 |
| 2 | 210528 R<br>267448 R<br>269814 R | NC_022222.1<br>NC_017340.1<br>NC_010079.1 | 6850<br>04_02981<br>USA300_TCH1516 |
| 3 | 1362060 F | NC_017340.1 | 04_02981 |
| 4 | 1252703 R<br>1520285 F<br>1523326 F | NC_021670.1<br>NC_017351.1<br>NC_017340.1 | Bmb9393<br>11819_97<br>04_02981 |
| 5 | 1619285 R<br>1661238 R | NC_017340.1<br>NC_010079.1 | 04_02981<br>USA300_TCH1516 |
| 6 | 1641150 R | NC_017340.1 | 04_02981 |
| 7 | 170059 F<br>142263 F | NC_002953.3<br>NC_017337.1 | MSSA476<br>ED133 |
| 8 | 517571 F<br>554542 F | NC_017340.1<br>NC_018608.1 | 04_02981<br>08BA02176 |
| 9 | 978538 F | NC_017340.1 | 04_02981 |
| 10 | 1434811 R | NC_017340.1 | 04_02981 |

(continued)

| No. | position | reference genome | genome name |
|---|---|---|---|
| 11 | 953696 R<br>1010027 R | NC_022222.1<br>NC_010079.1 | 6850<br>USA300_TCH1516 |
| 12 | 208285 R<br>161011 R | NC_017340.1<br>NC_007795.1 | 04_02981<br>NCTC_8325 |
| 13 | 2179136 R<br>2149064 R<br>2107689 R | NC_017351.1<br>NC_017340.1<br>NC_018608.1 | 11819_97<br>04_02981<br>08BA02176 |
| 14 | 2358535 F | NC_017340.1 | 04_02981 |
| 15 | 2023012 R | NC_017340.1 | 04_02981 |
| 16 | 2777211 F<br>2779170 F | NC_007795.1<br>NC_017340.1 | NCTC_8325<br>04_02981 |
| 17 | 1801995 R<br>1790672 R | NC_018608.1<br>NC_017340.1 | 08BA02176<br>04_02981 |
| 18 | 976788 F<br>878040 F | NC_017340.1<br>NC_007795.1 | 04_02981<br>NCTC_8325 |
| 19 | 2101899 R<br>1972149 R | NC_021670.1<br>NC_017340.1 | Bmb9393<br>04_02981 |
| 20 | 1875550 F<br>2006001 F<br>1959494 F | NC_022222.1<br>NC_010079.1<br>NC_017340.1 | 6850<br>USA300_TCH1516<br>04_02981 |
| 21 | 705667 R | NC_017340.1 | 04_02981 |
| 22 | 2268723 F<br>2221448 F | NC_010079.1<br>NC_017340.1 | USA300_TCH1516<br>04_02981 |
| 23 | 1814108 R | NC_017340.1 | 04_02981 |
| 24 | 531649 R<br>531398 R | NC_017340.1<br>NC_017351.1 | 04_02981<br>11819_97 |
| 25 | 1754561 F<br>1691742 F | NC_017340.1<br>NC_007795.1 | 04_02981<br>NCTC_8325 |
| 26 | 1958403 R<br>2004910 R | NC_017340.1<br>NC_010079.1 | 04_02981<br>USA300_TCH1516 |
| 27 | 1242653 R<br>1294527 R<br>1299554 R | NC_022222.1<br>NC_010079.1<br>NC_017340.1 | 6850<br>USA300_TCH1516<br>04_02981 |
| 28 | 2590222 R<br>2637689 R | NC_017340.1<br>NC_010079.1 | 04_02981<br>USA300_TCH1516 |
| 29 | 1881161 R<br>1871101 R<br>1759861 R<br>1855493 R<br>1858794 R<br>1964828 R | NC_010079.1<br>NC_021059.1<br>NC_022222.1<br>NC_018608.1<br>NC_021554.1<br>NC_021670.1 | USA300_TCH1516<br>M1<br>6850<br>08BA02176<br>CC45<br>Bmb9393 |
| 30 | 1050123 R<br>1147277 R | NC_007795.1<br>NC_017340.1 | NCTC_8325<br>04_02981 |

(continued)

| No. | position | reference genome | genome name |
|-----|----------|-----------------|-------------|
| 31 | 2005634 F<br>2039052 F<br>2187801 F | NC_016912.1<br>NC_022222.1<br>NC_010079.1 | VC40<br>6850<br>USA300_TCH1516 |
| 32 | 350202 F<br>402479 F<br>352104 F | NC_022222.1<br>NC_017340.1<br>NC_007795.1 | 6850<br>04_02981<br>NCTC_8325 |
| 33 | 920768 F<br>956878 F<br>956978 F<br>858255 F | NC_021059.1<br>NC_018608.1<br>NC_017340.1<br>NC_007795.1 | M1<br>08BA02176<br>04_02981<br>NCTC_8325 |
| 34 | 1121847 R<br>1024692 R | NC_017340.1<br>NC_007795.1 | 04_02981<br>NCTC_8325 |
| 35 | 429303 F | NC_017340.1 | 04_02981 |
| 36 | 1812380 R<br>1775835 R<br>1714993 R | NC_010079.1<br>NC_017340.1<br>NC_007795.1 | USA300_TCH1516<br>04_02981<br>NCTC_8325 |
| 37 | 1928346 F | NC_017340.1 | 04_02981 |
| 38 | 1388095 R | NC_022226.1 | CN1 |
| 39 | 559072 R<br>504007 R | NC_017340.1<br>NC_007795.1 | 04_02981<br>NCTC_8325 |
| 40 | 2719339 R<br>2668764 R | NC_010079.1<br>NC_017340.1 | USA300_TCH1516<br>04_02981 |
| 41 | 1124668 F<br>1121585 F | NC_017340.1<br>NC_010079.1 | 04_02981<br>USA300_TCH1516 |
| 42 | 158073 F<br>193628 F<br>138357 F<br>196480 F<br>189192 F | NC_022226.1<br>NC_021059.1<br>NC_022222.1<br>NC_010079.1<br>NC_017340.1 | CN1<br>M1<br>6850<br>USA300_TCH1516<br>04_02981 |
| 43 | 1187805 F<br>1078815 F<br>1182930 F | NC_017340.1<br>NC_022113.1<br>NC_010079.1 | 04_02981<br>55_2053<br>USA300_TCH1516 |
| 44 | 1376396 R<br>1323236 R<br>1315892 R<br>1379143 R<br>1399306 F | NC_010079.1<br>NC_022222.1<br>NC_022226.1<br>NC_017340.1<br>NC_021670.1 | USA300_TCH1516<br>6850<br>CN1<br>04_02981<br>Bmb9393 |
| 45 | 2398505 R | NC_017340.1 | 04_02981 |
| 46 | 2753541 F<br>2803165 F | NC_017340.1<br>NC_010079.1 | 04_02981<br>USA300_TCH1516 |

(continued)

| No. | position | reference genome | genome name |
|---|---|---|---|
| 47 | 1415365 R | NC_017340.1 | 04_02981 |
| | 1428821 R | NC_018608.1 | 08BA02176 |
| | 1318646 R | NC_007795.1 | NCTC_8325 |
| | 1412563 R | NC_010079.1 | USA300_TCH1516 |
| | 1381147 R | NC_021059.1 | M1 |
| 48 | 1678734 R | NC_017340.1 | 04_02981 |
| | 1720315 R | NC_010079.1 | USA300_TCH1516 |
| 49 | 854815 R | NC_007795.1 | NCTC_8325 |
| | 953539 R | NC_017340.1 | 04_02981 |
| | 948900 R | NC_010079.1 | USA300_TCH1516 |
| 50 | 1675156 R | NC_017340.1 | 04_02981 |

A seventh aspect of the present teaching relates to a method of selecting a treatment of a patient suffering from an infection with a potentially resistant Staphylococcus, particularly Staphylococcus aureus, strain, comprising the steps of:

a) obtaining or providing a sample containing or suspected of containing at least one Staphylococcus, particularly Staphylococcus aureus, strain from the patient;

b) determining the presence of at least one genetic variation in at least two positions from the group of positions annotated with Nos. 1 - 50 with regard to the reference genomes with the genome names given in Table 1, wherein the presence of said at least two genetic variations is indicative of a resistance to one or more antimicrobial, e.g. antibiotic, drugs, wherein for some positions more than one position in different reference genomes is annotated;

c) identifying said at least one or more antimicrobial, e.g. antibiotic, drugs; and

d) selecting one or more antimicrobial, e.g. antibiotic, drugs different from the ones identified in step c) and being suitable for the treatment of a Staphylococcus, particularly Staphylococcus aureus, infection.

Further, an eighth aspect of the present teaching relates to a diagnostic method of determining an infection of a patient with a Staphylococcus species, particularly Staphylococcus aureus, potentially resistant to antimicrobial drug, e.g. antibiotic, treatment, comprising the steps of:

a) obtaining or providing a sample containing or suspected of containing at least one Staphylococcus, particularly Staphylococcus aureus, strain from the patient;

b) determining the presence of at least one genetic variation in at least two positions from the group of positions annotated with Nos. 1 - 50 with regard to the reference genomes with the genome names given in Tables 3a and/or 3b below, wherein the presence of said at least two genetic variations is indicative of an infection with an antimicrobial drug, e.g. antibiotic, resistant Staphylococcus, particularly Staphylococcus aureus, strain in said patient, wherein for some positions more than one position in different reference genomes is annotated.

Table 3a: List of positions with Nos. 1 - 50

| No. | position | reference genome | genome name |
|---|---|---|---|
| 1 | 1958403 R | NC_017340.1 | 04_02981 |
| | 2004910 R | NC_010079.1 | USA300_TCH1516 |
| 2 | 1641150 R | NC_017340.1 | 04_02981 |
| 3 | 978538 F | NC_017340.1 | 04_02981 |
| 4 | 705667 R | NC_017340.1 | 04_02981 |
| 5 | 1434811 R | NC_017340.1 | 04_02981 |

(continued)

| No. | position | reference genome | genome name |
|-----|----------|------------------|-------------|
| 6 | 953696 R<br>1010027 R | NC_022222.1<br>NC_010079.1 | 6850<br>USA300_TCH1516 |
| 7 | 2101899 R<br>1972149 R | NC_021670.1<br>NC_017340.1 | Bmb9393<br>04_02981 |
| 8 | 208285 R<br>161011 R | NC_017340.1<br>NC_007795.1 | 04_02981<br>NCTC_8325 |
| 9 | 2179136 R<br>2149064 R<br>2107689 R | NC_017351.1<br>NC_017340.1<br>NC_018608.1 | 11819_97<br>04_02981<br>08BA02176 |
| 10 | 2358535 F | NC_017340.1 | 04_02981 |
| 11 | 2023012 R | NC_017340.1 | 04_02981 |
| 12 | 2777211 F<br>2779170 F | NC_007795.1<br>NC_017340.1 | NCTC_8325<br>04_02981 |
| 13 | 1801995 R<br>1790672 R | NC_018608.1<br>NC_017340.1 | 08BA02176<br>04_02981 |
| 14 | 1754561 F<br>1691742 F | NC_017340.1<br>NC_007795.1 | 04_02981<br>NCTC_8325 |
| 15 | 1362060 F | NC_017340.1 | 04_02981 |
| 16 | 1242653 R<br>1294527 R<br>1299554 R | NC_022222.1<br>NC_010079.1<br>NC_017340.1 | 6850<br>USA300_TCH1516<br>04_02981 |
| 17 | 1252703 R<br>1520285 F<br>1523326 F | NC_021670.1<br>NC_017351.1<br>NC_017340.1 | Bmb9393<br>11819_97<br>04_02981 |
| 18 | 1619285 R<br>1661238 R | NC_017340.1<br>NC_010079.1 | 04_02981<br>USA300_TCH1516 |
| 19 | 1875550 F<br>2006001 F<br>1959494 F | NC_022222.1<br>NC_010079.1<br>NC_017340.1 | 6850<br>USA300_TCH1516<br>04_02981 |
| 20 | 976788 F<br>878040 F | NC_017340.1<br>NC_007795.1 | 04_02981<br>NCTC_8325 |
| 21 | 2590222 R<br>2637689 R | NC_017340.1<br>NC_010079.1 | 04_02981<br>USA300_TCH1516 |
| 22 | 210528 R<br>267448 R<br>269814 R | NC_022222.1<br>NC_017340.1<br>NC_010079.1 | 6850<br>04_02981<br>USA300_TCH1516 |
| 23 | 1814108 R | NC_017340.1 | 04_02981 |
| 24 | 170059 F<br>142263 F | NC_002953.3<br>NC_017337.1 | MSSA476<br>ED133 |
| 25 | 534953 F<br>543821 F | NC_017340.1<br>NC_010079.1 | 04_02981<br>USA300_TCH1516 |
| 26 | 517571 F<br>554542 F | NC_017340.1<br>NC_018608.1 | 04_02981<br>08BA02176 |

(continued)

| No. | position | reference genome | genome name |
|---|---|---|---|
| 27 | 531649 R<br>531398 R | NC_017340.1<br>NC_017351.1 | 04_02981<br>11819_97 |
| 28 | 1050123 R<br>1147277 R | NC_007795.1<br>NC_017340.1 | NCTC_8325<br>04_02981 |
| 29 | 1881161 R<br>1871101 R<br>1759861 R<br>1855493 R<br>1858794 R<br>1964828 R | NC_010079.1<br>NC_021059.1<br>NC_022222.1<br>NC_018608.1<br>NC_021554.1<br>NC_021670.1 | USA300_TCH1516<br>M1<br>6850<br>08BA02176<br>CC45<br>Bmb9393 |
| 30 | 2268723 F<br>2221448 F | NC_010079.1<br>NC_017340.1 | USA300_TCH1516<br>04_02981 |
| 31 | 920768 F<br>956878 F<br>956978 F<br>858255 F | NC_021059.1<br>NC_018608.1<br>NC_017340.1<br>NC_007795.1 | M1<br>08BA02176<br>04_02981<br>NCTC_8325 |
| 32 | 2005634 F<br>2039052 F<br>2187801 F | NC_016912.1<br>NC_022222.1<br>NC_010079.1 | VC40<br>6850<br>USA300_TCH1516 |
| 33 | 429303 F | NC_017340.1 | 04_02981 |
| 34 | 350202 F<br>402479 F<br>352104 F | NC_022222.1<br>NC_017340.1<br>NC_007795.1 | 6850<br>04_02981<br>NCTC_8325 |
| 35 | 158073 F<br>193628 F<br>138357 F<br>196480 F<br>189192 F | NC_022226.1<br>NC_021059.1<br>NC_022222.1<br>NC_010079.1<br>NC_017340.1 | CN1<br>M1<br>6850<br>USA300_TCH1516<br>04_02981 |
| 36 | 1121847 R<br>1024692 R | NC_017340.1<br>NC_007795.1 | 04_02981<br>NCTC_8325 |
| 37 | 2719339 R<br>2668764 R | NC_010079.1<br>NC_017340.1 | USA300_TCH1516<br>04_02981 |
| 38 | 1388095 R | NC_022226.1 | CN1 |
| 39 | 1415365 R<br>1428821 R<br>1318646 R<br>1412563 R<br>1381147 R | NC_017340.1<br>NC_018608.1<br>NC_007795.1<br>NC_010079.1<br>NC_021059.1 | 04_02981<br>08BA02176<br>NCTC_8325<br>USA300_TCH1516<br>M1 |
| 40 | 1678734 R<br>1720315 R | NC_017340.1<br>NC_010079.1 | 04_02981<br>USA300_TCH1516 |
| 41 | 1928346 F | NC_017340.1 | 04_02981 |

(continued)

| No. | position | reference genome | genome name |
|---|---|---|---|
| 42 | 1376396 R | NC_010079.1 | USA300_TCH1516 |
|  | 1323236 R | NC_022222.1 | 6850 |
|  | 1315892 R | NC_022226.1 | CN1 |
|  | 1379143 R | NC_017340.1 | 04_02981 |
|  | 1399306 F | NC_021670.1 | Bmb9393 |
| 43 | 1338943 R | NC_017340.1 | 04_02981 |
| 44 | 1124668 F | NC_017340.1 | 04_02981 |
|  | 1121585 F | NC_010079.1 | USA300_TCH1516 |
| 45 | 559072 R | NC_017340.1 | 04_02981 |
|  | 504007 R | NC_007795.1 | NCTC_8325 |
| 46 | 1675156 R | NC_017340.1 | 04_02981 |
| 47 | 1187805 F | NC_017340.1 | 04_02981 |
|  | 1078815 F | NC_022113.1 | 55_2053 |
|  | 1182930 F | NC_010079.1 | USA300_TCH1516 |
| 48 | 1356138 F | NC_017340.1 | 04_02981 |
| 49 | 854815 R | NC_007795.1 | NCTC_8325 |
|  | 953539 R | NC_017340.1 | 04_02981 |
|  | 948900 R | NC_010079.1 | USA300_TCH1516 |
| 50 | 2459738 F | NC_017340.1 | 04_02981 |
|  | 2364478 F | NC_022222.1 | 6850 |

Table 3b: List of positions with Nos. 1 - 50

| No. | position | reference genome | genome name |
|---|---|---|---|
| 1 | 1958403 R | NC_017340.1 | 04_02981 |
|  | 2004910 R | NC_010079.1 | USA300_TCH1516 |
| 2 | 1641150 R | NC_017340.1 | 04_02981 |
| 3 | 978538 F | NC_017340.1 | 04_02981 |
| 4 | 705667 R | NC_017340.1 | 04_02981 |
| 5 | 1434811 R | NC_017340.1 | 04_02981 |
| 6 | 953696 R | NC_022222.1 | 6850 |
|  | 1010027 R | NC_010079.1 | USA300_TCH1516 |
| 7 | 2101899 R | NC_021670.1 | Bmb9393 |
|  | 1972149 R | NC_017340.1 | 04_02981 |
| 8 | 208285 R | NC_017340.1 | 04_02981 |
|  | 161011 R | NC_007795.1 | NCTC_8325 |
| 9 | 2179136 R | NC_017351.1 | 11819_97 |
|  | 2149064 R | NC_017340.1 | 04_02981 |
|  | 2107689 R | NC_018608.1 | 08BA02176 |
| 10 | 2358535 F | NC_017340.1 | 04_02981 |
| 11 | 2023012 R | NC_017340.1 | 04_02981 |

(continued)

| No. | position | reference genome | genome name |
|---|---|---|---|
| 12 | 2777211 F<br>2779170 F | NC_007795.1<br>NC_017340.1 | NCTC_8325<br>04_02981 |
| 13 | 1801995 R<br>1790672 R | NC_018608.1<br>NC_017340.1 | 08BA02176<br>04_02981 |
| 14 | 1754561 F<br>1691742 F | NC_017340.1<br>NC_007795.1 | 04_02981<br>NCTC_8325 |
| 15 | 1362060 F | NC_017340.1 | 04_02981 |
| 16 | 1242653 R<br>1294527 R<br>1299554 R | NC_022222.1<br>NC_010079.1<br>NC_017340.1 | 6850<br>USA300_TCH1516<br>04_02981 |
| 17 | 1252703 R<br>1520285 F<br>1523326 F | NC_021670.1<br>NC_017351.1<br>NC_017340.1 | Bmb9393<br>11819_97<br>04_02981 |
| 18 | 1619285 R<br>1661238 R | NC_017340.1<br>NC_010079.1 | 04_02981<br>USA300_TCH1516 |
| 19 | 1875550 F<br>2006001 F<br>1959494 F | NC_022222.1<br>NC_010079.1<br>NC_017340.1 | 6850<br>USA300_TCH1516<br>04_02981 |
| 20 | 976788 F<br>878040 F | NC_017340.1<br>NC_007795.1 | 04_02981<br>NCTC_8325 |
| 21 | 2590222 R<br>2637689 R | NC_017340.1<br>NC_010079.1 | 04_02981<br>USA300_TCH1516 |
| 22 | 210528 R<br>267448 R<br>269814 R | NC_022222.1<br>NC_017340.1<br>NC_010079.1 | 6850<br>04_02981<br>USA300_TCH1516 |
| 23 | 1814108 R | NC_017340.1 | 04_02981 |
| 24 | 170059 F<br>142263 F | NC_002953.3<br>NC_017337.1 | MSSA476<br>ED133 |
| 25 | 534953 F<br>543821 F | NC_017340.1<br>NC_010079.1 | 04_02981<br>USA300_TCH1516 |
| 26 | 517571 F<br>554542 F | NC_017340.1<br>NC_018608.1 | 04_02981<br>08BA02176 |
| 27 | 531649 R<br>531398 R | NC_017340.1<br>NC_017351.1 | 04_02981<br>11819_97 |
| 28 | 1050123 R<br>1147277 R | NC_007795.1<br>NC_017340.1 | NCTC_8325<br>04_02981 |
| 29 | 1881161 R<br>1871101 R<br>1759861 R<br>1855493 R<br>1858794 R<br>1964828 R | NC_010079.1<br>NC_021059.1<br>NC_022222.1<br>NC_018608.1<br>NC_021554.1<br>NC_021670.1 | USA300_TCH1516<br>M1<br>6850<br>08BA02176<br>CC45<br>Bmb9393 |
| 30 | 2268723 F<br>2221448 F | NC_010079.1<br>NC_017340.1 | USA300_TCH1516<br>04_02981 |

(continued)

| No. | position | reference genome | genome name |
|---|---|---|---|
| 31 | 920768 F<br>956878 F<br>956978 F<br>858255 F | NC_021059.1<br>NC_018608.1<br>NC_017340.1<br>NC_007795.1 | M1<br>08BA02176<br>04_02981<br>NCTC_8325 |
| 32 | 2005634 F<br>2039052 F<br>2187801 F | NC_016912.1<br>NC_022222.1<br>NC_010079.1 | VC40<br>6850<br>USA300_TCH1516 |
| 33 | 429303 F | NC_017340.1 | 04_02981 |
| 34 | 350202 F<br>402479 F<br>352104 F | NC_022222.1<br>NC_017340.1<br>NC_007795.1 | 6850<br>04_02981<br>NCTC_8325 |
| 35 | 158073 F<br>193628 F<br>138357 F<br>196480 F<br>189192 F | NC_022226.1<br>NC_021059.1<br>NC_022222.1<br>NC_010079.1<br>NC_017340.1 | CN1<br>M1<br>6850<br>USA300_TCH1516<br>04_02981 |
| 36 | 1121847 R<br>1024692 R | NC_017340.1<br>NC_007795.1 | 04_02981<br>NCTC_8325 |
| 37 | 2719339 R<br>2668764 R | NC_010079.1<br>NC_017340.1 | USA300_TCH1516<br>04_02981 |
| 38 | 1388095 R | NC_022226.1 | CN1 |
| 39 | 1415365 R<br>1428821 R<br>1318646 R<br>1412563 R | NC_017340.1<br>NC_018608.1<br>NC_007795.1<br>NC_010079.1 | 04_02981<br>08BA02176<br>NCTC_8325<br>USA300_TCH1516 |
|  | 1381147 R | NC_021059.1 | M1 |
| 40 | 1678734 R<br>1720315 R | NC_017340.1<br>NC_010079.1 | 04_02981<br>USA300_TCH1516 |
| 41 | 1928346 F | NC_017340.1 | 04_02981 |
| 42 | 1376396 R<br>1323236 R<br>1315892 R<br>1379143 R<br>1399306 F | NC_010079.1<br>NC_022222.1<br>NC_022226.1<br>NC_017340.1<br>NC_021670.1 | USA300_TCH1516<br>6850<br>CN1<br>04_02981<br>Bmb9393 |
| 43 | 1124668 F<br>1121585 F | NC_017340.1<br>NC_010079.1 | 04_02981<br>USA300_TCH1516 |
| 44 | 559072 R<br>504007 R | NC_017340.1<br>NC_007795.1 | 04_02981<br>NCTC_8325 |
| 45 | 1675156 R | NC_017340.1 | 04_02981 |
| 46 | 1187805 F<br>1078815 F<br>1182930 F | NC_017340.1<br>NC_022113.1<br>NC_010079.1 | 04_02981<br>55_2053<br>USA300_TCH1516 |
| 47 | 1356138 F | NC_017340.1 | 04_02981 |

(continued)

| No. | position | reference genome | genome name |
|---|---|---|---|
| 48 | 854815 R<br>953539 R<br>948900 R | NC_007795.1<br>NC_017340.1<br>NC_010079.1 | NCTC_8325<br>04_02981<br>USA300_TCH1516 |
| 49 | 2459738 F<br>2364478 F | NC_017340.1<br>NC_022222.1 | 04_02981<br>6850 |
| 50 | 1812380 R<br>1775835 R<br>1714993 R | NC_010079.1<br>NC_017340.1<br>NC_007795.1 | USA300_TCH1516<br>04_02981<br>NCTC_8325 |

A ninth aspect of the present teaching relates to a method of selecting a treatment of a patient suffering from an infection with a potentially resistant Staphylococcus, particularly Staphylococcus aureus, strain, comprising the steps of:

a) obtaining or providing a sample containing or suspected of containing at least one Staphylococcus, particularly Staphylococcus aureus, strain from the patient;
b) determining the presence of at least one genetic variation in at least two positions from the group of positions annotated with Nos. 1 - 50 with regard to the reference genomes with the genome names given in Tables 3a and/or 3b, wherein the presence of said at least two genetic variations is indicative of a resistance to one or more antimicrobial, e.g. antibiotic, drugs, wherein for some positions more than one position in different reference genomes is annotated;
c) identifying said at least one or more antimicrobial, e.g. antibiotic, drugs; and
d) selecting one or more antimicrobial, e.g. antibiotic, drugs different from the ones identified in step c) and being suitable for the treatment of a Staphylococcus, particularly Staphylococcus aureus, infection.

Further aspects and embodiments of the teaching are disclosed in the dependent claims and can be taken from the following description, figures and examples, without being limited thereto.

Figures

The enclosed drawings should illustrate embodiments of the present teaching and convey a further understanding thereof. In connection with the description they serve as explanation of concepts and principles of the teaching. Other embodiments and many of the stated advantages can be derived in relation to the drawings. The elements of the drawings are not necessarily to scale towards each other. Identical, functionally equivalent and acting equal features and components are denoted in the figures of the drawings with the same reference numbers, unless noted otherwise.

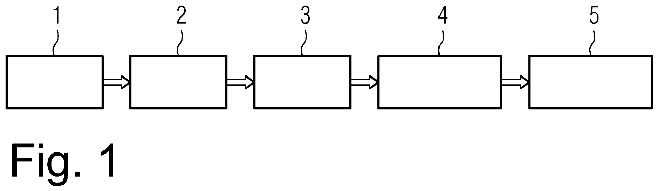

Fig. 1 shows schematically a read-out concept for a diagnostic test according to a method of the present teaching.

Detailed description

Definitions

Unless defined otherwise, technical and scientific terms used herein have the same meaning as commonly understood by one of ordinary skill in the art to which this invention belongs.

An "antimicrobial drug" in the present teaching refers to a group of drugs that includes antibiotics, antifungals, anti-protozoals, and antivirals. According to certain embodiments, the antimicrobial drug is an antibiotic.

The term "nucleic acid molecule" refers to a polynucleotide molecule having a defined sequence. It comprises DNA molecules, RNA molecules, nucleotide analog molecules and combinations and derivatives thereof, such as DNA molecules or RNA molecules with incorporated nucleotide analogs or cDNA.

The term "nucleic acid sequence information" relates to an information which can be derived from the sequence of a nucleic acid molecule, such as the sequence itself or a variation in the sequence as compared to a reference sequence.

In general, the term "genetic variation" relates to a position in the genome where at least two random organisms of a species are different. The term "genetic variation" particularly relates to a variation in the sequence as compared to one or more reference sequences, e.g. single nucleotide polymorphisms (SNPs), mutations, copy number variations, etc. Such reference sequences can be sequences determined in a predominant wild type organism or a reference organism, e.g. a defined and known bacterial strain or substrain, e.g. of a bac terial species like Staphylococcus aureus, which can have large variations in gene content among closely related strains. A genetic variation is for example a deletion of one or multiple nucleotides, an insertion of one or multiple nucleotides, or substitution of one or multiple nucleotides, duplication of one or a sequence of multiple nucleotides, translocation of one or a sequence of multiple nucleotides, and, in particular, a single nucleotide polymorphism (SNP).

For clearly identifying the variations, these can then be annotated to one or more reference sequences, e.g. a defined and known bacterial strain or substrain, but also a pan-genome of a microorganism like Staphylococcus aureus. The pan-genome generally includes the genes present in all strains of the microorganism, e.g. the bacterial species, as well as genes present in two or more strains, and genes specific to single strains.

According to certain embodiments, genetic variations were obtained with alignment-free methods, e.g. for detecting single base exchanges, for example based on contigs that were constructed by assemblies. For example, reads obtained from sequencing can be assembled to contigs and the contigs can be compared to each other.

In the context of the present teaching a "sample" is a sample which comprises at least one nucleic acid molecule from a bacterial microorganism. Examples for samples are: cells, tissue, body fluids, biopsy specimens, blood, urine, saliva, sputum, plasma, serum, cell culture supernatant, swab sample and others. According to certain embodiments, the sample is a patient sample (clinical isolate).

New and highly efficient methods of sequencing nucleic acids referred to as next generation sequencing have opened the possibility of large scale genomic analysis. The term "next generation sequencing" or "high throughput sequencing" refers to high-throughput sequencing technologies that parallelize the sequencing process, producing thousands or millions of sequences at once. Examples include Massively Parallel Signature Sequencing (MPSS), Polony sequencing, 454 pyrosequencing, Illumina (Solexa) sequencing, SOLID sequencing, Ion semiconductor sequencing, DNA nanoball sequencing, Helioscope(TM) single molecule sequencing, Single Molecule SMRT(TM) sequencing, Single Molecule real time (RNAP) sequencing, Nanopore DNA sequencing, Sequencing By Hybridization, Amplicon Sequencing, GnuBio.

Within the present description the term "microorganism" comprises the term microbe. The type of microorganism is not particularly restricted, unless noted otherwise or obvious, and, for example, comprises bacteria, viruses, fungi, microscopic algae und protozoa, as well as combinations thereof. According to certain aspects, it refers to one or more Staphylococcus aureus strains.

A reference to a microorganism or microorganisms in the present description comprises a reference to one microorganism as well a plurality of microorganisms, e.g. two, three, four, five, six or more microorganisms.

A vertebrate within the present teaching refers to animals having a vertebrae, which includes mammals - including humans, birds, reptiles, amphibians and fishes. The present teaching thus is not only suitable for human medicine, but also for veterinary medicine.

According to certain embodiments, the patient in the present methods is a vertebrate, more preferably a mammal and most preferred a human patient.

Before the teaching is described in exemplary detail, it is to be understood that this teaching is not limited to the particular component parts of the process steps of the methods described herein as such methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. For example, the term "a" as used herein can be understood as one single entity or in the meaning of "one or more" entities. It is also to be understood that plural forms include singular and/or plural referents unless the context clearly dictates otherwise. It is moreover to be understood that, in case parameter ranges are given which are delimited by numeric values, the ranges are deemed to include these limitation values.

Regarding the dosage of the antimicrobial, e.g. antibiotic, drugs, it is referred to the established principles of pharmacology in human and veterinary medicine. For example, Forth, Henschler, Rummel "Allgemeine und spezielle Pharmakologie und Toxikologie", 9th edition, 2005 might be used as a guideline. Regarding the formulation of a ready-to-use medicament, reference is made to "Remington, The Science and Practice of Pharmacy", 22nd edition, 2013.

Assembling of a gene sequence can be carried out by any known method and is not particularly limited.

According to a first aspect, the present teaching relates to a method of determining an antimicrobial drug, e.g. antibiotic, resistance profile for a microorganism, particularly a bacterial microorganism, comprising:

> obtaining or providing a first data set of gene sequences of a plurality of clinical isolates of the microorganism;
> wherein at least a part of the gene sequences of the first data set are assembled;
> analyzing the gene sequences of the first data set for genetic variants to obtain a third data set of genetic variants;
> providing a second data set of antimicrobial drug, e.g. antibiotic, resistance and/or susceptibility of the plurality of clinical isolates of the microorganism;

correlating the third data set with the second data set and statistically analyzing the correlation; and
determining the genetic sites in the genome of the microorganism with antimicrobial drug, e.g. antibiotic, resistance.

In this method, as well as the other methods of the teaching, the first data set of gene sequences of a plurality of clinical isolates can be provided or obtained in any way, preferably non-invasive, and can be e.g. provided from *in vitro* samples.

According to certain embodiments, the obtaining or providing of gene sequences of a plurality of clinical isolates in this method - as well as the other methods of the teaching - can comprise the following:

A sample of a vertebrate, e.g. a human, e.g. is provided or obtained and nucleic acid sequences, e.g. DNA or RNA sequences, are recorded by a known method for recording nucleic acid, which is not particularly limited. For example, nucleic acid can be recorded by a sequencing method, wherein any sequencing method is appropriate, particularly sequencing methods wherein a multitude of sample components, as e.g. in a blood sample, can be analyzed for nucleic acids and/or nucleic acid fragments and/or parts thereof contained therein in a short period of time, including the nucleic acids and/or nucleic acid fragments and/or parts thereof of at least one microorganism of interest, particularly a bacterial microorganism, e.g. of the species Staphylococcus aureus. For example, sequencing can be carried out using polymerase chain reaction (PCR), particularly multiplex PCR, or high throughput sequencing or next generation sequencing, preferably using high-throughput sequencing. For sequencing, preferably an *in vitro* sample is used.

The data obtained by the sequencing can be in any format, and can then be used to identify the nucleic acids of the microorganism, e.g. of Staphylococcus aureus species, to be identified, by known methods, e.g. fingerprinting methods, comparing genomes and/or aligning to at least one, or more, genomes of one or more species of the microorganism of interest, i.e. a reference genome, etc., forming a third data set of aligned genes for a microorganism, particularly Staphylococcus aureus - discarding additional data from other sources, e.g. the vertebrate. According to certain embodiments, at least a part of the gene sequences of the first data set are assembled, wherein assembly can be carried out by any known method and is not particularly limited. According to certain embodiments, the data of the gene sequences are essentially all or all assembled. However, also data from genomes of known species, e.g. from bacterial species like Staphylococcus aureus, that are already known, e.g. from databases like at the NCBI, can be used in the first data set.

For some organisms, it might be useful in genome-wide association studies to reference the points of interest, e.g. mutations, to one constant reference for enhanced standardization. In case of the human with a high consistency of the genome and 99% identical sequences among individuals this is easy and represents the standard, as corresponding reference genomes are available in databases.

In case of organisms that trigger infectious diseases (e.g. bacteria and viruses) this is much more difficult, though, and particularly also genetic variations that are not on genes, particularly known genes, can be missed when aligning sequence data to a reference genome. One possibility to overcome this is to fall back on a virtual pan-genome which contains all sequences of a certain genus or to perform reference free variation calling. A further possibility is the analysis of all available references, which is much more complex. Therein all n references from a database (e.g. RefSeq) are extracted and compared with the newly sequenced bacterial genomes k. After this, matrices (% of mapped reads, % of covered genome) can be applied and the data can be compared to several reference genomes. In such a case, n x k complete alignments are carried out. Having a big number of references, stable results can be obtained, as is the case for e.g. Staphylococcus aureus. Further, due to the high division rate under stress / an exogenous signal a jump in the mutation rate can be observed.

According to the teaching, the gene sequence of the first data set are assembled, at least in part, with known methods, e.g. by de-novo assembly or mapping assembly. The sequence assembly is not particularly limited, and any known genome assembler can be used, e.g. based on Sanger, 454, Solexa, Illumina, SOLid technologies, etc., as well as hybrids/mixtures thereof.

According to certain embodiments, the data of nucleic acids of different origin than the microorganism of interest, e.g. a bacterial microorganism like Staphylococcus aureus, can be removed after the nucleic acids of interest are identified, e.g. by filtering the data out. Such data can e.g. include nucleic acids of a patient, e.g. the vertebrate, e.g. human, and/or other microorganisms, etc. This can be done by e.g. computational subtraction, as developed by Meyerson et al. 2002. For this, also aligning to the genome of the vertebrate, etc., is possible. For aligning, several alignment-tools are available. This way the original data amount from the sample can be drastically reduced.

After such removal of "excess" data, obtaining the third data set can be carried out for the microorganism, e.g. Staphylococcus aureus, as described above.

Using these techniques, genetic variations in the gene sequences of the microorganism of interest, e.g. a bacterial microorganism like Staphylococcus aureus, can be obtained for various species.

When testing these same species for antimicrobial drug, e.g. antibiotic, susceptibility of a number of antimicrobial drugs, e.g. antibiotics, e.g. using standard culturing methods on dishes with antimicrobial drug, e.g. antibiotic, intake, as e.g. described below, the results of these antimicrobial drug, e.g. antibiotic, susceptibility tests can then be cross-referenced/correlated with the genetic variations in the genome of the respective microorganism, e.g. Staphylococcus aureus. Using several, e.g. 50 or more than 50, 100 or more than 100, 200 or more than 200, 400 or more than 400, 800 or more than 800, or 900 or more than 900 different isolates of the same or different species of a microorganism, e.g. of Staphylococcus aureus, statistical analysis can be carried out on the obtained cross-referenced data between genetic variations and antimicrobial drug, e.g. antibiotic, susceptibility for these microorganisms, using known methods.

Regarding culturing methods, samples of microorganisms can be e.g. cultured overnight. On the next day individual colonies can be used for identification of organisms, either by culturing or using mass spectroscopy. Based on the identity of organisms new plates containing increasing concentration of antibiotics used for the treatment of these organisms are inoculated and grown for additional 12 - 24 hours. The lowest drug concentration which inhibits growth (minimal inhibitory concentration - MIC) can be used to determine susceptibility/resistance for tested antibiotics.

Also, resistance testing can be carried out by determining e.g. known resistance genes in the different isolates, e.g. in case of methicillin resistant Staphylococcus aureus (MRSA) and methicillin susceptible Staphylococcus aureus (MSSA), but also regarding resistances of Staphylococcus to one or more (different) drugs, e.g. antibiotics. For determining resistances, respectively susceptibility, the data from culturing methods and/or from determining known resistance genes, as well as data obtained in different ways, e.g. based on mass spectrometry (possibly also in connection with culturing) can be used.

Correlation of the genetic variations with antimicrobial drug, e.g. antibiotic, resistance can be carried out in a usual way and is not particularly limited. For example, resistances can be correlated to genetic variances in the whole genome of the respective microorganism or only parts thereof, for example only coding parts of the genome. In some cases even only genetic variations in genes, e.g. certain genes, or certain mutations, e.g. SNPs, in genes can be determined. After correlation, statistical analysis can be carried out.

According to certain embodiments, the genetic variants in the gene sequences of the first data set are single nucleotide polymorphisms (SNPs).

According to certain embodiments, the data of the first data set, particularly SNPs, can be filtered prior to a possible annotation to a pan-genome and/or reference genome(s) and the correlation with the resistance/susceptibility data.

For example, to reduce the number of similar annotations they can be filtered and aggregated by one or more of the following:

- Only annotations for which the considered SNP lies on a protein can be kept and the further data discarded
- Only annotations which do not contain "hypothetical proteins" can be kept
- Annotations can be sorted by SNP identification number (ID) and gene product
- For a unique pair of SNP IDs and gene products only the first annotation can be kept

Also, according to certain embodiments, the following SNPs can be excluded:

- SNPs without any annotation or SNPs whose all annotations contain flag "synonymous", so that only SNPs with at least one non-synonymous annotation, e.g. a non-synonymous coding, are considered
- Constant SNPs, i.e. with the same value for all samples
- Almost constant SNPs: SNPs whose most frequent value has a frequency $\geq$ 95%, i.e. min. 95% of all samples have the same SNP value
- SNPs with a missing value ("-") for more than 10% of samples can also be removed

According to certain embodiments, the SNPs are detected alignment-free. This way also SNPs can be found that are not found in one or more certain reference genomes. For example, the assembled gene sequences can be compared to each other, as described above. Nevertheless, as noted above, it is also possible to include known gene sequences of microorganisms of e.g. the same species, e.g. Staphylococcus species, particularly Staphylococcus aureus, that are e.g. deposited for the public, e.g. at the NCBI, and use also these data for finding genetic variations.

According to certain embodiments, the SNPs are annotated to a pan-genome of the microorganism and/or annotated to one or more reference genomes of the microorganism, e.g. a Staphylococcus species, particularly Staphylococcus aureus. For example, according to certain embodiments the microorganism used in the above method is a Staphylococcus species, particularly Staphylococcus aureus, and the antimicrobial drug is methicillin, and/or one or more of the antibiotics described below. For such embodiments, the 50 genetic variations with the highest statistical probability (particularly using 49 finished S. aureus genomes from NCBI including the chromosome and available plasmids and 995 S. aureus de novo assemblies which have an assembly) determined according to the present method obtained are the ones given in Table 1. In Table 1, the position of the genetic variation (named "position"; with R being reverse direction and F being forward direction) are given for each variation (given with consecutive numbers 1 - 50) with reference to one or more known reference genomes from the NCBI (with the NCBI number given in the column "reference genome" and the genome name given in the column "genome name"). The reference genomes are attached to this application as sequence listing.

The reference genomes used in Table 1 for annotation thereby were obtained from the following Staphylococcus aureus strains and are as follows: NC_017340, NC_010079, NC_022222, NC_021670, NC_017351, NC_002953, NC_017337, NC_018608, NC_007795, NC_021059, NC_021554, NC_016912, NC_022226, and NC_022113, given in the following in the same order in more detail:

http://www.genome.jp/dbget-bin/www_bget?refseq+NC_017340

```
LOCUS         NC_017340 2821452 bp DNA circular CON 15-JUL-2015
DEFINITION    Staphylococcus aureus 04-02981, complete genome.
ACCESSION     NC_017340
VERSION       NC _ 017340.1 GI:387149188
DBLINK        BioProject: PRJNA224116
              BioSample: SAMN02603764
              Assembly: GCF_000025145.1
KEYWORDS      RefSeq.
SOURCE        Staphylococcus aureus 04-02981
ORGANISM      Staphylococcus aureus 04-02981
              Bacteria; Firmicutes; Bacilli; Bacillales; Staphylococcus.
REFERENCE     1 (bases 1 to 2821452)
AUTHORS       Nubel,U., Dordel,J., Kurt,K., Strommenger,B., Westh,H., Shukla,S.K., Zemlickova,H., Leblois,R.,
              Wirth,T., Jombart,T., Balloux,F. and Witte,W.
TITLE         A timescale for evolution, population expansion, and spatial spread of an emerging clone of
              methicillin-resistant Staphylococcus aureus
JOURNAL       PLoS Pathog. 6 (4), E1000855 (2010)
PUBMED        20386717
REMARK        Publication Status: Online-Only
REFERENCE     2 (bases 1 to 2821452)
AUTHORS       Nuebel,U., Dordel,J., Kurt,K., Strommenger,B., Westh,H., Shukla,S.K., Zemlickova,H., Leblois,R.,
              Wirth,T., Jombart,T., Balloux,F. and Witte,W.
TITLE         Direct Submission
JOURNAL       Submitted (05-NOV-2010) Nosocomial Infections, Robert Koch Institute, Burgstr. 37, Wernigerode
              38855, Germany
REMARK        Sequence update by submitter
REFERENCE     3 (bases 1 to 2821452)
AUTHORS       Nuebel,U., Dordel,J., Kurt,K., Strommenger,B., Westh,H., Shukla,S.K., Zemlickova,H., Leblois,R.,
              Wirth,T., Jombart,T., Balloux,F. and Witte,W.
TITLE         Direct Submission
JOURNAL       Submitted (22-DEC-2009) Nosocomial Infections, Robert Koch Institute, Burgstr. 37, Wernigerode
              38855, Germany
```

http://www.genome.jp/dbget-bin/www_bget?refseq+NC_010079

```
LOCUS         NC_010079 2872915 bp DNA circular CON 15-JUL-2015
DEFINITION    Staphylococcus aureus subsp. aureus USA300_TCH1516, complete genome.
ACCESSION     NC_010079
VERSION       NC_010079.1 GI:161508266
DBLINK        BioProject: PRJNA224116
              BioSample: SAMN00253845
              Assembly: GCF_000017085.1
KEYWORDS      RefSeq.
SOURCE        Staphylococcus aureus subsp. aureus USA300_TCH1516
```

(continued)

| | |
|---|---|
| ORGANISM | Staphylococcus aureus subsp. aureus USA300_TCH1516 |
| | Bacteria; Firmicutes; Bacilli; Bacillales; Sta-phylococcus. |
| REFERENCE | 1 (bases 1 to 2872915) |
| AUTHORS | Highlander,S.K., Hulten,K.G., Qin,X., Jiang,H., Yerrapragada,S., Mason,E.O. Jr., Shang,Y., Williams,T.M., Fortunov,R.M., Liu,Y., Igboeli,O., Petrosino,J., Tirumalai,M., Uzman,A., Fox,G.E., Cardenas,A.M., Muzny,D.M., Hemphill,L., Ding,Y., Dugan,S., Blyth,P.R., Buhay,C.J., Dinh,H.H., Hawes,A.C., Holder,M., Kovar,C.L., Lee,S.L., Liu,W., Nazareth,L.V., Wang,Q., Zhou,J., Kaplan, S.L. and Weinstock,G.M. |
| TITLE | Subtle genetic changes enhance virulence of methicillin resistant and sensitive Staphylococcus aureus |
| JOURNAL | BMC Microbiol. 7, 99 (2007) |
| PUBMED | 17986343 |
| REMARK | Publication Status: Online-Only |
| REFERENCE | 2 (bases 1 to 2872915) |
| AUTHORS | Muzny,D., Qin,X., Buhay,C., Dugan-Rocha,S., Ding,Y., Chen,G., Hawes,A., Holder,M., Jhangiani, S., Johnson,A., Khan,Z., Li,Z., Liu,W., Liu,X., Perez,L., Shen,H., Wang,Q., Watt,J., Xi,L., Xin,Y., Zhou,J., Deng,J., Jiang,H., Liu,Y., Qu,J., Song,X.-Z., Zhang,L., Villasana, D., Johnson, A., Liu,J., Liyanage,D., Lorensuhewa,L., Robinson,T., Song,A., Song,B.-B., Dinh,H., Thornton,R., Coyle,M., Francis- |
| | co,L., Jackson,L., Javaid,M., Korchina,V., Kovar,C., Mata,R., Mathew,T., Ngo,R., Nguyen,L., Nguyen,N., Okwuonu,G., Ongeri,F., Pham,C., Simmons, D., Wilczek-Boney,K., Hale,W., Jakkamsetti,A., Pham,P., Ruth,R., San Lucas,F., Warren,J., Zhang,J., Zhao,Z., Zhou,C., Zhu,D., Lee,S., Bess,C., Blankenburg,K., Forbes,L., Fu,Q., Gubbala,S., Hirani,K., Jayaseelan,J.C., Lara, F., Munidasa,M., Palculict,T., Patil,S., Pu,L.-L., Saada,N., Tang,L., Weissenberger,G., Zhu,Y., Hemphill,L., Shang,Y., Youmans,B., Ayvaz,T., Ross,M., Santibanez,J., Aqrawi,P., Gross,S., Joshi, V., Fowler,G., Nazareth,L., Reid,J., Worley,K., Petrosino,J., Highlander,S. and Gibbs,R. |
| TITLE | Direct Submission |
| JOURNAL | Submitted (20-JUN-2007) Molecular Virology and Microbiology and the Human Genome Sequencing Center, Baylor College of Medicine, One Baylor Plaza, Houston, TX 77030, USA |

http://www.genome.jp/dbget-bin/www_bget?refseq+NC_022222

| | |
|---|---|
| LOCUS | NC_022222 2736560 bp DNA circular CON 17-FEB-2015 |
| DEFINITION | Staphylococcus aureus subsp. aureus 6850, complete genome. |
| ACCESSION | NC_022222 |
| VERSION | NC_022222.1 GI:537441500 |
| DBLINK | BioProject: PRJNA224116 |
| | BioSample: SAMN02604264 |
| | Assembly: GCF_000462955.1 |
| KEYWORDS | RefSeq. |
| SOURCE | Staphylococcus aureus subsp. aureus 6850 |
| ORGANISM | Staphylococcus aureus subsp. aureus 6850 |
| | Bacteria; Firmicutes; Bacilli; Bacillales; Staphylococcus. |
| REFERENCE | 1 (bases 1 to 2736560) |
| AUTHORS | Fraunholz,M., Bernhardt,J., Schuldes,J., Daniel,R., Hecker,M. and Sinha,B. |
| TITLE | Complete Genome Sequence of Staphylococcus aureus 6850, a Highly Cytotoxic and Clinically Virulent Methicillin-Sensitive Strain with Distant Relatedness to Prototype Strains |
| JOURNAL | Genome Announc 1 (5) (2013) |
| PUBMED | 24072870 |
| REMARK | Publication Status: Online-Only |
| REFERENCE | 2 (bases 1 to 2736560) |
| AUTHORS | Fraunholz,M.J., Bernhardt,J., Hecker,M., Schuldes,J., Daniel,R. and Sinha,B. |

(continued)

| | |
|---|---|
| TITLE | Direct Submission |
| JOURNAL | Submitted (26-AUG-2013) Department of Microbiology, University of Wuerzburg, Am Hubland, Wuerzburg 97074, Germany |

http://www.genome.jp/dbget-bin/www_bget?refseq+NC_021670

| | |
|---|---|
| LOCUS | NC_021670 2980548 bp DNA circular CON 07-FEB-2015 |
| DEFINITION | Staphylococcus aureus Bmb9393, complete genome. |
| ACCESSION | NC_021670 |
| VERSION | NC_021670.1 GI:521210823 |
| DBLINK | BioProject: PRJNA224116 |
| | BioSample: SAMN02603524 |
| | Assembly: GCF_000418345.1 |
| KEYWORDS | RefSeq. |
| SOURCE | Staphylococcus aureus Bmb9393 |
| ORGANISM | Staphylococcus aureus Bmb9393 |
| | Bacteria; Firmicutes; Bacilli; Bacillales; Staphylococcus. |
| REFERENCE | 1 (bases 1 to 2980548) |
| AUTHORS | Costa,M.O., Beltrame,C.O., Ferreira,F.A., Botel-ho,A.M., Lima,N.C., Souza,R.C., de Almeida,L.G., Vasconcelos,A.T., Nicolas,M.F. and Figueiredo,A.M. |
| TITLE | Complete Genome Sequence of a Variant of the Methicillin-Resistant Staphylococcus aureus ST239 Lineage, Strain BMB9393, Displaying Superior Ability To Accumulate ica-Independent Biofilm |
| JOURNAL | Genome Announc 1 (4) (2013) |
| PUBMED | 23929475 |
| REMARK | Publication Status: Online-Only |
| REFERENCE | 2 (bases 1 to 2980548) |
| AUTHORS | Costa,M.O.C., Beltrame,C.O., Lima,N.C.B., Al-meida,L.G.P., Vasconcelos,A.T.R., Ferreira,F.A., Nicolas,M.F. and Figueiredo,A.M.S. |
| TITLE | Direct Submission |
| JOURNAL | Submitted (15-APR-2013) Labinfo, LNCC - Laboratorio Nacional de Computacao Cientifica, Rua Getulio Vargas 333, Petropolis, RJ 25651070, Brazil |

http://www.genome.jp/dbget-bin/www_bget?refseq+NC_017351

| | |
|---|---|
| LOCUS | NC_017351 2846546 bp DNA circular CON 07-FEB-2015 |
| DEFINITION | Staphylococcus aureus subsp. aureus 11819-97, complete genome. |
| ACCESSION | NC_017351 |
| VERSION | NC_017351.1 GI:385780298 |
| DBLINK | BioProject: PRJNA224116 |
| | BioSample: SAMN02603886 |
| | Assembly: GCF_000239235.1 |
| KEYWORDS | RefSeq. |
| SOURCE | Staphylococcus aureus subsp. aureus 11819-97 |
| ORGANISM | Staphylococcus aureus subsp. aureus 11819-97 Bacteria; Firmicutes; Bacilli; Bacillales; Staphylococcus. |
| REFERENCE | 1 (bases 1 to 2846546) |
| AUTHORS | Stegger,M., Price,L.B., Larsen,A.R., Gillece,J.D., Waters,A.E., Skov,R. and Andersen,P.S. |
| TITLE | Genome sequence of Staphylococcus aureus strain 11819-97, an ST80-IV European community-acquired methicillin-resistant isolate |
| JOURNAL | J. Bacteriol. 194 (6), 1625-1626 (2012) |
| PUBMED | 22374956 |

| REFERENCE | 2 (bases 1 to 2846546) |
|---|---|
| AUTHORS | Stegger,M., Price,L.B., Larsen,A.R., Gillece,J.D., Waters,A.E., Skov,R. and Andersen,P.S. |
| TITLE | Direct Submission |
| JOURNAL | Submitted (13-DEC-2011) Department of Microbiological Surveillance and Research, Statens Serum Institut, Oerestads Boulevard 5, 2300 Copenhagen S, 5 Artillerivej, Denmark |

http://www.genome.jp/dbget-bin/www_bget?refseq+NC_002953

| LOCUS | NC_002953 2799802 bp DNA circular CON 07-FEB-2015 |
|---|---|
| DEFINITION | Staphylococcus aureus strain MSSA476, complete genome. |
| ACCESSION | NC_002953 |
| VERSION | NC_002953.3 GI:49484912 |
| DBLINK | BioProject: PRJNA224116 |
| | Assembly: GCF_000011525.1 |
| KEYWORDS | RefSeq; complete genome. |
| SOURCE | Staphylococcus aureus subsp. aureus MSSA476 |
| ORGANISM | Staphylococcus aureus subsp. aureus MSSA476 |
| | Bacteria; Firmicutes; Bacilli; Bacillales; Staphylococcus. |
| REFERENCE | 1 (bases 1 to 2799802) |
| AUTHORS | Holden,M.T., Feil,E.J., Lindsay,J.A., Peacock,S.J., Day,N.P., Enright,M.C., Foster,T.J., Moore, C.E., |
| | Hurst,L., Atkin,R., Barron,A., Bason,N., Bentley,S.D., Chillingworth,C., Chillingworth,T., Churcher, C., Clark,L., Corton,C., Cronin,A., Doggett,J., Dowd,L., Feltwell,T., Hance,Z., Harris,B., Hauser,H., Holroyd,S., Jagels,K., James,K.D., Lennard,N., Line,A., Mayes,R., Moule,S., Mungall,K., Ormond, D., Quail,M.A., Rabbinowitsch,E., Rutherford,K., Sanders,M., Sharp,S., Simmonds,M., Stevens,K., Whitehead,S., Barrell,B.G., Spratt,B.G. and Parkhill,J. |
| TITLE | Complete genomes of two clinical Staphylococcus aureus strains: evidence for the rapid evolution of virulence and drug resistance |
| JOURNAL | Proc. Natl. Acad. Sci. U.S.A. 101 (26), 9786-9791 (2004) |
| PUBMED | 15213324 |
| REFERENCE | 2 (bases 1 to 2799802) |
| AUTHORS | Holden,M.T.G. |
| TITLE | Direct Submission |
| JOURNAL | Submitted (23-JUN-2004) Submitted on behalf of the Pathogen Sequencing Unit, Sanger Institute, Wellcome Trust Genome Campus, Hinxton, Cambridge CB10 1SA, E-mail: mh3@sanger.ac.uk |

http://www.genome.jp/dbget-bin/www_bget?refseq+NC_017337

| LOCUS | NC_017337 2832478 bp DNA circular CON 07-FEB-2015 |
|---|---|
| DEFINITION | Staphylococcus aureus subsp. aureus ED133, complete genome. |
| ACCESSION | NC_017337 |
| VERSION | NC_017337.1 GI:384546269 |
| DBLINK | BioProject: PRJNA224116 |
| | BioSample: SAMN02604166 |
| | Assembly: GCF_000210315.1 |
| KEYWORDS | RefSeq. |
| SOURCE | Staphylococcus aureus subsp. aureus ED133 |
| ORGANISM | Staphylococcus aureus subsp. aureus ED133 |
| | Bacteria; Firmicutes; Bacilli; Bacillales; Staphylococcus. |
| REFERENCE | 1 (bases 1 to 2832478) |

(continued)

| | |
|---|---|
| AUTHORS | Guinane,C.M., Ben Zakour,N.L., Tormo-Mas,M.A., Weinert,L.A., Lowder,B.V., Cartwright,R.A., Smyth,D.S., Smyth,C.J., Lindsay,J.A., Gould,K.A., Witney,A., Hinds,J., Bollback,J.P., Rambaut,A., Penades,J.R. and Fitzgerald,J.R. |
| TITLE | Evolutionary genomics of Staphylococcus aureus reveals insights into the origin and molecular basis of ruminant host adaptation |
| JOURNAL | Genome Biol Evol 2, 454-466 (2010) |
| PUBMED | 20624747 |
| REMARK | Publication Status: Online-Only |
| REFERENCE | 2 (bases 1 to 2832478) |
| AUTHORS | Guinane,C.M., Ben Zakour,N.L., Tormo-Mas,M.A., Weinert,L.A., Lowder,B.V., Cartwright,R.A., Smyth,D.S., Smyth,C.J., Lindsay,J., Gould,K.A., Witney,A., Hinds,J., Bollback,J.P., Rambaut,A., Penades,J. and Fitzgerald,J.R. |
| TITLE | Direct Submission |
| JOURNAL | Submitted (29-MAR-2010) The Roslin Institute and Centre for Infectious Diseases, Royal (Dick) School of Veterinary Studies, University of Edinburgh, The Chancellor's Building, New Royal Infirmary, 49 Little France Crescent, Edinburgh EH16 4SB, United Kingdom |

http://www.genome.jp/dbget-bin/www_bget?refseq+NC_018608

| | |
|---|---|
| LOCUS | NC_018608 2782313 bp DNA circular CON 07-FEB-2015 |
| DEFINITION | Staphylococcus aureus 08BA02176, complete genome. |
| ACCESSION | NC_018608 |
| VERSION | NC_018608.1 GI:404477334 |
| DBLINK | BioProject: PRJNA224116 |
| | BioSample: SAMN02603722 |
| | Assembly: GCF_000296595.1 |
| KEYWORDS | RefSeq. |
| SOURCE | Staphylococcus aureus 08BA02176 |
| ORGANISM | Staphylococcus aureus 08BA02176 |
| | Bacteria; Firmicutes; Bacilli; Bacillales; Staphylococcus. |
| REFERENCE | 1 (bases 1 to 2782313) |
| AUTHORS | Golding,G.R., Bryden,L., Levett,P.N., McDonald,R.R., Wong,A., Graham,M.R., Tyler,S., Van Domselaar,G., Mabon,P., Kent,H., Butaye,P., Smith,T.C., Kadlec,K., Schwarz,S., Weese,S.J. and Mulvey,M.R. |
| TITLE | whole-genome sequence of livestock-associated st398 methicillin-resistant staphylococcus aureus Isolated from Humans in Canada |
| JOURNAL | J. Bacteriol. 194 (23), 6627-6628 (2012) |
| PUBMED | 23144384 |
| REFERENCE | 2 (bases 1 to 2782313) |
| AUTHORS | Golding,G.R., Bryden,L., Levett,P.N., McDonald,R.R., Wong,A., Graham,M.R., Tyler,S., Van Domselaar,G., Mabon,P., Kent,H., Butaye,P., Smith,T.C., Kadlec,K., Schwarz,S., Weese,S.J. and Mulvey,M.R. |
| TITLE | Direct Submission |
| JOURNAL | Submitted (31-AUG-2012) Antimicrobial Resistance and Nosocomial Infections, Public Health Agency of Canada, National Microbiology Laboratory, 1015 Arlington Street, Winnipeg, Manitoba R3E 3R2, Canada |

http://www.genome.jp/dbget-bin/www_bget?refseq+NC_007795

| | |
|---|---|
| LOCUS | NC_007795 2821361 bp DNA circular CON 16-DEC-2014 |
| DEFINITION | Staphylococcus aureus subsp. aureus NCTC 8325 chromosome, complete genome. |
| ACCESSION | NC_007795 |

(continued)

| VERSION | NC_007795.1 GI:88193823 |
| DBLINK | BioProject: PRJNA57795 |
| KEYWORDS | RefSeq. |
| SOURCE | Staphylococcus aureus subsp. aureus NCTC 8325 |
| ORGANISM | Staphylococcus aureus subsp. aureus NCTC 8325 |
| | Bacteria; Firmicutes; Bacilli; Bacillales; Staphylococcus. |
| REFERENCE | 1 (bases 1 to 2821361) |
| AUTHORS | Gillaspy,A.F., Worrell,V., Orvis,J., Roe,B.A., Dyer,D.W. and Iandolo,J.J. |
| TITLE | The Staphylococcus aureus NCTC8325 Genome |
| JOURNAL | (in) Fischetti,V., Novick,R., Ferretti,J., Portnoy, D. and Rood, J. (Eds.); GRAM POSITIVE PATHOGENS; ASM Press (2006) |
| REFERENCE | 2 (bases 1 to 2821361) |
| CONSRTM | NCBI Genome Project |
| TITLE | Direct Submission |
| JOURNAL | Submitted (18-FEB-2006) National Center for Biotechnology Information, NIH, Bethesda, MD 20894, USA |
| REFERENCE | 3 (bases 1 to 2821361) |
| AUTHORS | Gillaspy,A.F., Worrell,V., Orvis,J., Roe,B.A., Dyer,D.W. and Iandolo,J.J. |
| TITLE | Direct Submission |
| JOURNAL | Submitted (27-JAN-2006) Microbiology and Immunology, The University of Oklahoma Health Sciences Center, 940 Stanton L. Young Boulevard, Oklahoma City, OK 73104, USA |

http://www.genome.jp/dbget-bin/www_bget?refseq+NC-021059

| LOCUS | NC_021059 2864125 bp DNA circular CON 01-MAR-2015 |
| DEFINITION | Staphylococcus aureus M1, complete genome. |
| ACCESSION | NC_021059 |
| VERSION | NC_021059.1 GI:479328021 |
| DBLINK | BioProject: PRJNA224116 |
| | Assembly: GCF_000367745.1 |
| KEYWORDS | RefSeq; complete genome. |
| SOURCE | Staphylococcus aureus M1 |
| ORGANISM | Staphylococcus aureus M1 |
| | Bacteria; Firmicutes; Bacilli; Bacillales; Staphylococcus. |
| REFERENCE | 1 |
| AUTHORS | Larner-Svensson,H., Worning,P., Bartels,M.D., Hestbjerg Hansen,L., Boye,K. and Westh,H. |
| TITLE | Complete Genome Sequence of Staphylococcus aureus Strain M1, a Unique t024-ST8-IVa Danish Methicillin-Resistant S. aureus Clone |
| JOURNAL | Genome Announc 1 (3) (2013) |
| PUBMED | 23792746 |
| REMARK | Publication Status: Online-Only |
| REFERENCE | 2 (bases 1 to 2864125) |
| AUTHORS | Worning,P. |
| TITLE | Direct Submission |
| JOURNAL | Submitted (18-MAR-2013) Dept. of Clinical Microbiology, Hvidovre Hospital, Kettegaard Alle 30, DK-2650, DENMARK |

http://www.genome.jp/dbget-bin/www_bget?refseq+NC_021554

| LOCUS | NC_021554 2850503 bp DNA circular CON 07-FEB-2015 |
| DEFINITION | Staphylococcus aureus CA-347, complete genome. |

(continued)

| ACCESSION | NC_021554 |
| --- | --- |
| VERSION | NC_021554.1 GI:514064966 |
| DBLINK | BioProject: PRJNA224116 |
| | BioSample: SAMN02603909 |
| | Assembly: GCF_000412775.1 |
| KEYWORDS | RefSeq. |
| SOURCE | Staphylococcus aureus CA-347 |
| ORGANISM | Staphylococcus aureus CA-347 |
| | Bacteria; Firmicutes; Bacilli; Bacillales; Staphylococcus. |
| REFERENCE | 1 (bases 1 to 2850503) |
| AUTHORS | Stegger,M., Driebe,E.M., Roe,C., Lemmer,D., Bowers,J.R., Engelthaler,D.M., Keim,P. and Andersen,P.S. |
| TITLE | Genome Sequence of Staphylococcus aureus Strain CA-347, a USA600 Methicillin-Resistant Isolate |
| JOURNAL | Genome Announc 1 (4) (2013) |
| PUBMED | 23887918 |
| REMARK | Publication Status: Online-Only |
| REFERENCE | 2 (bases 1 to 2850503) |
| AUTHORS | Stegger,M., Driebe,E.M., Roe,C., Lemmer,D., Engelthaler,D.M., Keim,P. and Andersen,P.S. |
| TITLE | Direct Submission |
| JOURNAL | Submitted (10-JUN-2013) CPHCP, TGen North, 3051 W. Shamrell Blvd., Ste. 106, Flagstaff, AZ 86001, USA |

http://www.genome.jp/dbget-bin/www_bget?refseq+NC_016912

| LOCUS | NC_016912 2692570 bp DNA circular CON 07-FEB-2015 |
| --- | --- |
| DEFINITION | Staphylococcus aureus subsp. aureus VC40, complete genome. |
| ACCESSION | NC_016912 |
| VERSION | NC_016912.1 GI:379013365 |
| DBLINK | BioProject: PRJNA224116 |
| | BioSample: SAMN02603393 |
| | Assembly: GCF_000245495.1 |
| KEYWORDS | RefSeq. |
| SOURCE | Staphylococcus aureus subsp. aureus VC40 |
| ORGANISM | Staphylococcus aureus subsp. aureus VC40 |
| | Bacteria; Firmicutes; Bacilli; Bacillales; Staphylococcus. |
| REFERENCE | 1 (bases 1 to 2692570) |
| AUTHORS | Sass,P., Berscheid,A., Jansen,A., Oedenkoven,M., Szekat,C., Strittmatter,A., Gottschalk,G. and Bierbaum,G. |
| TITLE | Genome sequence of Staphylococcus aureus VC40, a vancomycin- and daptomycin-resistant strain, to study the genetics of development of resistance to currently applied last-resort antibiotics |
| JOURNAL | J. Bacteriol. 194 (8), 2107-2108 (2012) |
| PUBMED | 22461548 |
| REFERENCE | 2 (bases 1 to 2692570) |
| AUTHORS | Sass,P., Berscheid,A., Jansen,A., Oedenkoven,M., Szekat,C., Strittmatter,A., Gottschalk,G. and Bierbaum,G. |
| TITLE | Direct Submission |
| JOURNAL | Submitted (25-AUG-2011) Institute of Medical Microbiology, Immunology and Parasitology, University of Bonn, Sigmund-Freud-Str. 25, Bonn 53105, Germany |

http://www.genome.jp/dbget-bin/www_bget?refseq+NC_022226

```
LOCUS        NC_022226 2751266 bp DNA circular CON 01-MAR-2015
DEFINITION   Staphylococcus aureus subsp. aureus CN1, complete genome.
ACCESSION    NC_022226
VERSION      NC_022226.1 GI:537459744
DBLINK       BioProject: PRJNA224116
             BioSample: SAMN02603420
             Assembly: GCF_000463055.1
KEYWORDS     RefSeq.
SOURCE       Staphylococcus aureus subsp. aureus CN1
ORGANISM     Staphylococcus aureus subsp. aureus CN1
             Bacteria; Firmicutes; Bacilli; Bacillales; Staphylococcus.
REFERENCE    1 (bases 1 to 2751266)
AUTHORS      Chen,Y., Chatterjee,S.S., Porcella,S.F., Yu,Y.S. and Otto,M.
TITLE        Complete genome sequence of a Panton-Valentine leukocidin-negative community-associated
             methicillin-resistant Staphylococcus aureus strain of sequence type 72 from Korea
JOURNAL      PLoS ONE 8 (8), E72803 (2013)
PUBMED       23977354
REMARK       Publication Status: Online-Only
REFERENCE    2 (bases 1 to 2751266)
AUTHORS      Otto,M. and Porcella,S.F.
TITLE        Direct Submission
JOURNAL      Submitted (04-DEC-2012) Laboratory of Human Bacterial Pathogenesis, NIAID/NIH, 9000 Rockville
             Pike, Bethesda, MD 20892, USA
```

http://www.genome.jp/dbget-bin/www_bget?refseq+NC_022113

```
LOCUS        NC_022113 2756919 bp DNA circular CON 07-FEB-2015
DEFINITION   Staphylococcus aureus subsp. aureus 55/2053, complete genome.
ACCESSION    NC_022113 NZ_ACJR01000000-NZ_ACJR01000094 NZ_GG700533-NZ_GG700558
VERSION      NC_022113.1 GI:532358222
DBLINK       BioProject: PRJNA224116
             BioSample: SAMN00103091
             Assembly: GCF_000160335.2
KEYWORDS     RefSeq.
SOURCE       Staphylococcus aureus subsp. aureus 55/2053
ORGANISM     Staphylococcus aureus subsp. aureus 55/2053
             Bacteria; Firmicutes; Bacilli; Bacillales; Staphylococcus.
REFERENCE    (bases 1 to 2756919)
1
AUTHORS      Feldgarden,M., Robinson,A., Wong,A., Smyth,D., Young,S.K., Zeng,Q., Gargeya,S., Fitzgerald,M.,
             Haas,B., Abouelleil,A., Alvarado,L., Arachchi,H.M., Berlin,A.,
             Brown,A., Chapman,S.B., Chen,Z., Dunbar,C., Gearin,G., Goldberg,J., Griggs,A., Gujja,S.,
             Heiman,D., Howarth,C., Larson,L., Lui,A., MacDonald,P.J.P., Montmayeur,A., Murphy,C., Neiman,
             D., Pearson,M., Priest,M., Roberts,A., Saif,S., Shea,T., Sisk,P., Stolte,C., Sykes,S., Wortman,J.,
             Nusbaum,C. and Birren,B.
CONSRTM      The Broad Institute Genome Sequencing Platform
TITLE        The Genome Sequence of Staphylococcus aureus strain 55-2053
JOURNAL      Unpublished
REFERENCE    2 (bases 1 to 2756919)
```

(continued)

| | | |
|---|---|---|
| AUTHORS | | Feldgarden,M., Robinson,A., Wong,A., Smyth,D., Young,S.K., Zeng,Q., Gargeya,S., Fitzgerald,M., Haas,B., Abouelleil,A., Alvarado,L., Arachchi,H.M., Berlin,A., Brown,A., Chapman,S.B., Chen,Z., Dunbar,C., Gearin,G., Goldberg,J., Griggs,A., Gujja,S., Heiman,D., Howarth,C., Larson,L., Lui,A., MacDonald,P.J.P., Montmayeur,A., Murphy,C., Neiman,D., Pearson,M., Priest,M., Roberts,A., Saif, S., Shea,T., Sisk,P., Stolte,C., Sykes,S., Wortman,J., Nusbaum,C. and Birren,B. |
| CONSRTM | | The Broad Institute Genome Sequencing Platform |
| TITLE | | Direct Submission |
| JOURNAL | | Submitted (10-MAY-2013) Broad Institute of MIT and Harvard, 7 Cambridge Center, Cambridge, MA 02142, USA |
| REFERENCE | | 3 (bases 1 to 2756919) |
| AUTHORS | | Feldgarden,M., Robinson,A., Wong,A., Smyth,D., Young,S.K., Zeng,Q., Koehrsen,M., Godfrey,P., Alvarado,L., Berlin,A., Borenstein,D., Chen,Z., Engels,R., Freedman,E., Gellesch,M., Goldberg,J., Griggs,A., Gujja,S., Heiman,D., Hepburn,T., Howarth,C., Jen,D., Larson,L., Lewis,B., Mehta,T., Park,D., Pearson,M., Roberts,A., Saif,S., Shea,T., Shenoy,N., Sisk,P., Stolte,C., Sykes,S., Walk, T., White,J., Yandava,C., Wirth,D.F., Galagan,J., Nusbaum,C. and Birren,B. |
| CONSRTM | | The Broad Institute Genome Sequencing Platform |
| TITLE | | Direct Submission |
| JOURNAL | | Submitted (02-APR-2009) Broad Institute of MIT and Harvard, 7 Cambridge Center, Cambridge, MA 02142, USA |

In addition, the genetic variations can also be annotated to a pan-genome constructed from the genomes used, and can be numbered using consecutive numbers. In the present method, the construction of a pan-genome is not particularly limited and can be done using known methods.

However, other suitable reference genomes (e.g. used in the Examples, but also for other microorganisms) can be found at publicly available data bases like at the NCBI.

Statistical analysis of the correlation of the gene mutations with antimicrobial drug, e.g. antibiotic, resistance is not particularly limited and can be carried out, depending on e.g. the amount of data, in different ways, for example using analysis of variance (ANOVA), Student's t-test or Fisher's exact test, for example with a sample size n of 50, 100, 200, 300, 400, 800 or 900, and a level of significance ($\alpha$-error-level) of e.g. 0.05 or smaller, e.g. 0.05, preferably 0.01 or smaller. A statistical value can be obtained for each genetic variation and/or each position in the genome as well as for all antibiotics tested, a group of antibiotics or a single antibiotic. The obtained p-values can also be adapted for statistical errors, if needed.

For statistically sound results a multitude of individuals should be sampled, with $n$ = 50, 100, 200, 300, 400, 800 or 900, and a level of significance (a-error-level) of e.g. 0.05 or smaller, e.g. 0.05, preferably 0.01 or smaller. According to certain embodiments, particularly significant results can be obtained for n = 200, 300, 400, 800 or 900.

For statistically sound results a multitude of individuals should be sampled, with $n$ = 50 or more, 100 or more, 200 or more, 300 or more, 400 or more, 800 or more or 900 or more, and a level of significance ($\alpha$-error-level) of e.g. 0.05 or smaller, e.g. 0.05, preferably 0.01 or smaller. According to certain embodiments, particularly significant results can be obtained for n = 200 or more, 300 or more, 400 or more, 800 or more or 900 or more.

After the above procedure has been carried out for more than 900, e.g. 987, individual strains of Staphylococcus species, particularly Staphylococcus aureus, the data disclosed in Tables 1 and 2 were obtained for the statistically best correlations between genetic variations and antimicrobial drug, e.g. antibiotic, resistances, particularly methicillin resistance. Thus, genetic variations in the positions given in Tables 1 and 2, with regard to the several reference genomes as above, were proven as valid markers for antimicrobial drug, e.g. antibiotic, resistance.

When referring to the second data set, wherein the second data set e.g. comprises, respectively is, a set of antimicrobial drug, e.g. antibiotic, resistances of a plurality of clinical isolates, this can, within the scope of the teaching, also refer to a self-learning data base that, whenever a new sample is analyzed, can take this sample into the second data set and thus expand its data base. The second data set thus does not have to be static and can be expanded, either by external input or by incorporating new data due to self-learning. This is, however, not restricted to the third aspect of the teaching, but applies to other aspects of the teaching that refer to a second data set, which does not necessarily have to refer to antimicrobial drug resistance. The same applies, where applicable, to the first data set, e.g. in the third aspect.

According to certain embodiments, statistical analysis in the present methods is carried out using Fisher's test with $p < 10^{-6}$, preferably $p < 10^{-9}$.

The method of the first aspect of the present teaching, as well as related methods, e.g. according to the 2nd,

3rd and 4th aspect, can, according to certain embodiments, comprise correlating different genetic sites to each other. This way even higher statistical significance can be achieved.

According to certain embodiments of the method of the first aspect and related methods - as above, the second data set can be provided by culturing the clinical isolates of the microorganism on agar plates provided with antimicrobial drugs, e.g. antibiotics, at different concentrations, and the second data can be obtained by taking the minimal concentration of the plates that inhibits growth of the respective microorganism, e.g. Staphylococcus aureus.

According to certain embodiments of the method of the first aspect and related methods, the antimicrobial drug, e.g. antibiotic drug, is selected from the group consisting of β-lactams, β-lactam inhibitors, quinolines and derivatives thereof, e.g. fluoroquinolones, aminoglycosides, glycopeptides, lincosamides, macrolides, nitrofuranes, oxazolidinones polyketides, respectively tetracyclines, and folate synthesis inhibitors, e.g. benzene derived/sulfonamide antibiotics, preferably from the group consisting of Amoxicillin/Clavulanate, Ampicillin, Ampicillin/Sulbactam, Azithromycin, Cefalothin, Cefazolin, Cefepime, Cefotaxime, Cefoxitin, Ceftriaxone, Cefuroxime, Chloramphenicol, Ciprofloxacin, Clindamycin, Daptomycin, Ertapenem, Erythromycin, Fosfomycin, Fusidic acid, Gentamicin, Imipenem, Levofloxacin, Linezolid, Meropenem, Methicillin, Moxifloxacin, Mupirocin, Nitrofurantoin, Norfloxacin, Ofloxacin, Oxacillin, Penicillin G, Piperacillin/Tazobactam, Quinupristin/Dalfopristin, Rifampicin, Teicoplanin, Tetracycline, Tigecycline, Tobramycin, Trimethoprim/Sulfamethoxazole, and Vancomycin.

According to a second aspect, the present teaching discloses a diagnostic method of determining an infection of a patient with a microorganism, particularly a bacterial microorganism potentially resistant to antimicrobial drug treatment, comprising the steps of:

a) obtaining or providing a sample containing or suspected of containing a microorganism, particularly a bacterial microorganism, from the patient;
b) determining the presence of at least one genetic variant in at least one position of the microorganism, particularly the bacterial microorganism, as determined by the method of the first aspect of the teaching, wherein the presence of said at least one genetic variant is indicative of an infection with an antimicrobial drug resistant microorganism in said patient.

Again, the microorganism can be a Staphylococcus species, particularly Staphylococcus aureus, according to certain embodiments, and the drug methicillin and/or a drug as described below, e.g. with regard to the eight and ninth aspect.

With this method, any mutations in the genome of a microorganism, e.g. a Staphylococcus species, particularly Staphylococcus aureus, e.g. a clinical isolate with an unknown strain of the microorganism, particularly bacterial microorganism, correlated with antimicrobial drug, e.g. antibiotic, resistance can be determined and a thorough antimicrobial drug, e.g. antibiotic, resistance profile can be established.

Again, the different steps can herein be carried out as described with regard to the first aspect of the present teaching.

According to this aspect, an infection with a microorganism, particularly a bacterial microorganism, e.g. a Staphylococcus, particularly Staphylococcus aureus, infection, in a patient can be determined using sequencing methods, as well as a resistance to antimicrobial drugs, e.g. antibiotics, of the microorganism, e.g. a Staphylococcus species, particularly Staphylococcus aureus, can be determined in a short amount of time compared to conventional methods.

In a third aspect, the present teaching relates to a method of selecting a treatment of a patient suffering from an infection with a potentially resistant microorganism, particularly bacterial microorganism, comprising the steps of:

a) obtaining or providing a sample containing or suspected of containing a microorganism, particularly a bacterial microorganism, from the patient;
b) determining the presence of at least one genetic variant in at least one position of the microorganism, particularly bacterial microorganism, as determined by the method of the first aspect of the teaching, wherein the presence of said at least one genetic variant is indicative of a resistance to one or more antimicrobial drugs;
c) identifying said at least one or more antimicrobial drugs; and
d) selecting one or more antimicrobial drugs different from the ones identified in step c) and being suitable for the treatment of the infection with the microorganism, particularly the bacterial microorganism.

This method can be carried out similarly to the second aspect of the teaching and enables a fast was to select a suitable treatment with antibiotics for any infection with an unknown microorganism, particularly bacterial microorganism, e.g. Staphylococcus aureus.

In this method, as well as similar ones, no aligning is necessary, as the unknown sample can be directly correlated, after the genome or genome sequences are produced, with the second data set, and thus genetic variations and antimicrobial drug, e.g. antibiotic, resistances can be determined. The first data set can be assembled, for example, using known techniques.

According to certain embodiments, statistical analysis in the present method is carried out using Fisher's test with $p < 10^{-6}$, preferably $p < 10^{-9}$. Also, according to certain embodiments, the method further comprises correlating different genetic sites to each other.

A fourth aspect of the present teaching relates to a method of acquiring, respectively determining, an antimicrobial drug, e.g. antibiotic, resistance profile for a clinical isolate of a microorganism, particularly a bacterial microorganism, comprising:

obtaining or providing at least one gene sequence of the clinical isolate of the microorganism, particularly the bacterial microorganism; and

determining the presence of genetic variants in the at least one gene sequence of the clinical isolate of the microorganism, particularly bacterial microorganism, as determined by the method of the first aspect of the teaching.

With this method, antimicrobial drug, e.g. antibiotic, resistances in an unknown isolate of a microorganism, e.g. Staphylococcus aureus, can be determined.

A simple read out concept for a diagnostic test as described in this aspect is shown schematically in Fig. 1.

According to Fig. 1, a sample 1, e.g. blood from a patient, is used for molecular testing 2, e.g. using next generation sequencing (NGS), and then a molecular fingerprint 3 is taken, e.g. in case of NGS a sequence of selected genomic/plasmid regions or the whole genome is assembled. This is then compared to a reference library 4 containing several reference genomes and/or a pan-genome as obtained by the method of the first aspect, i.e. selected sequences or the whole sequence are/is compared to one or more reference sequences and/or a pan-genome, and genetic variations (SNPs, sequence- gene additions/deletions, etc.) are correlated with susceptibility/resistance profile of reference strains in the reference library. The reference library 4 herein contains many genomes and/or a pan-genome and is different from a reference genome. Then the result 5 is reported which can comprise ID (pathogen identification), i.e. a list of all (pathogenic) species identified in the sample, and AST (antimicrobial susceptibility testing), i.e. a list including a susceptibility /resistance profile for all species listed.

According to certain embodiments, statistical analysis in the present method is carried out using Fisher's test with $p < 10^{-6}$, preferably $p < 10^{-9}$. Also, according to certain embodiments, the method further comprises correlating different genetic sites to each other.

Again, in the third and fourth aspect, the different steps can herein be carried out as described with regard to the first aspect of the present teaching, and the microorganism can be a Staphylococcus species, particularly Staphylococcus aureus, according to certain embodiments, and the antibiotic can be methicillin and/or another antibiotic as described below according to certain embodiments. In this regard, it should be noted that resistance to methicillin can indicate, particular in Staphylococcus species, particularly Staphylococcus aureus, resistance to β-lactam antibiotics.

In a fifth aspect the present teaching relates to one or more computer program products comprising computer executable instructions which, when executed, perform a method according to any one of the first to the fourth aspect of the present teaching.

In certain embodiments the computer program product is one on which program commands or program codes of a computer program for executing said method are stored. According to certain embodiments the computer program product is a storage medium. As noted above, the computer program products of the present teaching can be self-learning, e.g. with respect to the first and second data sets.

In order to obtain the best possible information from the highly complex genetic data and develop an optimum model for diagnostic and therapeutical uses as well as the methods of the present teaching - which can be applied stably in clinical routine - a thorough in silico analysis can be necessary. The proposed principle is based on a combination of different approaches, e.g. assembly of the genome of the microorganisms, at least in part and optionally annotating the genomes to one or more reference genomes and/or a pan-genome, or, in the second, third and/or fourth aspect, alignment of the sequence data of the clinical isolate to be determined with one or more reference genomes and/or a pan-genome, and correlation of genetic variations found in every sample, e.g. from each patient, respectively an unknown clinical isolate, with all references and drugs, e.g. antibiotics, or only one or some of them, and search for mutations which occur in one or several drug and one or several strains.

Using the above steps a list of genetic variations as well as of positions with regard to one or more reference genomes and/or a pan-genome is generated. These can be stored in databases and statistical models can be derived from the databases. The statistical models can be based on at least one or more genetic variations in at least one or more positions. Statistical models that can be trained can be combined from genetic variations and positions. Examples of algorithms that can produce such models are association Rules, Support Vector Machines, Decision Trees, Decision Forests, Discriminant-Analysis, Cluster-Methods, and many more.

The goal of the training is to allow a reproducible, standardized application during routine procedures.

For this, for example, a genome or parts of the genome of a microorganism can be sequenced from a patient to be diagnosed. Afterwards, core characteristics can be derived from the sequence data which can be used to predict resistance. These are the points in the database used for the final model, i.e. at least one genetic variation or at least one position, but also combinations of genetic variations, etc.

The corresponding characteristics can be used as input for the statistical model and thus enable a prognosis for new patients. Not only the information regarding all resistances of all microorganisms, e.g. of Staphylococcus aureus, against all or only some or one drugs, e.g. antibiotics, can be integrated in a computer decision support tool, but also corresponding directives (e.g. EUCAST) so that only treatment proposals are made that are in line with the directives.

A tenth aspect of the present teaching relates to the use of the computer program product according to the fifth aspect, e.g. for acquiring an antimicrobial drug, e.g. antibiotic, resistance profile for microorganisms in the fourth aspect of the teaching and/or for use in the diagnostic method of the second method of the teaching and/or for selecting a treatment in the third aspect of the present teaching and/or in the method of the first aspect of the present teaching.

A sixth aspect of the present teaching discloses a diagnostic method of determining an infection of a patient with a Staphylococcus species, particularly Staphylococcus aureus, potentially resistant to antimicrobial drug, e.g. antibiotic, treatment, comprising the steps of:

a) obtaining or providing a sample containing or suspected of containing at least one Staphylococcus, particularly Staphylococcus aureus, strain from the patient;
b) determining the presence of at least one genetic variation in at least two positions from the group of positions annotated with Nos. 1 - 50 with regard to the reference genomes with the genome names given in Table 1, wherein the presence of said at least two genetic variations is indicative of an infection with an antimicrobial drug, e.g. antibiotic, resistant Staphylococcus, particularly Staphylococcus aureus, strain in said patient wherein for some positions more than one position in different reference genomes is annotated.

As noted above, in Table 1, the position of the genetic variation (named "position"; with R being reverse direction and F being forward direction) are given for each variation (given with consecutive numbers 1 - 50) with reference to one or more known reference genomes from the NCBI (with the NCBI number given in the column "reference genome" and the genome name given in the column "genome name").

An infection of a patient with Staphylococcus, particularly Staphylococcus aureus, potentially resistant to antimicrobial drug treatment herein means an infection of a patient with Staphylococcus aureus wherein it is unclear if the Staphylococcus, particularly Staphylococcus aureus, strain is susceptible to treatment with a specific antimicrobial drug or if it is resistant to the antimicrobial drug.

In step b) above, as well as corresponding steps, at least one genetic variation in at least two positions is determined, so that in total at least two genetic variations are determined, wherein the two genetic variations are in different positions. Again, it should be noted that in Table 1 a certain position can be annotated to more than one reference gene, so that also here only different positions are used, and not the same position that is annotated to different reference genomes.

In this method, as well as the other methods of the teaching, the sample can be provided or obtained in any way, preferably non-invasive, and can be e.g. provided as an *in vitro* sample or prepared as *in vitro* sample.

According to certain aspects, genetic variations in at least two, three, four, five, six, seven, eight, nine or ten positions are determined in any of the methods of the present teaching, e.g. in at least two positions or in at least three positions. Instead of testing only single positions, a combination of several variant positions can improve the prediction accuracy and further reduce false positive findings that are influenced by other factors. Therefore, it is in particular preferred to determine the presence of a genetic variation in 2, 3, 4, 5, 6, 7, 8 or 9 (or more) positions selected from Table 1.

For the above positions, i.e. the positions denoted in Table 1, the highest probability of a resistance to at least one antimicrobial drug, e.g. antibiotic, could be observed, with p-values smaller than $10^{-140}$, particularly smaller than $10^{-160}$, indicating the high significance of the values (n= 987; $\alpha = 10^{-9}$). Details regarding Table 1 can be taken from Table 2, respectively Tables 2a and 2b, disclosed in the Examples. Having at least two positions with genetic variations determined, a high probability of an antimicrobial drug, e.g. antibiotic, resistance could be determined. The genes in Table 1 thereby represent the 50 best genes for which a genetic variation was observed in the genomes of Staphylococcus, particularly Staphylococcus aureus, with regard to methicillin resistance/susceptibility as described above and below.

According to certain embodiments, the obtaining or providing a sample containing or suspected of containing at least one Staphylococcus species from the patient in this method - as well as the other methods of the teaching - can comprise the following:

A sample of a vertebrate, e.g. a human, e.g. is provided or obtained and nucleic acid sequences, e.g. DNA or RNA sequences, are recorded by a known method for recording nucleic acid, which is not particularly limited. For example, nucleic acid can be recorded by a sequencing method, wherein any sequencing method is appropriate, particularly sequencing methods wherein a multitude of sample components, as e.g. in a blood sample, can be analyzed for nucleic acids and/or nucleic acid fragments and/or parts thereof contained therein in a short period of time, including the nucleic acids and/or nucleic acid fragments and/or parts thereof of Staphylococcus, particularly Staphylococcus aureus. For example, sequencing can be carried out using polymerase chain reaction (PCR), particularly multiplex PCR, or high throughput sequencing or next generation sequencing, preferably using high-throughput sequencing. For sequencing, preferably an *in vitro* sample is used.

The data obtained by the sequencing can be in any format, and can then be analyzed as described with regard to the first to fourth aspect of the present teaching.

In a seventh aspect, the present teaching relates to a method of selecting a treatment of a patient suffering from an infection with a potentially resistant Staphylococcus, particularly Staphylococcus aureus, strain, comprising the steps of:

a) obtaining or providing a sample containing or suspected of containing at least one Staphylococcus, particularly Staphylococcus aureus, strain from the patient;
b) determining the presence of at least one genetic variation in at least two positions from the group of positions annotated with Nos. 1 - 50 with regard to the reference genomes with the genome names given in Table 1, wherein the presence of said at least two genetic variations is indicative of a resistance to one or more antimicrobial, e.g. antibiotic, drugs, wherein for some positions more than one position in different reference genomes is annotated;
c) identifying said at least one or more antimicrobial, e.g. antibiotic, drugs; and
d) selecting one or more antimicrobial, e.g. antibiotic, drugs different from the ones identified in step c) and being suitable for the treatment of a Staphylococcus, particularly Staphylococcus aureus, infection.

In this method, the steps a) of obtaining or providing a sample and b) of determining the presence of at least one genetic variation are as in the method of the sixth aspect.

The identification of the at least one or more antimicrobial, e.g. antibiotic, drug in step c) is then based on the results obtained in step b) and corresponds to the antimicrobial, e.g. antibiotic, drug(s) that correlate(s) with the genetic variations. Once these antimicrobial drugs, e.g. antibiotics, are ruled out, the remaining antimicrobial drugs, e.g. antibiotic drugs/antibiotics, can be selected in step d) as being suitable for treatment.

In the description, references to the sixth and seventh aspect also apply to the 11th, 12th, 13th and 14th aspect, referring to the same positions, unless clear from the context that they don't apply.

According to certain embodiments of the sixth and or seventh aspect, the antimicrobial drug, e.g. antibiotic, in the method of the sixth or seventh aspectis at least one from the group consisting of β-lactams, β-lactam inhibitors, quinolines and derivatives thereof, e.g. fluoroquinolones, aminoglycosides, glycopeptides, lincosamides, macrolides, nitrofuranes, oxazolidinones polyketides, respectively tetracyclines, and folate synthesis inhibitors, e.g. benzene derived/sulfonamide antibiotics, particularly from the group consisting of Amoxicillin/Clavulanate, Ampicillin, Ampicillin/Sulbactam, Azithromycin, Cefalothin, Cefazolin, Cefepime, Cefotaxime, Cefoxitin, Ceftriaxone, Cefuroxime, Chloramphenicol, Ciprofloxacin, Clindamycin, Daptomycin, Ertapenem, Erythromycin, Fosfomycin, Fusidic acid, Gentamicin, Imipenem, Levofloxacin, Linezolid, Meropenem, Methicillin, Moxifloxacin, Mupirocin, Nitrofurantoin, Norfloxacin, Ofloxacin, Oxacillin, Penicillin G, Piperacillin/Tazobactam, Quinupristin/Dalfopristin, Rifampicin, Teicoplanin, Tetracycline, Tigecycline, Tobramycin, Trimethoprim/Sulfamethoxazole, and Vancomycin, particularly Methicillin.

In the methods of the teaching the resistance of Staphylococcus, particularly Staphylococcus aureus, to one or more antimicrobial, e.g. antibiotic, drugs can be determined according to certain embodiments.

According to certain embodiments of the sixth and/or seventh aspect of the teaching, determining the nucleic acid sequence information or the presence of a genetic variation comprises determining the presence of a single nucleotide at a single position. Thus the teaching comprises methods wherein the presence of a single nucleotide polymorphism or mutation at a single nucleotide position is detected.

According to certain embodiments of the sixth and/or seventh aspect of the teaching, the resistance of a Staphylococcus aureus strain against 1, 2, 3, 4, 5, 6, 7, 8, 9, 10, 11, 12, 13, 14, 15 or 16, 17, 18, 19, 20 or more antibiotic drugs is determined.

According to certain embodiments of the sixth and/or seventh aspect of the teaching, a detected genetic variation is a genetic variation leading to an altered amino acid sequence, e.g. in a polypeptide derived from a respective gene, in which the detected genetic variation is located. According to this aspect, the detected genetic variation can thus lead to a truncated version of the polypeptide (wherein a new stop codon is created by the mutation) or a mutated version of the polypeptide having an amino acid exchange at the respective position.

According to certain embodiments of the sixth and/or seventh aspect of the teaching, determining the nucleic acid sequence information with the positions having a genetic variation or the presence of a genetic variation comprises determining a partial sequence or an entire sequence comprising the position with the genetic variation.

According to certain embodiments of the sixth and/or seventh aspect of the teaching, determining the nucleic acid sequence information with the positions having a genetic variation or the presence of a genetic variation comprises using a next generation sequencing or high throughput sequencing method. According to preferred embodiments of the sixth and/or seventh aspect of the teaching, a partial or entire genome sequence of a Staphylococcus, particularly Staphylococcus aureus, strain is determined by using a next generation sequencing or high throughput sequencing method.

According to certain embodiments of the sixth and/or seventh aspect, determining the nucleic acid sequence information or the presence of a genetic variation comprises determining a partial or entire sequence of the genome of the Staphylococcus species, particularly Staphylococcus aureus, wherein said partial or entire sequence of the genome comprises at least one of the positions with the genetic variation.

An eleventh aspect of the present teaching is directed to a method of treating a patient suffering from an antimicrobial drug, e.g. antibiotic, resistant Staphylococcus, particularly Staphylococcus aureus, infection, comprising the steps of:

a) obtaining or providing a sample containing or suspected of containing at least one Staphylococcus, particularly Staphylococcus aureus, strain from the patient;
b) determining the presence of at least one genetic variation in at least two positions from the group of positions annotated with Nos. 1 - 50 with regard to the reference genomes with the genome names given in Table 1, wherein the presence of said at least two genetic variations is indicative of a resistance to one or more antimicrobial, e.g. antibiotic, drugs, wherein for some positions more than one position in different reference genomes is annotated;
c) identifying said at least one or more antimicrobial, e.g. antibiotic, drugs;
d) selecting one or more antimicrobial, e.g. antibiotic, drugs different from the ones identified in step c) and being suitable for the treatment of a Staphylococcus, particularly Staphylococcus aureus, infection; and
e) treating the patient with said one or more antimicrobial, e.g. antibiotic, drugs.

Herein, steps a) to d) can be carried out as described with respect to the seventh aspect. Step e) can be sufficiently carried out without being restricted and can be done e.g. non-invasively.

A twelfth aspect of the present teaching discloses a diagnostic method of determining an infection of a patient with a Staphylococcus species, particularly Staphylococcus aureus, potentially resistant to antimicrobial drug, e.g. antibiotic, treatment, comprising the steps of:

a) obtaining or providing a sample containing or suspected of containing at least one Staphylococcus, particularly Staphylococcus aureus, strain from the patient;
b) determining the presence of at least one genetic variation in at least one position from the group of positions annotated with Nos. 1 - 50 with regard to the reference genomes with the genome names given in Table 1, wherein the presence of said at least one genetic variation is indicative of an infection with an antimicrobial drug, e.g. antibiotic, resistant Staphylococcus, particularly Staphylococcus aureus, strain in said patient, wherein for some positions more than one position in different reference genomes is annotated.

In a thirteenth aspect, the present teaching relates to a method of selecting a treatment of a patient suffering from an infection with a potentially resistant Staphylococcus, particularly Staphylococcus aureus, strain, comprising the steps of:

a) obtaining or providing a sample containing or suspected of containing at least one Staphylococcus, particularly Staphylococcus aureus, strain from the patient;
b) determining the presence of at least one genetic variation in at least one position from the group of positions annotated with Nos. 1 - 50 with regard to the reference genomes with the genome names given in Table 1, wherein the presence of said at least one genetic variation is indicative of a resistance to one or more antimicrobial, e.g. antibiotic, drugs, wherein for some positions more than one position in different reference genomes is annotated;
c) identifying said at least one or more antimicrobial, e.g. antibiotic, drugs; and
d) selecting one or more antimicrobial, e.g. antibiotic, drugs different from the ones identified in step c) and being suitable for the treatment of a Staphylococcus, particularly Staphylococcus aureus, infection.

Again, in the twelfth and the thirteenth aspect the steps correspond to those in the sixth or seventh aspect, although only a mutation in at least one gene is determined.

A fourteenth aspect of the present teaching is directed to a method of treating a patient suffering from an antimicrobial drug, e.g. antibiotic, resistant Staphylococcus, particularly Staphylococcus aureus, infection, comprising the steps of:

a) obtaining or providing a sample containing or suspected of containing at least one Staphylococcus aureus strain from the patient;

b) determining the presence of at least one genetic variation in at least one position from the group of positions annotated with Nos. 1 - 50 with regard to the reference genomes with the genome names given in Table 1, wherein the presence of said at least one genetic variation is indicative of a resistance to one or more antimicrobial, e.g. antibiotic, drugs, wherein for some positions more than one position in different reference genomes is annotated;

c) identifying said at least one or more antimicrobial, e.g. antibiotic, drugs;

d) selecting one or more antimicrobial, e.g. antibiotic, drugs different from the ones identified in step c) and being suitable for the treatment of a Staphylococcus, particularly Staphylococcus aureus, infection; and

e) treating the patient with said one or more antimicrobial, e.g. antibiotic, drugs.

Also in the fourteenth aspect of the teaching, steps a) to d) are analogous to the steps in the method of the eleventh aspect of the present teaching. Step e) can again be sufficiently carried out without being restricted and can be done e.g. non-invasively.

An eighth aspect of the present teaching discloses a diagnostic method of determining an infection of a patient with a Staphylococcus species, particularly Staphylococcus aureus, potentially resistant to antimicrobial drug, e.g. antibiotic, treatment, comprising the steps of:

a) obtaining or providing a sample containing or suspected of containing at least one Staphylococcus, particularly Staphylococcus aureus, strain from the patient;

b) determining the presence of at least one genetic variation in at least two positions from the group of positions annotated with Nos. 1 - 50 with regard to the reference genomes with the genome names given in Tables 3a and/or 3b, particular with regard to the reference genomes with the genome names given in Table 3b, wherein the presence of said at least two genetic variations is indicative of an infection with an antimicrobial drug, e.g. antibiotic, resistant Staphylococcus, particularly Staphylococcus aureus, strain in said patient wherein for some positions more than one position in different reference genomes is annotated.

As noted above, in Tables 3a and 3b, the position of the genetic variation (named "position"; with R being reverse direction and F being forward direction) are given for each variation (given with consecutive numbers 1 - 50) with reference to one or more known reference genomes from the NCBI (with the NCBI number given in the column "reference genome" and the genome name given in the column "genome name").

An infection of a patient with a Staphylococcus species, particularly Staphylococcus aureus, potentially resistant to antimicrobial drug treatment herein means an infection of a patient with a Staphylococcus species, particularly Staphylococcus aureus, wherein it is unclear if the Staphylococcus species, particularly Staphylococcus aureus, is susceptible to treatment with a specific antimicrobial drug or if it is resistant to the antimicrobial drug.

In step b) above, as well as corresponding steps, at least one genetic variation in at least two positions is determined, so that in total at least two genetic variations are determined, wherein the two genetic variations are in different positions. Again, it should be noted that in Tables 3a and 3b a certain position can be annotated to more than one reference gene, so that also here only different positions are used, and not the same position that is annotated to different reference genomes.

In this method, as well as the other methods of the teaching, the sample can be provided or obtained in any way, preferably non-invasive, and can be e.g. provided as an in *vitro* sample or prepared as *in vitro* sample.

According to certain aspects, genetic variations in at least two, three, four, five, six, seven, eight, nine or ten positions are determined in any of the methods of the present teaching, e.g. in at least two positions or in at least three positions. Instead of testing only single positions, a combination of several variant positions can improve the prediction accuracy and further reduce false positive findings that are influenced by other factors. Therefore, it is in particular preferred to determine the presence of a genetic variation in 2, 3, 4, 5, 6, 7, 8 or 9 (or more) positions selected from Tables 3a and/or 3b.

For the above positions, i.e. the positions denoted in Tables 3a and/or 3b, the highest probability of a resistance to at least one antimicrobial drug, e.g. antibiotic, could be observed, with p-values smaller than $10^{-160}$, particularly smaller than $10^{-190}$, indicating the high significance of the values (n= 985; $\alpha$ = $10^{-9}$). Details regarding Tables 3a and 3b can be taken from Table 4, particularly Tables 4a-d with regard to Table 3a and Tables 4e-h with regard to Table 3b, disclosed in the Examples. Having at least two positions with genetic variations determined, a high probability of an antimicrobial drug, e.g. antibiotic, resistance could be determined. The genes in Table 3a thereby represent the 50 best genes for which a mutation was observed in the genomes of Staphylococcus species, particularly S. aureus, particularly with regard to resistance to the antibiotics described below, i.e. the group consisting of Amoxicillin/Clavulanate, Ampicillin, Ampicillin/Sulbactam, Azithromycin, Cefalothin, Cefazolin, Cefepime, Cefotaxime, Cefoxitin, Ceftriaxone, Cefuroxime, Chloramphenicol, Ciprofloxacin, Clindamycin, Daptomycin, Ertapenem, Erythromycin, Fosfomycin, Fusidic acid, Gentamicin, Imipenem, Levofloxacin, Linezolid, Meropenem, Moxifloxacin, Mupirocin, Nitrofurantoin, Norfloxacin, Ofloxacin, Oxacillin, Penicillin G, Piperacillin/Tazobactam, Quinupristin/Dalfopristin, Rifampicin, Teicoplanin, Tetracycline, Tigecycline, Tobramycin, Trimethoprim/Sulfamethoxazole, and Vancomycin, whereas the genes in Table 3b represent the 50 best genes for which a cross-correlation could be observed for the antimicrobial drug, e.g. antibiotic, susceptibility testing, particularly with regard to resistance to the antibiotics as above with regard to Table 3a, for Staphylococcus species, particularly S. aureus, as described below.

According to certain embodiments, the obtaining or providing a sample containing or suspected of containing at least one Staphylococcus from the patient in this method - as well as the other methods of the teaching - can comprise the following:

A sample of a vertebrate, e.g. a human, e.g. is provided or obtained and nucleic acid sequences, e.g. DNA or RNA sequences, are recorded by a known method for recording nucleic acid, which is not particularly limited. For example, nucleic acid can be recorded by a sequencing method, wherein any sequencing method is appropriate, particularly sequencing methods wherein a multitude of sample components, as e.g. in a blood sample, can be analyzed for nucleic acids and/or nucleic acid fragments and/or parts thereof contained therein in a short period of time, including the nucleic acids and/or nucleic acid fragments and/or parts thereof of the Staphylococcus species, particularly Staphylococcus aureus. For example, sequencing can be carried out using polymerase chain reaction (PCR), particularly multiplex PCR, or high throughput sequencing or next generation sequencing, preferably using high-throughput sequencing. For sequencing, preferably an *in vitro* sample is used.

The data obtained by the sequencing can be in any format, and can then be analyzed as described with regard to the first to fourth aspect of the present teaching.

In a ninth aspect, the present teaching relates to a method of selecting a treatment of a patient suffering from an infection with a potentially resistant Staphylococcus, particularly Staphylococcus aureus, strain, comprising the steps of:

> a) obtaining or providing a sample containing or suspected of containing at least one Staphylococcus, particularly Staphylococcus aureus, strain from the patient;
> b) determining the presence of at least one genetic variation in at least two positions from the group of positions annotated with Nos. 1 - 50 with regard to the reference genomes with the genome names given in Tables 3a and/or 3b, particular with regard to the reference genomes with the genome names given in Table 3b, wherein the presence of said at least two genetic variations is indicative of a resistance to one or more antimicrobial, e.g. antibiotic, drugs, wherein for some positions more than one position in different reference genomes is annotated;
> c) identifying said at least one or more antimicrobial, e.g. antibiotic, drugs; and
> d) selecting one or more antimicrobial, e.g. antibiotic, drugs different from the ones identified in step c) and being suitable for the treatment of a Staphylococcus, particularly Staphylococcus aureus, infection.

In this method, the steps a) of obtaining or providing a sample and b) of determining the presence of at least one genetic variation are as in the method of the eighth aspect.

The identification of the at least one or more antimicrobial, e.g. antibiotic, drug in step c) is then based on the results obtained in step b) and corresponds to the antimicrobial, e.g. antibiotic, drug(s) that correlate(s) with the genetic variations. Once these antimicrobial drugs, e.g. antibiotics, are ruled out, the remaining antimicrobial drugs, e.g. antibiotic drugs/antibiotics, can be selected in step d) as being suitable for treatment.

In the description, references to the eighth and ninth aspect also apply to the 15th, 16th, 17th and 18th aspect, referring to the same positions, unless clear from the context that they don't apply.

According to certain embodiments of the eighth and/or ninth aspect, the antimicrobial drug, e.g. antibiotic, in the method of the eighth or ninth aspect, as well as in the other methods of the teaching, is at least one from the group consisting of β-lactams, β-lactam inhibitors, quinolines and derivatives thereof, e.g. fluoroquinolones, aminoglycosides, glycopeptides, lincosamides, macrolides, nitrofuranes, oxazolidinones polyketides, respectively tetracyclines, and folate synthesis inhibitors, e.g. benzene derived/sulfonamide antibiotics, particularly from the group consisting of Amoxicillin/Clavulanate, Ampicillin, Ampicillin/Sulbactam, Azithromycin, Cefalothin, Cefazolin, Cefepime, Cefotaxime, Cefoxitin, Ceftriaxone, Cefuroxime, Chloramphenicol, Ciprofloxacin, Clindamycin, Daptomycin, Ertapenem, Erythromycin, Fosfomycin, Fusidic acid, Gentamicin, Imipenem, Levofloxacin, Linezolid, Meropenem, Methicillin, Moxifloxacin, Mupirocin, Nitrofurantoin, Norfloxacin, Ofloxacin, Oxacillin, Penicillin G, Piperacillin/Tazobactam, Quinupristin/Dalfopristin, Rifampicin, Teicoplanin, Tetracycline, Tigecycline, Tobramycin, Trimethoprim/Sulfamethoxazole, and Vancomycin. In the eighth and/or ninth aspect, as well as the fifteenth to eighteenth aspect, , the antimicrobial drug, e.g. antibiotic is preferably at least one from the group consisting of β-lactams, β-lactam inhibitors, quinolines and derivatives thereof, e.g. fluoroquinolones, aminoglycosides, glycopeptides, lincosamides, macrolides, nitrofuranes, oxazolidinones polyketides, respectively tetracyclines, and folate synthesis inhibitors, e.g. benzene derived/sulfonamide antibiotics, particularly from the group consisting of Amoxicillin/Clavulanate, Ampicillin, Ampicillin/Sulbactam, Azithromycin, Cefalothin, Cefazolin, Cefepime, Cefotaxime, Cefoxitin, Ceftriaxone, Cefuroxime, Chloramphenicol, Ciprofloxacin, Clindamycin, Daptomycin, Ertapenem, Erythromycin, Fosfomycin, Fusidic acid, Gentamicin, Imipenem, Levofloxacin, Linezolid, Meropenem, Moxifloxacin, Mupirocin, Nitrofurantoin, Norfloxacin, Ofloxacin, Oxacillin, Penicillin G, Piperacillin/Tazobactam, Quinupristin/Dalfopristin, Rifampicin, Teicoplanin, Tetracycline, Tigecycline, Tobramycin, Trimethoprim/Sulfamethoxazole, and Vancomycin.

In the methods of the teaching the resistance of a Staphylococcus species, particularly Staphylococcus aureus, to one or more antimicrobial, e.g. antibiotic, drugs can be determined according to certain embodiments.

According to certain embodiments of the eighth and/or ninth aspect of the teaching, determining the nucleic acid sequence information or the presence of a genetic variation comprises determining the presence of a single nucleotide at a single position. Thus the teaching comprises methods wherein the presence of a single nucleotide polymorphism or mutation at a single nucleotide position is detected.

According to certain embodiments of the eighth and/or ninth aspect of the teaching, the resistance of a Staphylococcus, particularly Staphylococcus aureus, strain against 1, 2, 3, 4, 5, 6, 7, 8, 9, 10, 11, 12, 13, 14, 15 or 16, 17, 18, 19, 20 or more antibiotic drugs is determined.

According to certain embodiments of the eighth and/or ninth aspect of the teaching, a detected genetic variation is a genetic variation leading to an altered amino acid sequence, e.g. in a polypeptide derived from a respective gene, in which the detected genetic variation is located. According to this aspect, the detected genetic variation can thus lead to a truncated version of the polypeptide (wherein a new stop codon is created by the mutation) or a mutated version of the polypeptide having an amino acid exchange at the respective position.

According to certain embodiments of the eighth and/or ninth aspect of the teaching, determining the nucleic acid sequence information with the positions having a genetic variation or the presence of a genetic variation comprises determining a partial sequence or an entire sequence comprising the position with the genetic variation.

According to certain embodiments of the eighth and/or ninth aspect of the teaching, determining the nucleic acid sequence information with the positions having a genetic variation or the presence of a genetic variation comprises using a next generation sequencing or high throughput sequencing method. According to preferred embodiments of the eighth and/or ninth aspect of the teaching, a partial or entire genome sequence of a Staphylococcus, particularly Staphylococcus aureus, strain is determined by using a next generation sequencing or high throughput sequencing method.

According to certain embodiments of the eighth and/or ninth aspect, determining the nucleic acid sequence information or the presence of a genetic variation comprises determining a partial or entire sequence of the genome of the Staphylococcus species, particularly Staphylococcus aureus, wherein said partial or entire sequence of the genome comprises at least one of the positions with the genetic variation.

According to certain embodiments of the eighth and/or ninth aspect of the teaching, as well as 15th, 16th, 17th and/or 18th aspect, the position is from Table 3a, and the antibiotic class is at least one of the ones (column: sign_phenos_class) given for the respective position in Table 4a and/or the antibiotic is at least one of the ones (column: sign_phenos) given for the respective position in Table 4a.

According to certain embodiments of the eighth and/or ninth aspect of the teaching, as well as 15th, 16th, 17th and/or 18th aspect, the position is from Table 3a, and at least one antibiotic is from the antibiotic class (column: best_pheno_class) given for the respective position in Table 4d and/or at least one antibiotic is the antibiotic (column: best_pheno) given for the respective position in Table 4d.

According to certain embodiments of the eighth and/or ninth aspect of the teaching, as well as 15th, 16th, 17th and/or 18th aspect, the position is from Table 3b, and the antibiotic class is at least one of the ones (column: sign_phenos_class) given for the respective position in Table 4e and/or the antibiotic is at least one of the ones (column: sign_phenos) given for the respective position in Table 4e.

According to certain embodiments of the eighth and/or ninth aspect of the teaching, as well as 15th, 16th, 17th and/or 18th aspect, the position is from Table 3b, and at least one antibiotic is from the antibiotic class (column: best_pheno_class) given for the respective position in Table 4h and/or at least one antibiotic is the antibiotic (column: best_pheno) given for the respective position in Table 4h.

A fifteenth aspect of the present teaching is directed to a method of treating a patient suffering from an antimicrobial drug, e.g. antibiotic, resistant Staphylococcus, particularly Staphylococcus aureus, infection, comprising the steps of:

a) obtaining or providing a sample containing or suspected of containing at least one Staphylococcus, particularly Staphylococcus aureus, strain from the patient;
b) determining the presence of at least one genetic variation in at least two positions from the group of positions annotated with Nos. 1 - 50 with regard to the reference genomes with the genome names given in Tables 3a and/or 3b, particular with regard to the reference genomes with the genome names given in Table 3b, wherein the presence of said at least two genetic variations is indicative of a resistance to one or more antimicrobial, e.g. antibiotic, drugs, wherein for some positions more than one position in different reference genomes is annotated;
c) identifying said at least one or more antimicrobial, e.g. antibiotic, drugs;
d) selecting one or more antimicrobial, e.g. antibiotic, drugs different from the ones identified in step c) and being suitable for the treatment of the Staphylococcus, particularly Staphylococcus aureus, infection; and
e) treating the patient with said one or more antimicrobial, e.g. antibiotic, drugs.

Herein, steps a) to d) can be carried out as described with respect to the ninth aspect. Step e) can be sufficiently carried out without being restricted and can be done e.g. non-invasively.

A sixteenth aspect of the present teaching discloses a diagnostic method of determining an infection of a patient with a Staphylococcus species, particularly Staphylococcus aureus, potentially resistant to antimicrobial drug, e.g. antibiotic, treatment, comprising the steps of:

a) obtaining or providing a sample containing or suspected of containing at least one Staphylococcus, particularly Staphylococcus aureus, strain from the patient;
b) determining the presence of at least one genetic variation in at least one position from the group of positions annotated with Nos. 1 - 50 with regard to the reference genomes with the genome names given in Tables 3a and/or 3b, particular with regard to the reference genomes with the genome names given in Table 3b, wherein the presence of said at least one genetic variation is indicative of an infection with an antimicrobial drug, e.g. antibiotic, resistant Staphylococcus, particularly Staphylococcus aureus, strain in said patient, wherein for some positions more than one position in different reference genomes is annotated.

In a seventeenth aspect, the present teaching relates to a method of selecting a treatment of a patient suffering from an infection with a potentially resistant Staphylococcus, particularly Staphylococcus aureus, strain, comprising the steps of:

a) obtaining or providing a sample containing or suspected of containing at least one Staphylococcus, particularly Staphylococcus aureus, strain from the patient;
b) determining the presence of at least one genetic variation in at least one position from the group of positions annotated with Nos. 1 - 50 with regard to the reference genomes with the genome names given in Tables 3a and/or 3b, particular with regard to the reference genomes with the genome names given in Table 3b, wherein the presence of said at least one genetic variation is indicative of a resistance to one or more antimicrobial, e.g. antibiotic, drugs, wherein for some positions more than one position in different reference genomes is annotated;
c) identifying said at least one or more antimicrobial, e.g. antibiotic, drugs; and
d) selecting one or more antimicrobial, e.g. antibiotic, drugs different from the ones identified in step c) and being suitable for the treatment of a Staphylococcus, particularly Staphylococcus aureus, infection.

Again, in the sixteenth and the seventeenth aspect the steps correspond to those in the eighth or ninth aspect, although only a mutation in at least one gene is determined.

An eighteenth aspect of the present teaching is directed to a method of treating a patient suffering from an antimicrobial drug, e.g. antibiotic, resistant Staphylococcus, particularly Staphylococcus aureus, infection, comprising the steps of:

a) obtaining or providing a sample containing or suspected of containing at least one Staphylococcus, particularly Staphylococcus aureus, strain from the patient;
b) determining the presence of at least one genetic variation in at least one position from the group of positions annotated with Nos. 1 - 50 with regard to the reference genomes with the genome names given in Tables 3a and/or 3b, particular with regard to the reference genomes with the genome names given in Table 3b, wherein the presence of said at least one genetic variation is indicative of a resistance to one or more antimicrobial, e.g. antibiotic, drugs, wherein for some positions more than one position in different reference genomes is annotated;
c) identifying said at least one or more antimicrobial, e.g. antibiotic, drugs;
d) selecting one or more antimicrobial, e.g. antibiotic, drugs different from the ones identified in step c) and being suitable for the treatment of a Staphylococcus, particularly Staphylococcus aureus, infection; and
e) treating the patient with said one or more antimicrobial, e.g. antibiotic, drugs.

Also in the eighteenth aspect of the teaching, steps a) to d) are analogous to the steps in the method of the fifteenth aspect of the present invention. Step e) can again be sufficiently carried out without being restricted and can be done e.g. non-invasively.

Examples

The present teaching will now be described in detail with reference to several examples thereof. However, these examples are illustrative and do not limit the scope of the teaching.

Example 1: Determination of genetic resistance profile for MRSA/MSSA phenotype

Whole genome sequencing was carried out in addition to classical antimicrobial susceptibility testing of the same isolates for a cohort of 1001 specimens of S. aureus, of which 995 had an assembly and 987 had an assembly and an MRSA/MSSA phenotype. These 987 samples were used for further analysis. The whole genome sequencing allowed performing genome wide correlation studies to find genetic variants (e.g. point mutations, small insertions and deletion, larger structural variants, plasmid copy number gains, gene dosage effects) in the genome and plasmids that are significantly correlated to the resistance against one or several drugs. The approach also allowed for comparing the relevant sites in the genome to each other.

In the approach the different sources of genetic resistance as well as the different ways of how bacteria can become resistant were covered. By measuring clinical isolates collected in a broad geographical area and across a broad time span of three decades a complete picture going far beyond the rather artificial step of laboratory generated resistance mechanisms was tried to be generated.

The detailed procedure is given in the following:

Bacterial Strains

The inventors selected 1001 specimens of S. aureus from the microbiology strain collection at Siemens Healthcare Diagnostics (West Sacramento, CA) for susceptibility testing and whole genome sequencing, of which 987 were further analyzed, as stated above. To include data on the different ways how resistance mechanisms are acquired Staphylococcus aureus isolates collected over more than three decades were analyzed such that also horizontal gene transfer could potentially be discovered.

Determination of Methicillin resistance / susceptibility MRSA and MSSA strains were determined by culturing according to standard procedures, determining the phenotype of the strains, and confirmed by further tests using e.g. the genetic information.

DNA extraction

DNA extraction and purification was carried out using the MagAttract HMW DNA Kit (Qiagen) procedure with the following changes. After up to $2\times10^9$ bacteria (1ml culture) were centrifuged in a 2 ml tube (10 min, 5000 $\times$ g) and the supernatant was discharged, it was again centrifuged 1 min and the sample was taken. The resulting pellet was dispersed in 160 $\mu$l P1, 20 $\mu$l lysozyme (100 mg/ml) and 4 $\mu$l lysostaphin were added and mixed, and the suspension was incubated at 37°C at 900 rpm for 30 mins in a thermal mixer. Afterwards 300 $\mu$l lysis buffer and proteinase K (30 $\mu$l) (both from the blood kit for Maxwell of Promega) were added and the whole again incubated for 30 mins at 56°C and 900 rpm. The samples as a whole (~ 510 $\mu$l) were then transferred to the Maxwell cartridges for further processing, using the Tissue LEV Total RNA Kit AS1220 or the XAS1220 Custom Kit (Promega).

Next Generation Sequencing

Prior to library preparation, quality control of isolated bacterial DNA was conducted using a Qubit 2.0 Fluorometer (Qubit dsDNA BR Assay Kit, Life Technologies) and an Agilent 2200 TapeStation (Genomic DNA ScreenTape, Agilent Technologies). NGS libraries were prepared in 96 well format using NexteraXT DNA Sample Preparation Kit and NexteraXT Index Kit for 96 Indexes (Illumina) according to the manufacturer's protocol. The resulting sequencing libraries were quantified in a qPCR-based approach using the KAPA SYBR FAST qPCR MasterMix Kit (Peqlab) on a ViiA 7 real time PCR system (Life Technologies). 96 samples were pooled per lane for paired-end sequencing (2x 100bp) on Illumina Hiseq2000 or Hiseq2500 sequencers using TruSeq PE Cluster v3 and TruSeq SBS v3 sequncing chemistry (Illumina). Basic sequencing quality parameters were determined using the FastQC quality control tool for high throughput sequence data (Babraham Bioinformatics Institute).

Data analysis

Trimmomatic (version 0.32, Bolger AM, Lohse M, Usadel B. Trimmomatic: a flexible trimmer for Illumina sequence data. Bioinformatics. 2014;30(15):2114-2120. doi:10.1093/bioinformatics/btu170) was used for adapter and quality trimming of raw reads with following parameters ILLUMINACLIP:NexteraPE-PE.fa:1:50:30 LEADING:3 TRAILING:3 SLIDINGWINDOW:4:15 MINLEN:36. *De novo* assemblies were constructed using SPAdes (version 3.0.0, Bankevich A, Nurk S, Antipov D, et al. SPAdes: A New Genome Assembly Algorithm and Its Applications to Single-Cell Sequencing. Journal of Computational Biology. 2012;19(5):455-477. doi:10.1089/cmb.2012.0021) with parameters -t 20 -m 256 -k 21,33,55,77 --careful -1 fp.fastq.gz -2 rp.fastq.gz. To determine the quality of the assemblies we ran QUAST (version 2.3) with minimal length threshold of 500 bp. Resulting metric values not matching the RefSeq assembly quality criteria (N50 > 5000, L50 < 20, # contigs < 1000) were highlighted.

SNP calling:

Reference-free SNP calling was performed using tool kSNP3 which applies k-mer analysis, i.e. the tool considers all possible k-mers found in given data. The central base of a k-mer is the SNP (example k=21 "AAAGTTTCGCAGTTGGTAATA", SNP=A), the bases on its left and right site are SNP's context.

- Tool URL: http://sourceforge.net/projects/ksnp/files/

The following input was used:

- 49 finished S. aureus genomes from NCBI including the chromosome and available plasmids
- 995 S. aureus de novo assemblies (see above)
- In total: 995+49=1044 samples

The finished genomes were used to choose the parameter k, the chosen value was 21, as determined by the tool.

SNP calling results:

The output contained 487,415 SNPs annotated using given finished genomes resulting in 9,419,797 annotations in total.
SNPs can have following values: bases A/T/C/G or "-" (missing), the latter means, that the considered genomic part is missing (e.g. gene absence). With the results, the annotations used in Table 2 were obtained along with other annotations (see Table 2 and annotations for details)

Extracting SNP annotations:

To reduce the number of similar annotations they were filtered and aggregated as follows:

- Only annotations for which the considered SNP lies on a protein were kept
- Only annotations whose "product" entry and "note" entry do not contain "hypothetical protein" were kept
- Annotations were sorted by SNP ID ("LocusNum") and gene product ("product")
- For each unique pair of SNP ID and gene product only the first line was kept

Association testing:

In addition, the following SNPs were not considered:

- SNPs without any annotation or SNPs whose all annotations contained a flag "synonymous" were not considered → only SNPs with at least one non-synonymous annotation were considered
- Constant SNPs, i.e. same value for all samples were also not considered
- Almost constant SNPs: SNPs whose most frequent value had a frequency >= 95%, i.e. min. 95% of all samples have the same SNP value, were not considered as well
- SNPs with missing value ("-") for more than 10% of samples were also removed

In total 14,856 SNPs were kept for association tests. Fisher's exact two-sided test was applied with subsequent p-value adjustment using FDR and p-value threshold of $10^{-9}$. In total 7,925 SNPs have a significant adjusted p-value, 7101 of them have at least one annotation.

Annotation:

Annotation of the found SNPs was carried out using 49 reference genomes available at NCBI, with the names of the genomes and the reference sequence ID at NCBI (for chromosomes; respectively plasmids) given in the following Table 5.

| Genome name | RefSeq ID Chr; plasmids |
|---|---|
| 04_02981 | NC_017340.fna; |
| 08BA02176 | NC_018608.fna; |
| 11819_97 | NC_017351.fna;NC_017350.fna |
| 55 2053 | NC_022113.fna;NC_022126.fna |
| 6850 | NC_022222.fna; |
| 71193 | NC_017673.fna; |
| Bmb9393 | NC_021670.fna;NC_021657.fna |
| CC45 | NC_021554.fna;NC_021552.fna |
| CN1 | NC_022226.fna;NC_022227.fna, NC_022228.fna |
| COL | NC_002951.fna;NC_006629.fna |
| ECT_R_2 | NC_017343.fna;NC_017346.fna, NC_017344.fna |
| ED133 | NC_017337.fna; |
| ED98 | NC_013450.fna;NC_013451.fna, NC_013452.fna, NC_013453.fna |
| HO_5096_0412 | NC_017763.fna; |
| JH1 | NC_009632.fna;NC_009619.fna |
| JH9 | NC_009487.fna;NC_009477.fna |
| JKD6008 | NC_017341.fna; |
| JKD6159 | NC_017338.fna;NC_017339.fna |
| LGA251 | NC_017349.fna;NC_017348.fna |
| M013 | NC_016928.fna; |
| M1 | NC_021059.fna;NC_021060.fna |
| MRSA252 | NC_002952.fna; |
| MSHR1132 | NC_016941.fna;NC_016942.fna |
| MSSA476 | NC_002953.fna;NC_005951.fna |
| Mu3 | NC_009782.fna; |
| Mu50 | NC_002758.fna;NC_002774.fna |
| MW2 | NC_003923.fna; |
| N315 | NC_002745.fna;NC_003140.fna |
| NCTC 8325 | NC_007795.fna; |
| Newman | NC_009641.fna; |
| RF122 | NC_007622.fna; |
| SA40 | NC_022443.fna; |
| SA957 | NC_022442.fna; |
| ST228_10388 | NC_020529.fna;NC_020530.fna |
| ST228 10497 | NC_020564.fna;NC_020531.fna |
| ST228 15532 | NC_020532.fna;NC_020565.fna |
| ST228_16035 | NC_020533.fna;NC_020534.fna |
| ST228 18412 | NC_020537.fna;NC_020538.fna |

(continued)

| Genome name | RefSeq ID Chr; plasmids |
|---|---|
| ST398 | NC_017333.fna;NC_017335.fna, NC_017334.fna, NC_017336.fna |
| T0131 | NC_017347.fna; |
| TCH60 | NC_017342.fna;NC_017345.fna |
| TW20 | NC_017331.fna;NC_017332.fna, NC_017352.fna |
| uid193758 | NC_020566.fna;NC_020535.fna |
| uid193759 | NC_020536.fna;NC_020567.fna |
| uid193761 | NC_020568.fna;NC_020539.fna |
| USA300 FPR3757 | NC_007793.fna;NC_007792.fna, NC_007791.fna, NC_007790.fna |
| USA300 TCH1516 | NC_010079.fna;NC_010063.fna, NC_012417.fna |
| VC40 | NC_016912.fna; |
| Z172 | NC_022604.fna;NC_022610.fna, NC_022605.fna |

From the data, the 50 genes with the best p-value were chosen for the list of genetic variants with regard to methicillin resistance.

A full list of all positions, p-values, affected genes etc. is provided in Table 2, respectively Tables 2a and 2b, which corresponds to Table 1, and represents the genes having the lowest p-values after correlating the genetic variations with antibiotic resistance.

In Table 2, respectively Tables 2a and 2b, the positions are numbered according to the best p-value results, ranging from 1 to 50. Further, the positions are also annotated with regard to one or more reference genomes of the 49 finished S. aureus genomes from NCBI, wherein the found reference genomes are the following as annotated at the NCBI:

NC_017340, NC_010079, NC_022222, NC_021670, NC_017351,
NC_002953, NC_017337, NC_018608, NC_007795, NC_021059,
NC_021554, NC_016912, NC_022226, NC_022113
http://www.genome.jp/dbget-bin/www_bget?refseq+NC_017340
http://www.genome.jp/dbget-bin/www_bget?refseq+NC_010079
http://www.genome.jp/dbget-bin/www_bget?refseq+NC_022222
http://www.genome.jp/dbget-bin/www_bget?refseq+NC_021670
http://www.genome.jp/dbget-bin/www_bget?refseq+NC_017351
http://www.genome.jp/dbget-bin/www_bget?refseq+NC_002953
http://www.genome.jp/dbget-bin/www_bget?refseq+NC_017337
http://www.genome.jp/dbget-bin/www_bget?refseq+NC_018608
http://www.genome.jp/dbget-bin/www_bget?refseq+NC_007795
http://www.genome.jp/dbget-bin/www_bget?refseq+NC_021059
http://www.genome.jp/dbget-bin/www_bget?refseq+NC_021554
http://www.genome.jp/dbget-bin/www_bget?refseq+NC_016912
http://www.genome.jp/dbget-bin/www_bget?refseq+NC_022226
http://www.genome.jp/dbget-bin/www_bget?refseq+NC_022113

In Table 2, respectively Tables 2a and 2b, more than one annotation per SNP is possible for various positions with regard to the reference genomes for the following reason: the SNPs were annotated using all given finished genomes, thus a SNP may have multiple annotations even after the annotation aggregation, which was mentioned above. The reasons why a SNP may have more than one annotation after the aggregation can be as follows:

◦ The gene products have very similar but not equal information, e.g. "potassium-transporting ATPase A chain" and "potassium-transporting ATPase subunit A". In this case it may be not possible to apply a straightforward approach to remove such duplicates.
◦ The annotations may differ in the genes/gene products, then it may be not possible not say which of the annotations is the correct one.

Table 2a: List of positions (corresponding to Table 1)

| No. | p-value (FDR) | GenomeName | fasta_header | SNPPositioninGenome | gene |
|---|---|---|---|---|---|
| 1 | 2,8455E-163 | 04_02981 | gi\|387149188\|ref\|NC_017340.1\| | 534953 F | |
| | | USA300_TCH1516 | gi\|161508266\|ref\|NC_010079.1\| | 543821 F | |
| 2 | 2,8455E-163 | 6850 | gi\|537441500\|ref\|NC_022222.1\| | 210528 R | |
| | | 04_02981 | gi\|387149188\|ref\|NC_017340.1\| | 267448 R | |
| | | USA300_TCH1516 | gi\|161508266\|ref\|NC_010079.1\| | 269814 R | |
| 3 | 3,348E-163 | 04_02981 | gi\|387149188\|ref\|NC_017340.1\| | 1362060 F | |
| 4 | 3,348E-163 | Bmb9393 | gi\|521210823\|ref\|NC_021670.1\| | 1252703 R | |
| | | 11819_97 | gi\|385780298\|ref\|NC_017351.1\| | 1520285 F | |
| | | 04_02981 | gi\|387149188\|ref\|NC_017340.1\| | 1523326 F | |
| 5 | 3,5068E-163 | 04_02981 | gi\|387149188\|ref\|NC_017340.1\| | 1619285 R | |
| | | USA300_TCH1516 | gi\|161508266\|ref\|NC_010079.1\| | 1661238 R | |
| 6 | 4,5499E-163 | 04_02981 | gi\|387149188\|ref\|NC_017340.1\| | 1641150 R | |
| 7 | 4,5499E-163 | MSSA476 | gi\|49484912\|ref\|NC_002953.3\| | 170059 F | |
| | | ED133 | gi\|384546269\|ref\|NC_017337.1\| | 142263 F | |
| 8 | 4,5499E-163 | 04_02981 | gi\|387149188\|ref\|NC_017340.1\| | 517571 F | |
| | | 08BA02176 | gi\|404477334\|ref\|NC_018608.1\| | 554542 F | |
| 9 | 6,182E-163 | 04_02981 | gi\|387149188\|ref\|NC_017340.1\| | 978538 F | |
| 10 | 6,182E-163 | 04_02981 | gi\|387149188\|ref\|NC_017340.1\| | 1434811 R | |
| 11 | 6,182E-163 | 6850 | gi\|537441500\|ref\|NC_022222.1\| | 953696 R | |
| | | USA300_TCH1516 | gi\|161508266\|ref\|NC_010079.1\| | 1010027 R | rluA1 |
| 12 | 6,182E-163 | 04_02981 | gi\|387149188\|ref\|NC_017340.1\| | 208285 R | argC |
| | | NCTC_8325 | gi\|88193823\|ref\|NC_007795.1\| | 161011 R | argC |

(continued)

| No. | p-value (FDR) | GenomeName | fasta_header | SNPPositioninGenome | gene |
|---|---|---|---|---|---|
| 13 | 6,182E-163 | 11819_97 | gi\|385780298\|ref\|NC_017351.1\| | 2179136 R | |
| | | 04_02981 | gi\|387149188\|ref\|NC_017340.1\| | 2149064 R | |
| | | 08BA02176 | gi\|404477334\|ref\|NC_018608.1\| | 2107689 R | |
| 14 | 6,182E-163 | 04_02981 | gi\|387149188\|ref\|NC_017340.1\| | 2358535 F | |
| 15 | 6,182E-163 | 04_02981 | gi\|387149188\|ref\|NC_017340.1\| | 2023012 R | |
| 16 | 6,182E-163 | NCTC_8325 | gi\|88193823\|ref\|NC_007795.1\| | 2777211 F | |
| | | 04_02981 | gi\|387149188\|ref\|NC_017340.1\| | 2779170 F | |
| 17 | 6,182E-163 | 08BA02176 | gi\|404477334\|ref\|NC_018608.1\| | 1801995 R | |
| | | 04_02981 | gi\|387149188\|ref\|NC_017340.1\| | 1790672 R | |
| 18 | 7,2093E-163 | 04_02981 | gi\|387149188\|ref\|NC_017340.1\| | 976788 F | |
| | | NCTC_8325 | gi\|88193823\|ref\|NC_007795.1\| | 878040 F | |
| 19 | 7,2093E-163 | Bmb9393 | gi\|521210823\|ref\|NC_021670.1\| | 2101899 R | |
| | | 04_02981 | gi\|387149188\|ref\|NC_017340.1\| | 1972149 R | |
| 20 | 1,1926E-162 | 6850 | gi\|537441500\|ref\|NC_022222.1\| | 1875550 F | |
| | | USA300_TCH1516 | gi\|161508266\|ref\|NC_010079.1\| | 2006001 F | bcp |
| | | 04 02981 | gi\|387149188\|ref\|NC_017340.1\| | 1959494 F | |
| 21 | 1,2012E-162 | 04 02981 | gi\|387149188\|ref\|NC_017340.1\| | 705667 R | |
| 22 | 1,2012E-162 | USA300_TCH1516 | gi\|161508266\|ref\|NC_010079.1\| | 2268723 F | |
| | | 04_02981 | gi\|387149188\|ref\|NC_017340.1\| | 2221448 F | |
| 23 | 1,2797E-162 | 04_02981 | gi\|387149188\|ref\|NC_017340.1\| | 1814108 R | |
| 24 | 2,0719E-162 | 04_02981 | gi\|387149188\|ref\|NC_017340.1\| | 531649 R | |
| | | 11819_97 | gi\|385780298\|ref\|NC_017351.1\| | 531398 R | |
| 25 | 2,0719E-162 | 04_02981 | gi\|387149188\|ref\|NC_017340.1\| | 1754561 F | |
| | | NCTC_8325 | gi\|88193823\|ref\|NC_007795.1\| | 1691742 F | |

(continued)

| No. | p-value (FDR) | GenomeName | fasta_header | SNPPositioninGenome | gene |
|---|---|---|---|---|---|
| 26 | 2,0719E-162 | 04_02981 | gi\|387149188\|ref\|NC_017340.1\| | 1958403 R | |
| | | USA300_TCH1516 | gi\|161508266\|ref\|NC_010079.1\| | 2004910 R | |
| 27 | 2,2505E-162 | 6850 | gi\|537441500\|ref\|NC_022222.1\| | 1242653 R | |
| | | USA300_TCH1516 | gi\|161508266\|ref\|NC_010079.1\| | 1294527 R | ribC |
| | | 04_02981 | gi\|387149188\|ref\|NC_017340.1\| | 1299554 R | |
| 28 | 2,5515E-162 | 04_02981 | gi\|387149188\|ref\|NC_017340.1\| | 2590222 R | |
| | | USA300 TCH1516 | gi\|161508266\|ref\|NC_010079.1\| | 2637689 R | gntP |
| 29 | 8,302E-162 | USA300_TCH1516 | gi\|161508266\|ref\|NC_010079.1\| | 1881161 R | fmtB1 |
| | | M1 | gi\|479328021\|ref\|NC_021059.1\| | 1871101 R | |
| | | 6850 | gi\|537441500\|ref\|NC_022222.1\| | 1759861 R | |
| | | 08BA02176 | gi\|404477334\|ref\|NC_018608.1\| | 1855493 R | |
| | | CC45 | gi\|514064966\|ref\|NC_021554.1\| | 1858794 R | |
| | | Bmb9393 | gi\|521210823\|ref\|NC_021670.1\| | 1964828 R | |
| 30 | 1,7865E-161 | NCTC_8325 | gi\|88193823\|ref\|NC_007795.1\| | 1050123 R | |
| | | 04_02981 | gi\|387149188\|ref\|NC_017340.1\| | 1147277 R | |
| 31 | 9,7283E-147 | VC40 | gi\|379013365\|ref\|NC_016912.1\| | 2005634 F | |
| | | 6850 | gi\|537441500\|ref\|NC_022222.1\| | 2039052 F | |
| | | USA300_TCH1516 | gi\|161508266\|ref\|NC_010079.1\| | 2187801 F | rsbU |
| 32 | 2,0972E-146 | 6850 | gi\|537441500\|ref\|NC_022222.1\| | 350202 F | |
| | | 04_02981 | gi\|387149188\|ref\|NC_017340.1\| | 402479 F | |
| | | NCTC_8325 | gi\|88193823\|ref\|NC_007795.1\| | 352104 F | |
| 33 | 6,9539E-146 | M1 | gi\|479328021\|ref\|NC_021059.1\| | 920768 F | rocD |
| | | 08BA02176 | gi\|404477334\|ref\|NC_018608.1\| | 956878 F | rocD |
| | | 04_02981 | gi\|387149188\|ref\|NC_017340.1\| | 956978 F | rocD |
| | | NCTC_8325 | gi\|88193823\|ref\|NC_007795.1\| | 858255 F | rocD |

(continued)

| No. | p-value (FDR) | GenomeName | fasta_header | SNPPositioninGenome | gene |
|---|---|---|---|---|---|
| 34 | 8,7237E-146 | 04_02981 | gi\|387149188\|ref\|NC_017340.1\| | 1121847 R | |
| | | NCTC_8325 | gi\|88193823\|ref\|NC_007795.1\| | 1024692 R | |
| 35 | 1,2342E-145 | 04_02981 | gi\|387149188\|ref\|NC_017340.1\| | 429303 F | |
| 36 | 2,7651E-145 | USA300_TCH1516 | gi\|161508266\|ref\|NC_010079.1\| | 1812380 R | dnaE2 |
| | | 04_02981 | gi\|387149188\|ref\|NC_017340.1\| | 1775835 R | |
| | | NCTC_8325 | gi\|88193823\|ref\|NC_007795.1\| | 1714993 R | |
| 37 | 2,9677E-145 | 04_02981 | gi\|387149188\|ref\|NC_017340.1\| | 1928346 F | |
| 38 | 1,6935E-144 | CN1 | gi\|537459744\|ref\|NC_022226.1\| | 1388095 R | |
| 39 | 1,7849E-144 | 04_02981 | gi\|387149188\|ref\|NC_017340.1\| | 559072 R | |
| | | NCTC_8325 | gi\|88193823\|ref\|NC_007795.1\| | 504007 R | |
| 40 | 1,8015E-144 | USA300_TCH1516 | gi\|161508266\|ref\|NC_010079.1\| | 2719339 R | |
| | | 04_02981 | gi\|387149188\|ref\|NC_017340.1\| | 2668764 R | |
| 41 | 4,3308E-144 | 04_02981 | gi\|387149188\|ref\|NC_017340.1\| | 1124668 F | |
| | | USA300_TCH1516 | gi\|161508266\|ref\|NC_010079.1\| | 1121585 F | |
| 42 | 5,541E-144 | CN1 | gi\|537459744\|ref\|NC_022226.1\| | 158073 F | |
| | | M1 | gi\|479328021\|ref\|NC_021059.1\| | 193628 F | |
| | | 6850 | gi\|537441500\|ref\|NC_022222.1\| | 138357 F | |
| | | USA300_TCH1516 | gi\|161508266\|ref\|NC_010079.1\| | 196480 F | |
| | | 04_02981 | gi\|387149188\|ref\|NC_017340.1\| | 189192 F | |
| 43 | 6,1938E-144 | 04_02981 | gi\|387149188\|ref\|NC_017340.1\| | 1187805 F | |
| | | 55_2053 | gi\|532358222\|ref\|NC_022113.1\| | 1078815 F | |
| | | USA300_TCH1516 | gi\|161508266\|ref\|NC_010079.1\| | 1182930 F | |

(continued)

| No. | p-value (FDR) | GenomeName | fasta_header | SNPPositioninGenome | gene |
|-----|---------------|------------|--------------|---------------------|------|
| 44 | 1,7019E-143 | USA300_TCH1516 | gi\|161508266\|ref\|NC_010079.1\| | 1376396 R | sbcC |
| | | 6850 | gi\|537441500\|ref\|NC_022222.1\| | 1323236 R | |
| | | CN1 | gi\|537459744\|ref\|NC_022226.1\| | 1315892 R | |
| | | 04_02981 | gi\|387149188\|ref\|NC_017340.1\| | 1379143 R | |
| | | Bmb9393 | gi\|521210823\|ref\|NC_021670.1\| | 1399306 F | |
| 45 | 2,694E-143 | 04_02981 | gi\|387149188\|ref\|NC_017340.1\| | 2398505 R | |
| 46 | 3,3496E-143 | 04_02981 | gi\|387149188\|ref\|NC_017340.1\| | 2753541 F | |
| | | USA300_TCH1516 | gi\|161508266\|ref\|NC_010079.1\| | 2803165 F | |
| 47 | 3,5029E-143 | 04_02981 | gi\|387149188\|ref\|NC_017340.1\| | 1415365 R | |
| | | 08BA02176 | gi\|404477334\|ref\|NC_018608.1\| | 1428821 R | |
| | | NCTC_8325 | gi\|88193823\|ref\|NC_007795.1\| | 1318646 R | femB |
| | | USA300_TCH1516 | gi\|161508266\|ref\|NC_010079.1\| | 1412563 R | |
| | | M1 | gi\|479328021\|ref\|NC_021059.1\| | 1381147 R | |
| 48 | 3,5029E-143 | 04_02981 | gi\|387149188\|ref\|NC_017340.1\| | 1678734 R | |
| | | USA300 TCH1516 | gi\|161508266\|ref\|NC_010079.1\| | 1720315 R | |
| 49 | 8,2809E-143 | NCTC_8325 | gi\|88193823\|ref\|NC_007795.1\| | 854815 R | |
| | | 04_02981 | gi\|387149188\|ref\|NC_017340.1\| | 953539 R | prsA1 |
| | | USA300 TCH1516 | gi\|161508266\|ref\|NC_010079.1\| | 948900 R | |
| 50 | 8,2809E-143 | 04 02981 | gi\|387149188\|ref\|NC_017340.1\| | 1675156 R | |

Table 2b: list of positions (corresponding to Table 1,_continued)

| No. | Amino Acids | Codons | GenomeGI | Protein_GI |
|-----|-------------|--------|----------|------------|
| 1 | F_L | TTA_TTT | 387149188 | 446874184 |
| | F_L | TTA_TTT | 161508266 | 161508745 |
| 2 | A_V | GCA_GTA | 537441500 | 537465126 |
| | A_V | GCA_GTA | 387149188 | 447077358 |
| | A_V | GCA_GTA | 161508266 | 161508491 |
| 3 | I_N | AAT_ATT | 387149188 | 447178207 |

(continued)

| No. | Amino Acids | Codons | GenomeGI | Protein_GI |
|---|---|---|---|---|
| 4 | L_S | TCA_TTA | 521210823 | 521258120 |
|   | L_S | TCA_TTA | 385780298 | 446060496 |
|   | L_S | TCA_TTA | 387149188 | 446060495 |
| 5 | N_S | AAT_AGT | 387149188 | 446940596 |
|   | N_S | AAT_AGT | 161508266 | 161509778 |
| 6 | E_K | AAA_GAA | 387149188 | 446032753 |
| 7 | G_R | AGA_GGA | 49484912 | 487756815 |
|   | G_R | AGA_GGA | 384546269 | 446093782 |
| 8 | K_R | AAA_AGA | 387149188 | 446973880 |
|   | K_R | AAA_AGA | 404477334 | 446973883 |
| 9 | I_M | ATC_ATG | 387149188 | 446312722 |
| 10 | A_S | GCA_TCA | 387149188 | 446180863 |
| 11 | L_P | CCT_CTT | 537441500 | 537465549 |
|   | L_P | CCT_CTT | 161508266 | 161509205 |
| 12 | K_T | AAA_ACA | 387149188 | 446556386 |
|   | K_T | AAA_ACA | 88193823 | 88193961 |
| 13 | M_V | ATG_GTG | 385780298 | 446324804 |
|   | M_V | ATG_GTG | 387149188 | 446324797 |
|   | M_V | ATG_GTG | 404477334 | 446324791 |
| 14 | K_N | AAA_AAC | 387149188 | 445930822 |
| 15 | E_V | GAA_GTA | 387149188 | 446943955 |
| 16 | F_L | TTG_TTT | 88193823 | 88196623 |
|   | F_L | TTG_TTT | 387149188 | 446800117 |
| 17 | Q_R | CAG_CGG | 404477334 | 446795417 |
|   | Q_R | CAG_CGG | 387149188 | 446795407 |
| 18 | F_L | TTA_TTT | 387149188 | 447047252 |
|   | F_L | TTA_TTT | 88193823 | 88194665 |
| 19 | M_V | ATG_GTG | 521210823 | 752533903 |
|   | M_V | ATG_GTG | 387149188 | 446753128 |
| 20 | D_Y | GAT_TAT | 537441500 | 537465893 |
|   | D_Y | GAT_TAT | 161508266 | 161510081 |
|   | D_Y | GAT_TAT | 387149188 | 446862272 |
| 21 | E_K | AAG_GAG | 387149188 | 446725640 |
| 22 | L_S | TCA_TTA | 161508266 | 161510359 |
|   | L_S | TCA_TTA | 387149188 | 446293068 |
| 23 | A_V | GCG_GTG | 387149188 | 446784840 |
| 24 | N_T | AAT_ACT | 387149188 | 446076361 |
|   | N_T | AAT_ACT | 385780298 | 446076373 |
| 25 | L_V | GTA_TTA | 387149188 | 446028277 |
|   | L_V | GTA_TTA | 88193823 | 88195494 |
| 26 | A_T | ACA_GCA | 387149188 | 446792191 |
|   | A_T | ACA_GCA | 161508266 | 161510080 |

(continued)

| No. | Amino Acids | Codons | GenomeGI | Protein_GI |
|---|---|---|---|---|
| 27 | I_T | ACC_ATC | 537441500 | 537465687 |
| | I_T | ACC_ATC | 161508266 | 161509438 |
| | I_T | ACC_ATC | 387149188 | 446786934 |
| 28 | K_T | AAA_ACA | 387149188 | 446403560 |
| | K_T | AAA_ACA | 161508266 | 161510698 |
| 29 | K_T | AAG_ACG | 161508266 | 161509974 |
| | K_T | AAG_ACG | 479328021 | 505394769 |
| | K_T | AAG_ACG | 537441500 | 537465850 |
| | K_T | AAG_ACG | 404477334 | 446973259 |
| | K_T | AAG_ACG | 514064966 | 514074897 |
| | K_T | AAG_ACG | 521210823 | 521258173 |
| 30 | F_L | TTA_TTT | 88193823 | 88194836 |
| | F_L | TTA_TTT | 387149188 | 446593607 |
| 31 | I_V | ATA_GTA | 379013365 | 487720346 |
| | I_V | ATA_GTA | 537441500 | 537465949 |
| | I_V | ATA_GTA | 161508266 | 161510279 |
| 32 | K_T | AAA_ACA | 537441500 | 537465192 |
| | K_T | AAA_ACA | 387149188 | 446129782 |
| | K_T | AAA_ACA | 88193823 | 88194138 |
| 33 | E_K | AAA_GAA | 479328021 | 505394709 |
| | E_K | AAA_GAA | 404477334 | 446089469 |
| | E_K | AAA_GAA | 387149188 | 446089454 |
| | E_K | AAA_GAA | 88193823 | 88194651 |
| 34 | I_T | ACA_ATA | 387149188 | 446104798 |
| | I_T | ACA_ATA | 88193823 | 88194808 |
| 35 | G_V | GGA_GTA | 387149188 | 446343556 |
| 36 | D_E | GAA_GAT | 161508266 | 161509916 |
| | D_E | GAA_GAT | 387149188 | 446149063 |
| | D_E | GAA_GAT | 88193823 | 88195511 |
| 37 | A_G | GCA_GGA | 387149188 | 446506832 |
| 38 | C_Y | TAT_TGT | 537459744 | 686312170 |
| 39 | A_V | GCC_GTC | 387149188 | 446804811 |
| | A_V | GCC_GTC | 88193823 | 88194284 |
| 40 | P_T | ACA_CCA | 161508266 | 161510779 |
| | P_T | ACA_CCA | 387149188 | 446083969 |
| 41 | I_L | ATA_TTA | 387149188 | 446710589 |
| | I_L | ATA_TTA | 161508266 | 161509291 |
| 42 | I_V | ATT_GTT | 537459744 | 537467717 |
| | I_V | ATT_GTT | 479328021 | 505394663 |
| | I_V | ATT_GTT | 537441500 | 537465062 |
| | I_V | ATT_GTT | 161508266 | 161508437 |
| | I_V | ATT_GTT | 387149188 | 446513509 |

(continued)

| No. | Amino Acids | Codons | GenomeGI | Protein_GI |
|---|---|---|---|---|
| 43 | I_T<br>I_T<br>I_T | ACA_ATA<br>ACA_ATA<br>ACA_ATA | 387149188<br>532358222<br>161508266 | 446462960<br>532479591<br>161509351 |
| 44 | I_T<br>I_T<br>I_T<br>I_T<br>I_T | ACT_ATT<br>ACT_ATT<br>ACT_ATT<br>ACT_ATT<br>ACT_ATT | 161508266<br>537441500<br>537459744<br>387149188<br>521210823 | 161509514<br>537465718<br>537467986<br>446725826<br>521258127 |
| 45 | G_S | AGT_GGT | 387149188 | 446921498 |
| 46 | S_Y<br>S_Y | TAT_TCT<br>TAT_TCT | 387149188<br>161508266 | 446080575<br>161510854 |
| 47 | L_S<br>L_S<br>L_S<br>L_S<br>L_S | TCA_TTA<br>TCA_TTA<br>TCA_TTA<br>TCA_TTA<br>TCA_TTA | 387149188<br>404477334<br>88193823<br>161508266<br>479328021 | 446595763<br>446595752<br>88195101<br>161509542<br>505394733 |
| 48 | I_L<br>I_L | ATT_CTT<br>ATT_CTT | 387149188<br>161508266 | 446059917<br>161509840 |
| 49 | A_V<br>A_V<br>A_V | GCA_GTA<br>GCA_GTA<br>GCA_GTA | 88193823<br>387149188<br>161508266 | 88194648<br>445957208<br>161509155 |
| 50 | D_N | AAT_GAT | 387149188 | 446305320 |

In Table 2, respectively Tables 2a and 2b, the annotations obtained by the analysis contain the following information:

- No.: consecutive number
- p-value (FDR): significance value calculated for MRSA-MSSA using Fishers exact test and adjusted by FDR (Benjamini Hochberg method (Benjamini Hochberg, 1995))
- GenomeName: Name of the reference genome used for the annotation
- fasta_header: Header of the reference genome fasta file (including GI and NCBI RefSeq ID)
- SNPPositioninGenome: SNP position in the reference genome (F = forward; R = reverse)
- AminoAcids: Amino acids coded by the codon in which the SNP occurs (only one value for synonymous SNPs, otherwise at least 2), separated by "_"
- Codons: All found codons for the SNP, separated by "_"
- GenomeGI: GI number of the genome sequence
- Protein _GI: GI number of the protein sequence
- gene: gene symbol (if applicable)
- product: Gene product
- protein_id: GenBank accession of the protein

Further, in Table 2, all SNP are non-synonymous (1=yes, 0=no), and the SNPs lie within a coding region (are "OnProtein")

The p-value was calculated using the Fisher exact test based on contingency table with 4 fields: #samples Resistant / wild type; #samples Resistant / mutant; #samples not Resistant / wild type; #samples not Resistant / mutant

The test is based on the distribution of the samples in the 4 fields. Even distribution indicates no significance, while clustering into two fields indicates significance.

The following results were obtained

- A total of 7.101 non-synonymous SNPs associated with the MRSA/MSSA phenotypes (FDR adjusted p-value < $10^{-9}$) was detected.
- The biggest part of these were point mutations (i.e. single base exchanges)
- The highest significance reached was < $3*10^{-163}$.

Example 2: Determination of genetic resistance profile

The same bacteria used in Example 1, i.e. the cohort of 1001 specimens of S. aureus, were used in Example 2. Of those 985 had an assembly, a unique Kiel NGS ID (NGS data and assembly ID, a unique resistance profile (no different resistance profiles with different outcomes, and at least one drug with non-missing resistance value, so that these were further analyzed. The experiments were carried out as in Example 1, except that instead of a determination of Methicillin resistance / susceptibility, resistance / susceptibility was determined for the following antibiotics as described below: Amoxicillin/Clavulanate, Ampicillin, Ampicillin/Sulbactam, Azithromycin, Cefalothin, Cefazolin, Cefepime, Cefotaxime, Cefoxitin, Ceftriaxone, Cefuroxime, Chloramphenicol, Ciprofloxacin, Clindamycin, Daptomycin, Ertapenem, Erythromycin, Fosfomycin, Fusidic acid, Gentamicin, Imipenem, Levofloxacin, Linezolid, Meropenem, Moxifloxacin, Mupirocin, Nitrofurantoin, Norfloxacin, Ofloxacin, Oxacillin, Penicillin G, Piperacillin/Tazobactam, Quinupristin/Dalfopristin, Rifampicin, Teicoplanin, Tetracycline, Tigecycline, Tobramycin, Trimethoprim/Sulfamethoxazole, and Vancomycin.

For testing, standard procedures were used, i.e. VITEK 2 system and AST cards (Biomerieux), Microscan system and AST panels (Beckmann Coulter).

Data analysis was carried out as in Example 1.

For the resistance profiles only drugs with non-missing daga for at least 10% of the samples were kept, so that only 16 drugs remained: Ampicillin, Ampicillin/Sulbactam, Cefepime, Cefotaxime, Cefuroxime, Ciprofloxacin, Clindamycin, Erythromycin, Imipenem, Levofloxacin, Moxifloxacin, Oxacillin, Penicillin G, Piperacillin/Tazobactam, Tetracycline, and Tobramycin.

From the data, first the 50 genes with the best p-value were chosen for the list of mutations as well as the list of correlated antibiotic resistance, representing Tables 3a and 3b.

For correlation, the data was filtered by the following drug class ratio and the annotation product:

$$drug\,class\,ratio = \frac{number\,of\,significant\,drugs\,of\,that\,class}{number\,of\,tested\,drugs\,of\,that\,class}$$

The genes in Table 3a thereby represent the 50 best genes for which a mutation was observed in the genomes of S. aureus, whereas the genes in Table 3b represent the 50 best genes for which a cross-correlation could be observed for the antimicrobial drug, e.g. antibiotic, susceptibility testing. Details for Table 3a are given in Tables 4a-d, and details for Tables 3b in Tables 4e-h. The found reference genomes were as in Example 1.

Table 4a: List of positions (corresponding to Table 3a)

| No. | position | reference genome | genome name | sign_phenos | sign_phenos_class |
|---|---|---|---|---|---|
| 1 | 1958403 R | NC_ 017340.1 | 04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| | 2004910 R | NC_ 010079.1 | USA300_TCH1516 | | |

(continued)

| No. | position | reference genome | genome name | sign_phenos | sign_phenos_class |
|---|---|---|---|---|---|
| 2 | 1641150 R | NC_017340.1 | 04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 3 | 978538 F | NC_017340.1 | 04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 4 | 705667 R | NC_017340.1 | 04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 5 | 1434811 R | NC_017340.1 | 04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 6 | 953696 R | NC_022222.1 | 6850 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
|  | 1010027 R | NC_010079.1 | USA300_TCH1516 |  |  |
| 7 | 2101899 R | NC_021670.1 | Bmb9393 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
|  | 1972149 R | NC_017340.1 | 04_02981 |  |  |
| 8 | 208285 R | NC_017340.1 | 04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
|  | 161011 R | NC_007795.1 | NCTC_8325 |  |  |

(continued)

| No. | position | reference genome | genome name | sign_phenos | sign_phenos_class |
|---|---|---|---|---|---|
| 9 | 2179136 R<br>2149064 R<br>2107689 R | NC_017351.1<br>NC_017340.1<br>NC_018608.1 | 11819_97<br>04_02981<br>08BA02176 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 10 | 2358535 F | NC_017340.1 | 04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 11 | 2023012 R | NC_017340.1 | 04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 12 | 2777211 F<br>2779170 F | NC_007795.1<br>NC_017340.1 | NCTC_8325<br>04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 13 | 1801995 R<br>1790672 R | NC_018608.1<br>NC_017340.1 | 08BA02176<br>04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 14 | 1754561 F<br>1691742 F | NC_017340.1<br>NC_007795.1 | 04_02981<br>NCTC_8325 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 15 | 1362060 F | NC_017340.1 | 04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |

(continued)

| No. | position | reference genome | genome name | sign_phenos | sign_phenos_class |
|---|---|---|---|---|---|
| 16 | 1242653 R<br>1294527 R<br><br>1299554 R | NC_022222.1<br>NC_010079.1<br><br>NC_017340.1 | 6850<br>USA300_TCH1516<br><br>04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 17 | 1252703 R<br>1520285 F<br><br>1523326 F | NC_021670.1<br>NC_017351.1<br><br>NC_017340.1 | Bmb9393<br>11819_97<br><br>04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 18 | 1619285 R<br><br><br>1661238 R | NC_017340.1<br><br><br>NC_010079.1 | 04_02981<br><br><br>USA300_TCH1516 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 19 | 1875550 F<br>2006001 F<br><br>1959494 F | NC_022222.1<br>NC_010079.1<br><br>NC_017340.1 | 6850<br>USA300_TCH1516<br><br>04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 20 | 976788 F<br><br><br><br>878040 F | NC_017340.1<br><br><br><br>NC_007795.1 | 04_02981<br><br><br><br>NCTC_8325 | Ampicillin/Sulbactam;Cefepime; Cefotaxim; Cefuroxim;Ciprofloxacin; Clindamycin; Erythromycin; Imipenem;Levofloxacin; Moxifloxacin;Oxacillin;Penicillin G;Tobramycin | aminoglycoside;<br><br><br>fluoroquinolone; lactam; lincosamide; macrolide |
| 21 | 2590222 R<br><br><br>2637689 R | NC_017340.1<br><br><br>NC_010079.1 | 04_02981<br><br><br>USA300_TCH1516 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 22 | 210528 R<br><br>267448 R<br><br>269814 R | NC_022222.1<br>NC_017340.1<br><br>NC_010079.1 | 6850<br>04_02981<br><br>USA300_TCH1516 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |

(continued)

| No. | position | reference genome | genome name | sign_phenos | sign_phenos_class |
|---|---|---|---|---|---|
| 23 | 1814108 R | NC_017340.1 | 04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 24 | 170059 F | NC_002953.3 | MSSA476 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
|  | 142263 F | NC_017337.1 | ED133 |  |  |
| 25 | 534953 F | NC_017340.1 | 04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
|  | 543821 F | NC_010079.1 | USA300_TCH1516 |  |  |
| 26 | 517571 F | NC_017340.1 | 04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
|  | 554542 F | NC_018608.1 | 08BA02176 |  |  |
| 27 | 531649 R | NC_017340.1 | 04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
|  | 531398 R | NC_017351.1 | 11819_97 |  |  |
| 28 | 1050123 R | NC_007795.1 | NCTC_8325 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
|  | 1147277 R | NC_017340.1 | 04_02981 |  |  |

(continued)

| No. | position | reference genome | genome name | sign_phenos | sign_phenos_class |
|---|---|---|---|---|---|
| 29 | 1881161 R | NC_010079.1 | USA300_TCH1516 | | |
| | 1871101 R | NC_021059.1 | M1 | | |
| | 1759861 R | NC_022222.1 | 6850 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| | 1855493 R | NC_018608.1 | 08BA02176 | | |
| | 1858794 R | NC_021554.1 | CC45 | | |
| | 1964828 R | NC_021670.1 | Bmb9393 | | |
| 30 | 2268723 F | NC_010079.1 | USA300_TCH1516 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| | 2221448 F | NC_017340.1 | 04_02981 | | |
| 31 | 920768 F | NC_021059.1 | M1 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| | 956878 F | NC_018608.1 | 08BA02176 | | |
| | 956978 F | NC_017340.1 | 04_02981 | | |
| | 858255 F | NC_007795.1 | NCTC_8325 | | |
| 32 | 2005634 F | NC_016912.1 | VC40 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| | 2039052 F | NC_022222.1 | 6850 | | |
| | 2187801 F | NC_010079.1 | USA300_TCH1516 | | |
| 33 | 429303 F | NC_017340.1 | 04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 34 | 350202 F | NC_022222.1 | 6850 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| | 402479 F | NC_017340.1 | 04_02981 | | |
| | 352104 F | NC_007795.1 | NCTC_8325 | | |

(continued)

| No. | position | reference genome | genome name | sign_phenos | sign_phenos_class |
|---|---|---|---|---|---|
| 35 | 158073 F | NC_022226.1 | CN1 | | |
| | 193628 F | NC_021059.1 | M1 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| | 138357 F | NC_022222.1 | 6850 | | |
| | 196480 F | NC_010079.1 | USA300_TCH1516 | | |
| | 189192 F | NC_017340.1 | 04_02981 | | |
| 36 | 1121847 R | NC_017340.1 | 04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| | 1024692 R | NC_007795.1 | NCTC_8325 | | |
| 37 | 2719339 R | NC_010079.1 | USA300_TCH1516 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| | 2668764 R | NC_017340.1 | 04_02981 | | |
| 38 | 1388095 R | NC_022226.1 | CN1 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 39 | 1415365 R | NC_017340.1 | 04_02981 | Ampicillin/Sulbactam;Cefepime; Cefotaxim; Cefuroxim; Ciprofloxacin;Clindamycin; | aminoglycoside; fluoroquinolo- |
| | 1428821 R | NC_018608.1 | 08BA02176 | | |
| | 1318646 R | NC_007795.1 | NCTC_8325 | Erythromycin;Imipenem; Levofloxacin; Moxifloxacin; Oxacillin;Penicillin G; Tobramycin | ne;lactam; lincosamide;macrolide |
| | 1412563 R | NC_010079.1 | USA300_TCH1516 | | |
| | 1381147 R | NC_021059.1 | M1 | | |
| 40 | 1678734 R | NC_017340.1 | 04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| | 1720315 R | NC_010079.1 | USA300_TCH1516 | | |

(continued)

| No. | position | reference genome | genome name | sign_phenos | sign_phenos_class |
|---|---|---|---|---|---|
| 41 | 1928346 F | NC_ 017340.1 | 04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 42 | 1376396 R 1323236 R 1315892 R 1379143 R 1399306 F | NC_ 010079.1 NC_ 022222.1 NC_ 022226.1 NC_ 017340.1 NC_ 021670.1 | USA300_TCH1516 6850 CN1 04_02981 Bmb9393 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 43 | 1338943 R | NC_ 017340.1 | 04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 44 | 1124668 F 1121585 F | NC_ 017340.1 NC_ 010079.1 | 04_02981 USA300_TCH1516 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G;Tobramycin | aminoglycoside; fluoroquinolone; lactam; lincosa- mide;macrolide |
| 45 | 559072 R 504007 R | NC_ 017340.1 NC_ 007795.1 | 04_02981 NCTC_8325 | Ampicillin/Sulbactam;Cefepime; Cefotaxim; Cefuroxim; Ciprofloxacin;Clindamycin; Erythromycin;Imipenem; Levofloxacin; Moxifloxacin;Oxacillin;Penicillin G;Tobramycin | aminoglycoside; fluoroquinolone; lactam; lincosa- mide;macrolide |
| 46 | 1675156 R | NC_ 017340.1 | 04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |

(continued)

| No. | position | reference genome | genome name | sign_phenos | sign_phenos_class |
|---|---|---|---|---|---|
| 47 | 1187805 F<br>1078815 F<br>1182930 F | NC_017340.1<br>NC_022113.1<br>NC_010079.1 | 04_02981<br>55_2053<br>USA300_TCH1516 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 48 | 1356138 F | NC_017340.1 | 04_02981 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 49 | 854815 R<br>953539 R<br>948900 R | NC_007795.1<br>NC_017340.1<br>NC_010079.1 | NCTC_8325<br>04_02981<br>USA300_TCH1516 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |
| 50 | 2459738 F<br>2364478 F | NC_017340.1<br>NC_022222.1 | 04_02981<br>6850 | Ampicillin/Sulbactam;Cefepime; Cefotaxim; Cefuroxim; Ciprofloxacin;Clindamycin; Erythromycin;Imipenem; Levofloxacin; Moxifloxacin; Oxacillin;Penicillin G; Tobramycin | aminoglycoside; fluoroquinolone; lactam; lincosamide; macrolide |

Table 4b: List of positions (corresponding to Table 3a, continued)

| No. | best_pv | GenomeName | fasta_header | SNPPositioninGenome | gene |
|---|---|---|---|---|---|
| 1 | 6,0168E-193 | 04_02981<br>USA300_TCH1516 | gi\|387149188\|ref\|NC_017340.1\|<br>gi\|161508266\|ref\|NC_010079.1\| | 1958403 R<br>2004910 R | |
| 2 | 1,4167E-189 | 04_02981 | gi\|387149188\|ref\|NC_017340.1\| | 1641150 R | |
| 3 | 1,4167E-189 | 04_02981 | gi\|387149188\|ref\|NC_017340.1\| | 978538 F | |
| 4 | 1,4167E-189 | 04_02981 | gi\|387149188\|ref\|NC_017340.1\| | 705667 R | |
| 5 | 1,4167E-189 | 04_02981 | gi\|387149188\|ref\|NC_017340.1\| | 1434811 R | |
| 6 | 1,4167E-189 | 6850<br>USA300_TCH1516 | gi\|537441500\|ref\|NC_022222.1\|<br>gi\|161508266\|ref\|NC_010079.1\| | 953696 R<br>1010027 R | <br>rluA1 |

(continued)

| No. | best_pv | GenomeName | fasta_header | SNPPositioninGenome | gene |
|---|---|---|---|---|---|
| 7 | 1,4167E-189 | Bmb9393 | gi\|521210823\|ref\|NC_021670.1\| | 2101899 R | |
| | | 04_02981 | gi\|387149188\|ref\|NC_017340.1\| | 1972149 R | |
| 8 | 1,4167E-189 | 04_02981 | gi\|387149188\|ref\|NC_017340.1\| | 208285 R | argC |
| | | NCTC_8325 | gi\|88193823\|ref\|NC_007795.1\| | 161011 R | argC |
| 9 | 1,4167E-189 | 11819_97 | gi\|385780298\|ref\|NC_017351.1\| | 2179136 R | |
| | | 04_02981 | gi\|387149188\|ref\|NC_017340.1\| | 2149064 R | |
| | | 08BA02176 | gi\|404477334\|ref\|NC_018608.1\| | 2107689 R | |
| 10 | 1,4167E-189 | 04_02981 | gi\|387149188\|ref\|NC_017340.1\| | 2358535 F | |
| 11 | 1,4167E-189 | 04_02981 | gi\|387149188\|ref\|NC_017340.1\| | 2023012 R | |
| 12 | 1,4167E-189 | NCTC_8325 | gi\|88193823\|ref\|NC_007795.1\| | 2777211 F | |
| | | 04_02981 | gi\|387149188\|ref\|NC_017340.1\| | 2779170 F | |
| 13 | 1,4167E-189 | 08BA02176 | gi\|404477334\|ref\|NC_018608.1\| | 1801995 R | |
| | | 04_02981 | gi\|387149188\|ref\|NC_017340.1\| | 1790672 R | |
| 14 | 3,0731E-189 | 04_02981 | gi\|387149188\|ref\|NC_017340.1\| | 1754561 F | |
| | | NCTC_8325 | gi\|88193823\|ref\|NC_007795.1\| | 1691742 F | |
| 15 | 3,1179E-189 | 04_02981 | gi\|387149188\|ref\|NC_017340.1\| | 1362060 F | |
| 16 | 3,7988E-189 | 6850 | gi\|537441500\|ref\|NC_022222.1\| | 1242653 R | |
| | | USA300_TCH1516 | gi\|161508266\|ref\|NC_010079.1\| | 1294527 R | ribC |
| | | 04_02981 | gi\|387149188\|ref\|NC_017340.1\| | 1299554 R | |
| 17 | 3,9683E-189 | Bmb9393 | gi\|521210823\|ref\|NC_021670.1\| | 1252703 R | |
| | | 11819_97 | gi\|385780298\|ref\|NC_017351.1\| | 1520285 F | |
| | | 04_02981 | gi\|387149188\|ref\|NC_017340.1\| | 1523326 F | |
| 18 | 3,9683E-189 | 04_02981 | gi\|387149188\|ref\|NC_017340.1\| | 1619285 R | |
| | | USA300_TCH1516 | gi\|161508266\|ref\|NC_010079.1\| | 1661238 R | |

(continued)

| No. | best_pv | GenomeName | fasta_header | SNPPositioninGenome | gene |
|---|---|---|---|---|---|
| 19 | 3,9683E-189 | 6850 | gi\|537441500\|ref\|NC_022222.1\| | 1875550 F | |
| | | USA300_TCH1516 | gi\|161508266\|ref\|NC_010079.1\| | 2006001 F | bcp |
| | | 04_02981 | gi\|387149188\|ref\|NC_017340.1\| | 1959494 F | |
| 20 | 5,4236E-189 | 04_02981 | gi\|387149188\|ref\|NC_017340.1\| | 976788 F | |
| | | NCTC_8325 | gi\|88193823\|ref\|NC_007795.1\| | 878040 F | |
| 21 | 7,5452E-189 | 04_02981 | gi\|387149188\|ref\|NC_017340.1\| | 2590222 R | |
| | | USA300_TCH1516 | gi\|161508266\|ref\|NC_010079.1\| | 2637689 R | gntP |
| 22 | 9,5271E-189 | 6850 | gi\|537441500\|ref\|NC_022222.1\| | 210528 R | |
| | | 04_02981 | gi\|387149188\|ref\|NC_017340.1\| | 267448 R | |
| | | USA300_TCH1516 | gi\|161508266\|ref\|NC_010079.1\| | 269814 R | |
| 23 | 1,1093E-188 | 04_02981 | gi\|387149188\|ref\|NC_017340.1\| | 1814108 R | |
| 24 | 3,3279E-188 | MSSA476 ED133 | gi\|49484912\|ref\|NC_002953.3\| gi\|384546269\|ref\|NC_017337.1\| | 170059 F 142263 F | |
| 25 | 3,3279E-188 | 04_02981 | gi\|387149188\|ref\|NC_017340.1\| | 534953 F | |
| | | USA300_TCH1516 | gi\|161508266\|ref\|NC_010079.1\| | 543821 F | |
| 26 | 3,5518E-188 | 04_02981 | gi\|387149188\|ref\|NC_017340.1\| | 517571 F | |
| | | 08BA02176 | gi\|404477334\|ref\|NC_018608.1\| | 554542 F | |
| 27 | 1,1492E-187 | 04_02981 | gi\|387149188\|ref\|NC_017340.1\| | 531649 R | |
| | | 11819_97 | gi\|385780298\|ref\|NC_017351.1\| | 531398 R | |
| 28 | 1,1509E-187 | NCTC_8325 04_02981 | gi\|88193823\|ref\|NC_007795.1\| gi\|387149188\|ref\|NC_017340.1\| | 1050123 R 1147277 R | |

(continued)

| No. | best_pv | GenomeName | fasta_header | SNPPositioninGenome | gene |
|---|---|---|---|---|---|
| 29 | 9,0648E-187 | USA300_TCH1516 | gi\|161508266\|ref\|NC_010079.1\| | 1881161 R | fmtB1 |
| | | M1 | gi\|479328021\|ref\|NC_021059.1\| | 1871101 R | |
| | | 6850 | gi\|537441500\|ref\|NC_022222.1\| | 1759861 R | |
| | | 08BA02176 | gi\|404477334\|ref\|NC_018608.1\| | 1855493 R | |
| | | CC45 | gi\|514064966\|ref\|NC_021554.1\| | 1858794 R | |
| | | Bmb9393 | gi\|521210823\|ref\|NC_021670.1\| | 1964828 R | |
| 30 | 1,5807E-186 | USA300_TCH1516 | gi\|161508266\|ref\|NC_010079.1\| | 2268723 F | |
| | | 04_02981 | gi\|387149188\|ref\|NC_017340.1\| | 2221448 F | |
| 31 | 3,6257E-174 | M1 | gi\|479328021\|ref\|NC_021059.1\| | 920768 F | rocD |
| | | 08BA02176 | gi\|404477334\|ref\|NC_018608.1\| | 956878 F | rocD |
| | | 04_02981 | gi\|387149188\|ref\|NC_017340.1\| | 956978 F | rocD |
| | | NCTC_8325 | gi\|88193823\|ref\|NC_007795.1\| | 858255 F | rocD |
| 32 | 3,9753E-174 | VC40 | gi\|379013365\|ref\|NC_016912.1\| | 2005634 F | |
| | | 6850 | gi\|537441500\|ref\|NC_022222.1\| | 2039052 F | |
| | | USA300_TCH1516 | gi\|161508266\|ref\|NC_010079.1\| | 2187801 F | rsbU |
| 33 | 7,1007E-174 | 04_02981 | gi\|387149188\|ref\|NC_017340.1\| | 429303 F | |
| 34 | 1,8906E-173 | 6850 | gi\|537441500\|ref\|NC_022222.1\| | 350202 F | |
| | | 04_02981 | gi\|387149188\|ref\|NC_017340.1\| | 402479 F | |
| | | NCTC_8325 | gi\|88193823\|ref\|NC_007795.1\| | 352104 F | |
| 35 | 3,0713E-172 | CN1 | gi\|537459744\|ref\|NC_022226.1\| | 158073 F | |
| | | M1 | gi\|479328021\|ref\|NC_021059.1\| | 193628 F | |
| | | 6850 | gi\|537441500\|ref\|NC_022222.1\| | 138357 F | |
| | | USA300_TCH1516 | gi\|161508266\|ref\|NC_010079.1\| | 196480 F | |
| | | 04_02981 | gi\|387149188\|ref\|NC_017340.1\| | 189192 F | |
| 36 | 4,4332E-172 | 04_02981 | gi\|387149188\|ref\|NC_017340.1\| | 1121847 R | |
| | | NCTC_8325 | gi\|88193823\|ref\|NC_007795.1\| | 1024692 R | |

(continued)

| No. | best_pv | GenomeName | fasta_header | SNPPositioninGenome | gene |
|---|---|---|---|---|---|
| 37 | 4,4332E-172 | USA300_TCH1516 | gi\|161508266\|ref\|NC_010079.1\| | 2719339 R | |
| | | 04_02981 | gi\|387149188\|ref\|NC_017340.1\| | 2668764 R | |
| 38 | 5,5156E-172 | CN1 | gi\|537459744\|ref\|NC_022226.1\| | 1388095 R | |
| 39 | 1,2003E-171 | 04_02981 | gi\|387149188\|ref\|NC_017340.1\| | 1415365 R | |
| | | 08BA02176 | gi\|404477334\|ref\|NC_018608.1\| | 1428821 R | |
| | | NCTC_8325 | gi\|88193823\|ref\|NC_007795.1\| | 1318646 R | femB |
| | | USA300_TCH1516 | gi\|161508266\|ref\|NC_010079.1\| | 1412563 R | |
| | | M1 | gi\|479328021\|ref\|NC_021059.1\| | 1381147 R | |
| 40 | 1,2003E-171 | 04_02981 | gi\|387149188\|ref\|NC_017340.1\| | 1678734 R | |
| | | USA300_TCH1516 | gi\|161508266\|ref\|NC_010079.1\| | 1720315 R | |
| 41 | 1,2491E-171 | 04_02981 | gi\|387149188\|ref\|NC_017340.1\| | 1928346 F | |
| 42 | 2,3661E-171 | USA300_TCH1516 | gi\|161508266\|ref\|NC_010079.1\| | 1376396 R | sbcC |
| | | 6850 | gi\|537441500\|ref\|NC_022222.1\| | 1323236 R | |
| | | CN1 | gi\|537459744\|ref\|NC_022226.1\| | 1315892 R | |
| | | 04_02981 | gi\|387149188\|ref\|NC_017340.1\| | 1379143 R | |
| | | Bmb9393 | gi\|521210823\|ref\|NC_021670.1\| | 1399306 F | |
| 43 | 2,3661E-171 | 04_02981 | gi\|387149188\|ref\|NC_017340.1\| | 1338943 R | |
| 44 | 5,3408E-171 | 04_02981 | gi\|387149188\|ref\|NC_017340.1\| | 1124668 F | |
| | | USA300_TCH1516 | gi\|161508266\|ref\|NC_010079.1\| | 1121585 F | |
| 45 | 1,0222E-170 | 04_02981 | gi\|387149188\|ref\|NC_017340.1\| | 559072 R | |
| | | NCTC_8325 | gi\|88193823\|ref\|NC_007795.1\| | 504007 R | |
| 46 | 1,0498E-170 | 04_02981 | gi\|387149188\|ref\|NC_017340.1\| | 1675156 R | |
| 47 | 2,283E-170 | 04_02981 | gi\|387149188\|ref\|NC_017340.1\| | 1187805 F | |
| | | 55_2053 | gi\|532358222\|ref\|NC_022113.1\| | 1078815 F | |
| | | USA300_TCH1516 | gi\|161508266\|ref\|NC_010079.1\| | 1182930 F | |

(continued)

| No. | best_pv | GenomeName | fasta_header | SNPPositioninGenome | gene |
|-----|---------|------------|--------------|---------------------|------|
| 48 | 2,3666E-170 | 04_02981 | gi\|387149188\|ref\|NC_017340.1\| | 1356138 F | |
| 49 | 4,1086E-170 | NCTC_8325 04_02981 | gi\|88193823\|ref\|NC_007795.1\| gi\|387149188\|ref\|NC_017340.1\| | 854815 R 953539 R | |
| | | USA300_TCH1516 | gi\|161508266\|ref\|NC_010079.1\| | 948900 R | prsA1 |
| 50 | 4,2262E-169 | 04_02981 | gi\|387149188\|ref\|NC_017340.1\| | 2459738 F | |
| | | 6850 | gi\|537441500\|ref\|NC_022222.1\| | 2364478 F | |

Table 4c: List of positions (corresponding to Table 3a, continued)

| No. | AminoAcids | Codons | GenomeGI | Protein_GI |
|-----|-----------|--------|----------|-----------|
| 1 | A_T A_T | ACA_GCA ACA_GCA | 387149188 161508266 | 446792191 161510080 |
| 2 | E_K | AAA_GAA | 387149188 | 446032753 |
| 3 | I_M | ATC_ATG | 387149188 | 446312722 |
| 4 | E_K | AAG_GAG | 387149188 | 446725640 |
| 5 | A_S | GCA_TCA | 387149188 | 446180863 |
| 6 | L_P L_P | CCT_CTT CCT_CTT | 537441500 161508266 | 537465549 161509205 |
| 7 | M_V M_V | ATG_GTG ATG_GTG | 521210823 387149188 | 752533903 446753128 |
| 8 | K_T K_T | AAA_ACA AAA_ACA | 387149188 88193823 | 446556386 88193961 |
| 9 | M_V M_V M_V | ATG_GTG ATG_GTG ATG_GTG | 385780298 387149188 404477334 | 446324804 446324797 446324791 |
| 10 | K_N | AAA_AAC | 387149188 | 445930822 |
| 11 | E_V | GAA_GTA | 387149188 | 446943955 |
| 12 | F_L F_L | TTG_TTT TTG_TTT | 88193823 387149188 | 88196623 446800117 |
| 13 | Q_R Q_R | CAG_CGG CAG_CGG | 404477334 387149188 | 446795417 446795407 |
| 14 | L_V L_V | GTA_TTA GTA_TTA | 387149188 88193823 | 446028277 88195494 |
| 15 | I_N | AAT_ATT | 387149188 | 447178207 |
| 16 | I_T I_T I_T | ACC_ATC ACC_ATC ACC_ATC | 537441500 161508266 387149188 | 537465687 161509438 446786934 |

(continued)

| No. | AminoAcids | Codons | GenomeGI | Protein_GI |
|---|---|---|---|---|
| 17 | L_S | TCA_TTA | 521210823 | 521258120 |
| | L_S | TCA_TTA | 385780298 | 446060496 |
| | L_S | TCA_TTA | 387149188 | 446060495 |
| 18 | N_S | AAT_AGT | 387149188 | 446940596 |
| | N_S | AAT_AGT | 161508266 | 161509778 |
| 19 | D_Y | GAT_TAT | 537441500 | 537465893 |
| | D_Y | GAT_TAT | 161508266 | 161510081 |
| | D_Y | GAT_TAT | 387149188 | 446862272 |
| 20 | F_L | TTA_TTT | 387149188 | 447047252 |
| | F_L | TTA_TTT | 88193823 | 88194665 |
| 21 | K_T | AAA_ACA | 387149188 | 446403560 |
| | K_T | AAA_ACA | 161508266 | 161510698 |
| 22 | A_V | GCA_GTA | 537441500 | 537465126 |
| | A_V | GCA_GTA | 387149188 | 447077358 |
| | A_V | GCA_GTA | 161508266 | 161508491 |
| 23 | A_V | GCG_GTG | 387149188 | 446784840 |
| 24 | G_R | AGA_GGA | 49484912 | 487756815 |
| | G_R | AGA_GGA | 384546269 | 446093782 |
| 25 | F_L | TTA_TTT | 387149188 | 446874184 |
| | F_L | TTA_TTT | 161508266 | 161508745 |
| 26 | K_R | AAA_AGA | 387149188 | 446973880 |
| | K_R | AAA_AGA | 404477334 | 446973883 |
| 27 | N_T | AAT_ACT | 387149188 | 446076361 |
| | N_T | AAT_ACT | 385780298 | 446076373 |
| 28 | F_L | TTA_TTT | 88193823 | 88194836 |
| | F_L | TTA_TTT | 387149188 | 446593607 |
| 29 | K_T | AAG_ACG | 161508266 | 161509974 |
| | K_T | AAG_ACG | 479328021 | 505394769 |
| | K_T | AAG_ACG | 537441500 | 537465850 |
| | K_T | AAG_ACG | 404477334 | 446973259 |
| | K_T | AAG_ACG | 514064966 | 514074897 |
| | K_T | AAG_ACG | 521210823 | 521258173 |
| 30 | L_S | TCA_TTA | 161508266 | 161510359 |
| | L_S | TCA_TTA | 387149188 | 446293068 |
| 31 | E_K | AAA_GAA | 479328021 | 505394709 |
| | E_K | AAA_GAA | 404477334 | 446089469 |
| | E_K | AAA_GAA | 387149188 | 446089454 |
| | E_K | AAA_GAA | 88193823 | 88194651 |
| 32 | I_V | ATA_GTA | 379013365 | 487720346 |
| | I_V | ATA_GTA | 537441500 | 537465949 |
| | I_V | ATA_GTA | 161508266 | 161510279 |
| 33 | G_V | GGA_GTA | 387149188 | 446343556 |

(continued)

| No. | AminoAcids | Codons | GenomeGI | Protein_GI |
|---|---|---|---|---|
| 34 | K_T | AAA_ACA | 537441500 | 537465192 |
|    | K_T | AAA_ACA | 387149188 | 446129782 |
|    | K_T | AAA_ACA | 88193823 | 88194138 |
| 35 | I_V | ATT_GTT | 537459744 | 537467717 |
|    | I_V | ATT_GTT | 479328021 | 505394663 |
|    | I_V | ATT_GTT | 537441500 | 537465062 |
|    | I_V | ATT_GTT | 161508266 | 161508437 |
|    | I_V | ATT_GTT | 387149188 | 446513509 |
| 36 | I_T | ACA_ATA | 387149188 | 446104798 |
|    | I_T | ACA_ATA | 88193823 | 88194808 |
| 37 | P_T | ACA_CCA | 161508266 | 161510779 |
|    | P_T | ACA_CCA | 387149188 | 446083969 |
| 38 | C_Y | TAT_TGT | 537459744 | 686312170 |
| 39 | L_S | TCA_TTA | 387149188 | 446595763 |
|    | L_S | TCA_TTA | 404477334 | 446595752 |
|    | L_S | TCA_TTA | 88193823 | 88195101 |
|    | L_S | TCA_TTA | 161508266 | 161509542 |
|    | L_S | TCA_TTA | 479328021 | 505394733 |
| 40 | I_L | ATT_CTT | 387149188 | 446059917 |
|    | I_L | ATT_CTT | 161508266 | 161509840 |
| 41 | A_G | GCA_GGA | 387149188 | 446506832 |
| 42 | I_T | ACT_ATT | 161508266 | 161509514 |
|    | I_T | ACT_ATT | 537441500 | 537465718 |
|    | I_T | ACT_ATT | 537459744 | 537467986 |
|    | I_T | ACT_ATT | 387149188 | 446725826 |
|    | I_T | ACT_ATT | 521210823 | 521258127 |
| 43 | C_G | GGT_TGT | 387149188 | 445990753 |
| 44 | I_L | ATA_TTA | 387149188 | 446710589 |
|    | I_L | ATA_TTA | 161508266 | 161509291 |
| 45 | A_V | GCC_GTC | 387149188 | 446804811 |
|    | A_V | GCC_GTC | 88193823 | 88194284 |
| 46 | D_N | AAT_GAT | 387149188 | 446305320 |
| 47 | I_T | ACA_ATA | 387149188 | 446462960 |
|    | I_T | ACA_ATA | 532358222 | 532479591 |
|    | I_T | ACA_ATA | 161508266 | 161509351 |
| 48 | E_Q | CAA_GAA | 387149188 | 446764090 |
| 49 | A_V | GCA_GTA | 88193823 | 88194648 |
|    | A_V | GCA_GTA | 387149188 | 445957208 |
|    | A_V | GCA_GTA | 161508266 | 161509155 |
| 50 | H_Y | CAT_TAT | 387149188 | 446198905 |
|    | H_Y | CAT_TAT | 537441500 | 537466083 |

Table 4d: List of positions (corresponding to Table 3a, continued)

| No. | best_pheno | best_pheno_class | num_aminoglycoside | num_fluoroquinolone | num_lactam | num_lincosamide | num_macrolide | num_tetracycline |
|---|---|---|---|---|---|---|---|---|
| 1 | Moxifloxacin | fluoroquinolone | 1 | 3 | 7 | 1 | 1 | 0 |
| 2 | Moxifloxacin | fluoroquinolone | 1 | 3 | 7 | 1 | 1 | 0 |
| 3 | Moxifloxacin | fluoroquinolone | 1 | 3 | 7 | 1 | 1 | 0 |
| 4 | Moxifloxacin | fluoroquinolone | 1 | 3 | 7 | 1 | 1 | 0 |
| 5 | Moxifloxacin | fluoroquinolone | 1 | 3 | 7 | 1 | 1 | 0 |
| 6 | Moxifloxacin | fluoroquinolone | 1 | 3 | 7 | 1 | 1 | 0 |
| 7 | Moxifloxacin | fluoroquinolone | 1 | 3 | 7 | 1 | 1 | 0 |
| 8 | Moxifloxacin | fluoroquinolone | 1 | 3 | 7 | 1 | 1 | 0 |
| 9 | Moxifloxacin | fluoroquinolone | 1 | 3 | 7 | 1 | 1 | 0 |
| 10 | Moxifloxacin | fluoroquinolone | 1 | 3 | 7 | 1 | 1 | 0 |
| 11 | Moxifloxacin | fluoroquinolone | 1 | 3 | 7 | 1 | 1 | 0 |
| 12 | Moxifloxacin | fluoroquinolone | 1 | 3 | 7 | 1 | 1 | 0 |
| 13 | Moxifloxacin | fluoroquinolone | 1 | 3 | 7 | 1 | 1 | 0 |
| 14 | Moxifloxacin | fluoroquinolone | 1 | 3 | 7 | 1 | 1 | 0 |
| 15 | Moxifloxacin | fluoroquinolone | 1 | 3 | 7 | 1 | 1 | 0 |
| 16 | Moxifloxacin | fluoroquinolone | 1 | 3 | 7 | 1 | 1 | 0 |
| 17 | Moxifloxacin | fluoroquinolone | 1 | 3 | 7 | 1 | 1 | 0 |
| 18 | Moxifloxacin | fluoroquinolone | 1 | 3 | 7 | 1 | 1 | 0 |
| 19 | Moxifloxacin | fluoroquinolone | 1 | 3 | 7 | 1 | 1 | 0 |
| 20 | Moxifloxacin | fluoroquinolone | 1 | 3 | 7 | 1 | 1 | 0 |
| 21 | Moxifloxacin | fluoroquinolone | 1 | 3 | 7 | 1 | 1 | 0 |
| 22 | Moxifloxacin | fluoroquinolone | 1 | 3 | 7 | 1 | 1 | 0 |
| 23 | Moxifloxacin | fluoroquinolone | 1 | 3 | 7 | 1 | 1 | 0 |
| 24 | Moxifloxacin | fluoroquinolone | 1 | 3 | 7 | 1 | 1 | 0 |
| 25 | Moxifloxacin | fluoroquinolone | 1 | 3 | 7 | 1 | 1 | 0 |

| No. | best_pheno | best_pheno_class | num_aminoglycoside | num_fluoroquinolone | num_lactam | num_lincosamide | num_macrolide | num_tetracycline |
|-----|------------|------------------|--------------------|--------------------|-----------|-----------------|---------------|------------------|
| 26 | Moxifloxacin | fluoroquinolone | 1 | 3 | 7 | 1 | 1 | 0 |
| 27 | Moxifloxacin | fluoroquinolone | 1 | 3 | 7 | 1 | 1 | 0 |
| 28 | Moxifloxacin | fluoroquinolone | 1 | 3 | 7 | 1 | 1 | 0 |
| 29 | Moxifloxacin | fluoroquinolone | 1 | 3 | 7 | 1 | 1 | 0 |
| 30 | Moxifloxacin | fluoroquinolone | 1 | 3 | 7 | 1 | 1 | 0 |
| 31 | Moxifloxacin | fluoroquinolone | 1 | 3 | 7 | 1 | 1 | 0 |
| 32 | Moxifloxacin | fluoroquinolone | 1 | 3 | 7 | 1 | 1 | 0 |
| 33 | Moxifloxacin | fluoroquinolone | 1 | 3 | 7 | 1 | 1 | 0 |
| 34 | Moxifloxacin | fluoroquinolone | 1 | 3 | 7 | 1 | 1 | 0 |
| 35 | Moxifloxacin | fluoroquinolone | 1 | 3 | 7 | 1 | 1 | 0 |
| 36 | Moxifloxacin | fluoroquinolone | 1 | 3 | 7 | 1 | 1 | 0 |
| 37 | Moxifloxacin | fluoroquinolone | 1 | 3 | 7 | 1 | 1 | 0 |
| 38 | Moxifloxacin | fluoroquinolone | 1 | 3 | 7 | 1 | 1 | 0 |
| 39 | Moxifloxacin | fluoroquinolone | 1 | 3 | 7 | 1 | 1 | 0 |
| 40 | Moxifloxacin | fluoroquinolone | 1 | 3 | 7 | 1 | 1 | 0 |
| 41 | Moxifloxacin | fluoroquinolone | 1 | 3 | 7 | 1 | 1 | 0 |
| 42 | Moxifloxacin | fluoroquinolone | 1 | 3 | 7 | 1 | 1 | 0 |
| 43 | Moxifloxacin | fluoroquinolone | 1 | 3 | 7 | 1 | 1 | 0 |
| 44 | Moxifloxacin | fluoroquinolone | 1 | 3 | 7 | 1 | 1 | 0 |
| 45 | Moxifloxacin | fluoroquinolone | 1 | 3 | 7 | 1 | 1 | 0 |
| 46 | Moxifloxacin | fluoroquinolone | 1 | 3 | 7 | 1 | 1 | 0 |
| 47 | Moxifloxacin | fluoroquinolone | 1 | 3 | 7 | 1 | 1 | 0 |
| 48 | Moxifloxacin | fluoroquinolone | 1 | 3 | 7 | 1 | 1 | 0 |
| 49 | Moxifloxacin | fluoroquinolone | 1 | 3 | 7 | 1 | 1 | 0 |
| 50 | Moxifloxacin | fluoroquinolone | 1 | 3 | 7 | 1 | 1 | 0 |

Table 4e: List of positions (corresponding to Table 3b)

| No. | position | reference genome | genome name | sign_phenos | sign_phenos_class |
|---|---|---|---|---|---|
| 1 | 1958403 R | NC_ 017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| | 2004910 R | NC_ 010079.1 | USA300_TCH1516 | | |
| 2 | 1641150 R | NC_ 017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| 3 | 978538 F | NC_ 017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| 4 | 705667 R | NC_ 017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| 5 | 1434811 R | NC_ 017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| 6 | 953696 R | NC_ 022222.1 | 6850 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| | 1010027 R | NC_ 010079.1 | USA300_TCH1516 | | |
| 7 | 2101899 R | NC_ 021670.1 | Bmb9393 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| | 1972149 R | NC_ 017340.1 | 04_02981 | | |

(continued)

| No. | position | reference genome | genome name | sign_phenos | sign_phenos_class |
|---|---|---|---|---|---|
| 8 | 208285 R | NC_017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 161011 R | NC_007795.1 | NCTC_8325 |  |  |
| 9 | 2179136 R | NC_017351.1 | 11819_97 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 2149064 R | NC_017340.1 | 04_02981 |  |  |
|  | 2107689 R | NC_018608.1 | 08BA02176 |  |  |
| 10 | 2358535 F | NC_017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| 11 | 2023012 R | NC_017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| 12 | 2777211 F | NC_007795.1 | NCTC_8325 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 2779170 F | NC_017340.1 | 04_02981 |  |  |
| 13 | 1801995 R | NC_018608.1 | 08BA02176 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 1790672 R | NC_017340.1 | 04_02981 |  |  |
| 14 | 1754561 F | NC_017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 1691742 F | NC_007795.1 | NCTC_8325 |  |  |

(continued)

| No. | position | reference genome | genome name | sign_phenos | sign_phenos_class |
|---|---|---|---|---|---|
| 15 | 1362060 F | NC_017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| 16 | 1242653 R | NC022222.1_ NC_010079.1 | 6850 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 1294527 R |  | USA300_TCH1516 |  |  |
|  | 1299554 R | NC_017340.1 | 04_02981 |  |  |
| 17 | 1252703 R | NC_021670.1 | Bmb9393 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 1520285 F | NC_017351.1 | 11819_97 |  |  |
|  | 1523326 F | NC_017340.1 | 04_02981 |  |  |
| 18 | 1619285 R | NC_017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 1661238 R | NC_010079.1 | USA300_TCH1516 |  |  |
| 19 | 1875550 F | NC_022222.1 | 6850 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 2006001 F | NC_010079.1 | USA300_TCH1516 |  |  |
|  | 1959494 F | NC_017340.1 | 04_02981 |  |  |
| 20 | 976788 F | NC_017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 878040 F | NC_007795.1 | NCTC_8325 |  |  |
| 21 | 2590222 R | NC_017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 2637689 R | NC_010079.1 | USA300_TCH1516 |  |  |

(continued)

| No. | position | reference genome | genome name | sign_phenos | sign_phenos_class |
|---|---|---|---|---|---|
| 22 | 210528 R | NC_022222.1 | 6850 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 267448 R | NC_017340.1 | 04_02981 |  |  |
|  | 269814 R | NC_010079.1 | USA300_TCH1516 |  |  |
| 23 | 1814108 R | NC_017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| 24 | 170059 F | NC_002953.3 | MSSA476 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 142263 F | NC_017337.1 | ED133 |  |  |
| 25 | 534953 F | NC_017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 543821 F | NC_010079.1 | USA300_TCH1516 |  |  |
| 26 | 517571 F | NC_017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 554542 F | NC_018608.1 | 08BA02176 |  |  |
| 27 | 531649 R | NC_017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 531398 R | NC_017351.1 | 11819_97 |  |  |
| 28 | 1050123 R | NC_007795.1 | NCTC_8325 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 1147277 R | NC_017340.1 | 04_02981 |  |  |

(continued)

| No. | position | reference genome | genome name | sign_phenos | sign_phenos_class |
|---|---|---|---|---|---|
| 29 | 1881161 R | NC_ 010079.1 | USA300_TCH1516 | | |
| | 1871101 R | NC_ 021059.1 | M1 | | |
| | 1759861 R | NC_ 022222.1 | 6850 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| | 1855493 R | NC_ 018608.1 | 08BA02176 | | |
| | 1858794 R | NC_ 021554.1 | CC45 | | |
| | 1964828 R | NC_ 021670.1 | Bmb9393 | | |
| 30 | 2268723 F | NC_ 010079.1 | USA300_TCH1516 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| | 2221448 F | NC_ 017340.1 | 04_02981 | | |
| 31 | 920768 F | NC_ 021059.1 | M1 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| | 956878 F | NC_ 018608.1 | 08BA02176 | | |
| | 956978 F | NC_ 017340.1 | 04_02981 | | |
| | 858255 F | NC_ 007795.1 | NCTC_8325 | | |
| 32 | 2005634 F | NC_ 016912.1 | VC40 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| | 2039052 F | NC_ 022222.1 | 6850 | | |
| | 2187801 F | NC_ 010079.1 | USA300_TCH1516 | | |
| 33 | 429303 F | NC_ 017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| 34 | 350202 F | NC_ 022222.1 | 6850 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| | 402479 F | NC_ 017340.1 | 04_02981 | | |
| | 352104 F | NC_ 007795.1 | NCTC_8325 | | |

(continued)

| No. | position | reference genome | genome name | sign_phenos | sign_phenos_class |
|---|---|---|---|---|---|
| 35 | 158073 F | NC_022226.1 | CN1 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 193628 F | NC_021059.1 - | M1 |  |  |
|  | 138357 F | NC_022222.1 | 6850 |  |  |
|  | 196480 F | NC_010079.1 | USA300_TCH1516 |  |  |
|  | 189192 F | NC_017340.1 | 04_02981 |  |  |
| 36 | 1121847 R | NC_017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 1024692 R | NC_007795.1 | NCTC_8325 |  |  |
| 37 | 2719339 R | NC_010079.1 | USA300_TCH1516 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 2668764 R | NC_017340.1 | 04_02981 |  |  |
| 38 | 1388095 R | NC_022226.1 | CN1 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| 39 | 1415365 R | NC_017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 1428821 R | NC_018608.1 | 08BA02176 |  |  |
|  | 1318646 R | NC_007795.1 | NCTC_8325 |  |  |
|  | 1412563 R | NC_010079.1 | USA300_TCH1516 |  |  |
|  | 1381147 R | NC_021059.1 | M1 |  |  |
| 40 | 1678734 R | NC_017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
|  | 1720315 R | NC_010079.1 | USA300_TCH1516 |  |  |

(continued)

| No. | position | reference genome | genome name | sign_phenos | sign_phenos_class |
|---|---|---|---|---|---|
| 41 | 1928346 F | NC_ 017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| 42 | 1376396 R 1323236 R 1315892 R 1379143 R 1399306 F | NC_ 010079.1 NC_ 022222.1 NC_ 022226.1 NC_ 017340.1 NC_ 021670.1 | USA300_TCH1516 6850 CN1 04_02981 Bmb9393 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| 43 | 1124668 F 1121585 F | NC_ 017340.1 NC_ 010079.1 | 04_02981 USA300_TCH1516 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| 44 | 559072 R 504007 R | NC_ 017340.1 NC_ 007795.1 | 04_02981 NCTC_8325 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| 45 | 1675156 R | NC_ 017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| 46 | 1187805 F 1078815 F 1182930 F | NC_ 017340.1 NC_ 022113.1 NC_ 010079.1 | 04_02981 55_2053 USA300_TCH1516 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |

(continued)

| No. | position | reference genome | genome name | sign_phenos | sign_phenos_class |
|---|---|---|---|---|---|
| 47 | 1356138 F | NC_ 017340.1 | 04_02981 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| 48 | 854815 R<br>953539 R<br>948900 R | NC_ 007795.1<br>NC_ 017340.1<br>NC_ 010079.1 | NCTC_8325<br>04_02981<br>USA300_TCH1516 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| 49 | 2459738 F<br>2364478 F | NC_ 017340.1<br>NC_ 022222.1 | 04_02981<br>6850 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |
| 50 | 1812380 R<br>1775835 R<br>1714993 R | NC_ 010079.1<br>NC_ 017340.1<br>NC_ 007795.1 | USA300_TCH1516<br>04_02981<br>NCTC_8325 | Ampicillin/Sulbactam; Cefepime;Cefotaxim; Cefuroxim;Ciprofloxacin; Clindamycin; Erythromycin; Imipenem;Levofloxacin; Moxifloxacin;Oxacillin; Penicillin G;Tobramycin | aminoglycoside; fluoroquinolone; lactam; lincosamide; macrolide |

Table 4f: List of positions (corresponding to Table 3b, continued)

| No. | best_pv | GenomeName | fasta_header | SNPPositioninGenome | gene |
|---|---|---|---|---|---|
| 1 | 6,0168E-193 | 04_02981<br>USA300_TCH1516 | gi\|387149188\|ref\|NC_ 017340.1\|<br>gi\|161508266\|ref\|NC_ 010079.1\| | 1958403 R<br>2004910 R | |
| 2 | 1,4167E-189 | 04_02981 | gi\|387149188\|ref\|NC_ 017340.1\| | 1641150 R | |
| 3 | 1,4167E-189 | 04_02981 | gi\|387149188\|ref\|NC_ 017340.1\| | 978538 F | |
| 4 | 1,4167E-189 | 04_02981 | gi\|387149188\|ref\|NC_ 017340.1\| | 705667 R | |
| 5 | 1,4167E-189 | 04_02981 | gi\|387149188\|ref\|NC_ 017340.1\| | 1434811 R | |
| 6 | 1,4167E-189 | 6850<br>USA300_TCH1516 | gi\|537441500\|ref\|NC_ 022222.1\|<br>gi\|161508266\|ref\|NC_ 010079.1\| | 953696 R<br>1010027 R | rluA1 |

(continued)

| No. | best_pv | GenomeName | fasta_header | SNPPositioninGenome | gene |
|---|---|---|---|---|---|
| 7 | 1,4167E-189 | Bmb9393 | gi\|521210823\|ref\|NC_021670.1\| | 2101899 R | |
| | | 04_02981 | gi\|387149188\|ref\|NC_017340.1\| | 1972149 R | |
| 8 | 1,4167E-189 | 04_02981 | gi\|387149188\|ref\|NC_017340.1\| | 208285 R | argC |
| | | NCTC_8325 | gi\|88193823\|ref\|NC_007795.1\| | 161011 R | argC |
| 9 | 1,4167E-189 | 11819_97 | gi\|385780298\|ref\|NC_017351.1\| | 2179136 R | |
| | | 04_02981 | gi\|387149188\|ref\|NC_017340.1\| | 2149064 R | |
| | | 08BA02176 | gi\|404477334\|ref\|NC_018608.1\| | 2107689 R | |
| 10 | 1,4167E-189 | 04_02981 | gi\|387149188\|ref\|NC_017340.1\| | 2358535 F | |
| 11 | 1,4167E-189 | 04_02981 | gi\|387149188\|ref\|NC_017340.1\| | 2023012 R | |
| 12 | 1,4167E-189 | NCTC_8325 | gi\|88193823\|ref\|NC_007795.1\| | 2777211 F | |
| | | 04_02981 | gi\|387149188\|ref\|NC_017340.1\| | 2779170 F | |
| 13 | 1,4167E-189 | 08BA02176 | gi\|404477334\|ref\|NC_018608.1\| | 1801995 R | |
| | | 04_02981 | gi\|387149188\|ref\|NC_017340.1\| | 1790672 R | |
| 14 | 3,0731E-189 | 04_02981 | gi\|387149188\|ref\|NC_017340.1\| | 1754561 F | |
| | | NCTC_8325 | gi\|88193823\|ref\|NC_007795.1\| | 1691742 F | |
| 15 | 3,1179E-189 | 04_02981 | gi\|387149188\|ref\|NC_017340.1\| | 1362060 F | |
| 16 | 3,7988E-189 | 6850 | gi\|537441500\|ref\|NC_022222.1\| | 1242653 R | |
| | | USA300_TCH1516 | gi\|161508266\|ref\|NC_010079.1\| | 1294527 R | ribC |
| | | 04_02981 | gi\|387149188\|ref\|NC_017340.1\| | 1299554 R | |
| 17 | 3,9683E-189 | Bmb9393 | gi\|521210823\|ref\|NC_021670.1\| | 1252703 R | |
| | | 11819_97 | gi\|385780298\|ref\|NC_017351.1\| | 1520285 F | |
| | | 04_02981 | gi\|387149188\|ref\|NC_017340.1\| | 1523326 F | |
| 18 | 3,9683E-189 | 04_02981 | gi\|387149188\|ref\|NC_017340.1\| | 1619285 R | |
| | | USA300_TCH1516 | gi\|161508266\|ref\|NC_010079.1\| | 1661238 R | |

(continued)

| No. | best_pv | GenomeName | fasta_header | SNPPositioninGenome | gene |
|---|---|---|---|---|---|
| 19 | 3,9683E-189 | 6850 | gi\|537441500\|ref\|NC_022222.1\| | 1875550 F | |
| | | USA300_TCH1516 | gi\|161508266\|ref\|NC_010079.1\| | 2006001 F | bcp |
| | | 04_02981 | gi\|387149188\|ref\|NC_017340.1\| | 1959494 F | |
| 20 | 5,4236E-189 | 04_02981 | gi\|387149188\|ref\|NC_017340.1\| | 976788 F | |
| | | NCTC_8325 | gi\|88193823\|ref\|NC_007795.1\| | 878040 F | |
| 21 | 7,5452E-189 | 04_02981 | gi\|387149188\|ref\|NC_017340.1\| | 2590222 R | gntP |
| | | USA300_TCH1516 | gi\|161508266\|ref\|NC_010079.1\| | 2637689 R | |
| 22 | 9,5271E-189 | 6850 | gi\|537441500\|ref\|NC_022222.1\| | 210528 R | |
| | | 04_02981 | gi\|387149188\|ref\|NC_017340.1\| | 267448 R | |
| | | USA300_TCH1516 | gi\|161508266\|ref\|NC_010079.1\| | 269814 R | |
| 23 | 1,1093E-188 | 04_02981 | gi\|387149188\|ref\|NC_017340.1\| | 1814108 R | |
| 24 | 3,3279E-188 | MSSA476 | gi\|49484912\|ref\|NC_002953.3\| | 170059 F | |
| | | ED133 | gi\|384546269\|ref\|NC_017337.1\| | 142263 F | |
| 25 | 3,3279E-188 | 04_02981 | gi\|387149188\|ref\|NC_017340.1\| | 534953 F | |
| | | USA300_TCH1516 | gi\|161508266\|ref\|NC_010079.1\| | 543821 F | |
| 26 | 3,5518E-188 | 04_02981 | gi\|387149188\|ref\|NC_017340.1\| | 517571 F | |
| | | 08BA02176 | gi\|404477334\|ref\|NC_018608.1\| | 554542 F | |
| 27 | 1,1492E-187 | 04_02981 | gi\|387149188\|ref\|NC_017340.1\| | 531649 R | |
| | | 11819_97 | gi\|385780298\|ref\|NC_017351.1\| | 531398 R | |
| 28 | 1,1509E-187 | NCTC_8325 | gi\|88193823\|ref\|NC_007795.1\| | 1050123 R | |
| | | 04_02981 | gi\|387149188\|ref\|NC_017340.1\| | 1147277 R | |

(continued)

| No. | best_pv | GenomeName | fasta_header | SNPPositioninGenome | gene |
|---|---|---|---|---|---|
| 29 | 9,0648E-187 | USA300_TCH1516 | gi\|161508266\|ref\|NC_010079.1\| | 1881161 R | fmtB1 |
| | | M1 | gi\|479328021\|ref\|NC_021059.1\| | 1871101 R | |
| | | 6850 | gi\|537441500\|ref\|NC_022222.1\| | 1759861 R | |
| | | 08BA02176 | gi\|404477334\|ref\|NC_018608.1\| | 1855493 R | |
| | | CC45 | gi\|514064966\|ref\|NC_021554.1\| | 1858794 R | |
| | | Bmb9393 | gi\|521210823\|ref\|NC_021670.1\| | 1964828 R | |
| 30 | 1,5807E-186 | USA300_TCH1516 | gi\|161508266\|ref\|NC_010079.1\| | 2268723 F | |
| | | 04_02981 | gi\|387149188\|ref\|NC_017340.1\| | 2221448 F | |
| 31 | 3,6257E-174 | M1 | gi\|479328021\|ref\|NC_021059.1\| | 920768 F | rocD |
| | | 08BA02176 | gi\|404477334\|ref\|NC_018608.1\| | 956878 F | rocD |
| | | 04_02981 | gi\|387149188\|ref\|NC_017340.1\| | 956978 F | rocD |
| | | NCTC_8325 | gi\|88193823\|ref\|NC_007795.1\| | 858255 F | rocD |
| 32 | 3,9753E-174 | VC40 | gi\|379013365\|ref\|NC_016912.1\| | 2005634 F | |
| | | 6850 | gi\|537441500\|ref\|NC_022222.1\| | 2039052 F | |
| | | USA300_TCH1516 | gi\|161508266\|ref\|NC_010079.1\| | 2187801 F | rsbU |
| 33 | 7,1007E-174 | 04_02981 | gi\|387149188\|ref\|NC_017340.1\| | 429303 F | |
| 34 | 1,8906E-173 | 6850 | gi\|537441500\|ref\|NC_022222.1\| | 350202 F | |
| | | 04_02981 | gi\|387149188\|ref\|NC_017340.1\| | 402479 F | |
| | | NCTC_8325 | gi\|88193823\|ref\|NC_007795.1\| | 352104 F | |
| 35 | 3,0713E-172 | CN1 | gi\|537459744\|ref\|NC_022226.1\| | 158073 F | |
| | | M1 | gi\|479328021\|ref\|NC_021059.1\| | 193628 F | |
| | | 6850 | gi\|537441500\|ref\|NC_022222.1\| | 138357 F | |
| | | USA300_TCH1516 | gi\|161508266\|ref\|NC_010079.1\| | 196480 F | |
| | | 04_02981 | gi\|387149188\|ref\|NC_017340.1\| | 189192 F | |
| 36 | 4,4332E-172 | 04_02981 | gi\|387149188\|ref\|NC_017340.1\| | 1121847 R | |
| | | NCTC_8325 | gi\|88193823\|ref\|NC_007795.1\| | 1024692 R | |

(continued)

| No. | best_pv | GenomeName | fasta_header | SNPPositioninGenome | gene |
|---|---|---|---|---|---|
| 37 | 4,4332E-172 | USA300_TCH1516 | gi\|161508266\|ref\|NC_010079.1\| | 2719339 R | |
| | | 04_02981 | gi\|387149188\|ref\|NC_017340.1\| | 2668764 R | |
| 38 | 5,5156E-172 | CN1 | gi\|537459744\|ref\|NC_022226.1\| | 1388095 R | |
| 39 | 1,2003E-171 | 04_02981 | gi\|387149188\|ref\|NC_017340.1\| | 1415365 R | |
| | | 08BA02176 | gi\|404477334\|ref\|NC_018608.1\| | 1428821 R | |
| | | NCTC_8325 | gi\|88193823\|ref\|NC_007795.1\| | 1318646 R | femB |
| | | USA300_TCH1516 | gi\|161508266\|ref\|NC_010079.1\| | 1412563 R | |
| | | M1 | gi\|479328021\|ref\|NC_021059.1\| | 1381147 R | |
| 40 | 1,2003E-171 | 04_02981 | gi\|387149188\|ref\|NC_017340.1\| | 1678734 R | |
| | | USA300_TCH1516 | gi\|161508266\|ref\|NC_010079.1\| | 1720315 R | |
| 41 | 1,2491E-171 | 04_02981 | gi\|387149188\|ref\|NC_017340.1\| | 1928346 F | |
| 42 | 2,3661E-171 | USA300_TCH1516 | gi\|161508266\|ref\|NC_010079.1\| | 1376396 R | sbcC |
| | | 6850 | gi\|537441500\|ref\|NC_022222.1\| | 1323236 R | |
| | | CN1 | gi\|537459744\|ref\|NC_022226.1\| | 1315892 R | |
| | | 04_02981 | gi\|387149188\|ref\|NC_017340.1\| | 1379143 R | |
| | | Bmb9393 | gi\|521210823\|ref\|NC_021670.1\| | 1399306 F | |
| 43 | 5,3408E-171 | 04_02981 | gi\|387149188\|ref\|NC_017340.1\| | 1124668 F | |
| | | USA300_TCH1516 | gi\|161508266\|ref\|NC_010079.1\| | 1121585 F | |
| 44 | 1,0222E-170 | 04_02981 | gi\|387149188\|ref\|NC_017340.1\| | 559072 R | |
| | | NCTC_8325 | gi\|88193823\|ref\|NC_007795.1\| | 504007 R | |
| 45 | 1,0498E-170 | 04_02981 | gi\|387149188\|ref\|NC_017340.1\| | 1675156 R | |
| 46 | 2,283E-170 | 04_02981 | gi\|387149188\|ref\|NC_017340.1\| | 1187805 F | |
| | | 55_2053 | gi\|532358222\|ref\|NC_022113.1\| | 1078815 F | |
| | | USA300_TCH1516 | gi\|161508266\|ref\|NC_010079.1\| | 1182930 F | |
| 47 | 2,3666E-170 | 04_02981 | gi\|387149188\|ref\|NC_017340.1\| | 1356138 F | |

(continued)

| No. | best_pv | GenomeName | fasta_header | SNPPositioninGenome | gene |
|---|---|---|---|---|---|
| 48 | 4,1086E-170 | NCTC_8325 04_02981 | gi\|88193823\|ref\|NC_007795.1\| gi\|387149188\|ref\|NC_017340.1\| | 854815 R 953539 R | |
| | | USA300_TCH1516 | gi\|161508266\|ref\|NC_010079.1\| | 948900 R | prsA1 |
| 49 | 4,2262E-169 | 04_02981 | gi\|387149188\|ref\|NC_017340.1\| | 2459738 F | |
| | | 6850 | gi\|537441500\|ref\|NC_022222.1\| | 2364478 F | |
| 50 | 1,3444E-168 | USA300_TCH1516 | gi\|161508266\|ref\|NC_010079.1\| | 1812380 R | dnaE2 |
| | | 04_02981 | gi\|387149188\|ref\|NC_017340.1\| | 1775835 R | |
| | | NCTC_8325 | gi\|88193823\|ref\|NC_007795.1\| | 1714993 R | |

Table 4g: List of positions (corresponding to Table 3b, continued)

| No. | AminoAcids | Codons | GenomeGI | Protein_GI |
|---|---|---|---|---|
| 1 | A_T A_T | ACA_GCA ACA_GCA | 387149188 161508266 | 446792191 161510080 |
| 2 | E_K | AAA_GAA | 387149188 | 446032753 |
| 3 | I_M | ATC_ATG | 387149188 | 446312722 |
| 4 | E_K | AAG_GAG | 387149188 | 446725640 |
| 5 | A_S | GCA_TCA | 387149188 | 446180863 |
| 6 | L_P L_P | CCT_CTT CCT_CTT | 537441500 161508266 | 537465549 161509205 |
| 7 | M_V M_V | ATG_GTG ATG_GTG | 521210823 387149188 | 752533903 446753128 |
| 8 | K_T K_T | AAA_ACA AAA_ACA | 387149188 88193823 | 446556386 88193961 |
| 9 | M_V M_V M_V | ATG_GTG ATG_GTG ATG_GTG | 385780298 387149188 404477334 | 446324804 446324797 446324791 |
| 10 | K_N | AAA_AAC | 387149188 | 445930822 |
| 11 | E_V | GAA_GTA | 387149188 | 446943955 |
| 12 | F_L F_L | TTG_TTT TTG_TTT | 88193823 387149188 | 88196623 446800117 |
| 13 | Q_R Q_R | CAG_CGG CAG_CGG | 404477334 387149188 | 446795417 446795407 |
| 14 | L_V L_V | GTA_TTA GTA_TTA | 387149188 88193823 | 446028277 88195494 |
| 15 | I_N | AAT_ATT | 387149188 | 447178207 |

(continued)

| No. | AminoAcids | Codons | GenomeGI | Protein_GI |
|-----|-----------|--------|----------|-----------|
| 16 | I_T | ACC_ATC | 537441500 | 537465687 |
|    | I_T | ACC_ATC | 161508266 | 161509438 |
|    | I_T | ACC_ATC | 387149188 | 446786934 |
| 17 | L_S | TCA_TTA | 521210823 | 521258120 |
|    | L_S | TCA_TTA | 385780298 | 446060496 |
|    | L_S | TCA_TTA | 387149188 | 446060495 |
| 18 | N_S | AAT_AGT | 387149188 | 446940596 |
|    | N_S | AAT_AGT | 161508266 | 161509778 |
| 19 | D_Y | GAT_TAT | 537441500 | 537465893 |
|    | D_Y | GAT_TAT | 161508266 | 161510081 |
|    | D_Y | GAT_TAT | 387149188 | 446862272 |
| 20 | F_L | TTA_TTT | 387149188 | 447047252 |
|    | F_L | TTA_TTT | 88193823 | 88194665 |
| 21 | K_T | AAA_ACA | 387149188 | 446403560 |
|    | K_T | AAA_ACA | 161508266 | 161510698 |
| 22 | A_V | GCA_GTA | 537441500 | 537465126 |
|    | A_V | GCA_GTA | 387149188 | 447077358 |
|    | A_V | GCA_GTA | 161508266 | 161508491 |
| 23 | A_V | GCG_GTG | 387149188 | 446784840 |
| 24 | G_R | AGA_GGA | 49484912 | 487756815 |
|    | G_R | AGA_GGA | 384546269 | 446093782 |
| 25 | F_L | TTA_TTT | 387149188 | 446874184 |
|    | F_L | TTA_TTT | 161508266 | 161508745 |
| 26 | K_R | AAA_AGA | 387149188 | 446973880 |
|    | K_R | AAA_AGA | 404477334 | 446973883 |
| 27 | N_T | AAT_ACT | 387149188 | 446076361 |
|    | N_T | AAT_ACT | 385780298 | 446076373 |
| 28 | F_L | TTA_TTT | 88193823 | 88194836 |
|    | F_L | TTA_TTT | 387149188 | 446593607 |
| 29 | K_T | AAG_ACG | 161508266 | 161509974 |
|    | K_T | AAG_ACG | 479328021 | 505394769 |
|    | K_T | AAG_ACG | 537441500 | 537465850 |
|    | K_T | AAG_ACG | 404477334 | 446973259 |
|    | K_T | AAG_ACG | 514064966 | 514074897 |
|    | K_T | AAG_ACG | 521210823 | 521258173 |
| 30 | L_S | TCA_TTA | 161508266 | 161510359 |
|    | L_S | TCA_TTA | 387149188 | 446293068 |
| 31 | E_K | AAA_GAA | 479328021 | 505394709 |
|    | E_K | AAA_GAA | 404477334 | 446089469 |
|    | E_K | AAA_GAA | 387149188 | 446089454 |
|    | E_K | AAA_GAA | 88193823 | 88194651 |
| 32 | I_V | ATA_GTA | 379013365 | 487720346 |
|    | I_V | ATA_GTA | 537441500 | 537465949 |
|    | I_V | ATA_GTA | 161508266 | 161510279 |

(continued)

| No. | AminoAcids | Codons | GenomeGI | Protein_GI |
|-----|-----------|--------|----------|-----------|
| 33 | G_V | GGA_GTA | 387149188 | 446343556 |
| 34 | K_T | AAA_ACA | 537441500 | 537465192 |
|    | K_T | AAA_ACA | 387149188 | 446129782 |
|    | K_T | AAA_ACA | 88193823 | 88194138 |
| 35 | I_V | ATT_GTT | 537459744 | 537467717 |
|    | I_V | ATT_GTT | 479328021 | 505394663 |
|    | I_V | ATT_GTT | 537441500 | 537465062 |
|    | I_V | ATT_GTT | 161508266 | 161508437 |
|    | I_V | ATT_GTT | 387149188 | 446513509 |
| 36 | I_T | ACA_ATA | 387149188 | 446104798 |
|    | I_T | ACA_ATA | 88193823 | 88194808 |
| 37 | P_T | ACA_CCA | 161508266 | 161510779 |
|    | P_T | ACA_CCA | 387149188 | 446083969 |
| 38 | C_Y | TAT_TGT | 537459744 | 686312170 |
| 39 | L_S | TCA_TTA | 387149188 | 446595763 |
|    | L_S | TCA_TTA | 404477334 | 446595752 |
|    | L_S | TCA_TTA | 88193823 | 88195101 |
|    | L_S | TCA_TTA | 161508266 | 161509542 |
|    | L_S | TCA_TTA | 479328021 | 505394733 |
| 40 | I_L | ATT_CTT | 387149188 | 446059917 |
|    | I_L | ATT_CTT | 161508266 | 161509840 |
| 41 | A_G | GCA_GGA | 387149188 | 446506832 |
| 42 | I_T | ACT_ATT | 161508266 | 161509514 |
|    | I_T | ACT_ATT | 537441500 | 537465718 |
|    | I_T | ACT_ATT | 537459744 | 537467986 |
|    | I_T | ACT_ATT | 387149188 | 446725826 |
|    | I_T | ACT_ATT | 521210823 | 521258127 |
| 43 | I_L | ATA_TTA | 387149188 | 446710589 |
|    | I_L | ATA_TTA | 161508266 | 161509291 |
| 44 | A_V | GCC_GTC | 387149188 | 446804811 |
|    | A_V | GCC_GTC | 88193823 | 88194284 |
| 45 | D_N | AAT_GAT | 387149188 | 446305320 |
| 46 | I_T | ACA_ATA | 387149188 | 446462960 |
|    | I_T | ACA_ATA | 532358222 | 532479591 |
|    | I_T | ACA_ATA | 161508266 | 161509351 |
| 47 | E_Q | CAA_GAA | 387149188 | 446764090 |
| 48 | A_V | GCA_GTA | 88193823 | 88194648 |
|    | A_V | GCA_GTA | 387149188 | 445957208 |
|    | A_V | GCA_GTA | 161508266 | 161509155 |
| 49 | H_Y | CAT_TAT | 387149188 | 446198905 |
|    | H_Y | CAT_TAT | 537441500 | 537466083 |
| 50 | D_E | GAA_GAT | 161508266 | 161509916 |
|    | D_E | GAA_GAT | 387149188 | 446149063 |
|    | D_E | GAA_GAT | 88193823 | 88195511 |

Table 4h: List of positions (corresponding to Table 3b, continued)

| No. | best_pheno | best_pheno_class | num_aminoglycoside | num_fluoroquinolone | num_lactam | num_lincosamide | num_macrolide | num_tetracycline |
|---|---|---|---|---|---|---|---|---|
| 1 | Moxifloxacin | fluoroquinolone | 1 | 3 | 7 | 1 | 1 | 0 |
| 2 | Moxifloxacin | fluoroquinolone | 1 | 3 | 7 | 1 | 1 | 0 |
| 3 | Moxifloxacin | fluoroquinolone | 1 | 3 | 7 | 1 | 1 | 0 |
| 4 | Moxifloxacin | fluoroquinolone | 1 | 3 | 7 | 1 | 1 | 0 |
| 5 | Moxifloxacin | fluoroquinolone | 1 | 3 | 7 | 1 | 1 | 0 |
| 6 | Moxifloxacin | fluoroquinolone | 1 | 3 | 7 | 1 | 1 | 0 |
| 7 | Moxifloxacin | fluoroquinolone | 1 | 3 | 7 | 1 | 1 | 0 |
| 8 | Moxifloxacin | fluoroquinolone | 1 | 3 | 7 | 1 | 1 | 0 |
| 9 | Moxifloxacin | fluoroquinolone | 1 | 3 | 7 | 1 | 1 | 0 |
| 10 | Moxifloxacin | fluoroquinolone | 1 | 3 | 7 | 1 | 1 | 0 |
| 11 | Moxifloxacin | fluoroquinolone | 1 | 3 | 7 | 1 | 1 | 0 |
| 12 | Moxifloxacin | fluoroquinolone | 1 | 3 | 7 | 1 | 1 | 0 |
| 13 | Moxifloxacin | fluoroquinolone | 1 | 3 | 7 | 1 | 1 | 0 |
| 14 | Moxifloxacin | fluoroquinolone | 1 | 3 | 7 | 1 | 1 | 0 |
| 15 | Moxifloxacin | fluoroquinolone | 1 | 3 | 7 | 1 | 1 | 0 |
| 16 | Moxifloxacin | fluoroquinolone | 1 | 3 | 7 | 1 | 1 | 0 |
| 17 | Moxifloxacin | fluoroquinolone | 1 | 3 | 7 | 1 | 1 | 0 |
| 18 | Moxifloxacin | fluoroquinolone | 1 | 3 | 7 | 1 | 1 | 0 |
| 19 | Moxifloxacin | fluoroquinolone | 1 | 3 | 7 | 1 | 1 | 0 |
| 20 | Moxifloxacin | fluoroquinolone | 1 | 3 | 7 | 1 | 1 | 0 |
| 21 | Moxifloxacin | fluoroquinolone | 1 | 3 | 7 | 1 | 1 | 0 |
| 22 | Moxifloxacin | fluoroquinolone | 1 | 3 | 7 | 1 | 1 | 0 |
| 23 | Moxifloxacin | fluoroquinolone | 1 | 3 | 7 | 1 | 1 | 0 |
| 24 | Moxifloxacin | fluoroquinolone | 1 | 3 | 7 | 1 | 1 | 0 |
| 25 | Moxifloxacin | fluoroquinolone | 1 | 3 | 7 | 1 | 1 | 0 |

| No. | best_pheno | best_pheno_class | num_aminoglycoside | num_fluoroquinolone | num_lactam | num_lincosamide | num_macrolide | num_tetracycline |
|---|---|---|---|---|---|---|---|---|
| 26 | Moxifloxacin | fluoroquinolone | 1 | 3 | 7 | 1 | 1 | 0 |
| 27 | Moxifloxacin | fluoroquinolone | 1 | 3 | 7 | 1 | 1 | 0 |
| 28 | Moxifloxacin | fluoroquinolone | 1 | 3 | 7 | 1 | 1 | 0 |
| 29 | Moxifloxacin | fluoroquinolone | 1 | 3 | 7 | 1 | 1 | 0 |
| 30 | Moxifloxacin | fluoroquinolone | 1 | 3 | 7 | 1 | 1 | 0 |
| 31 | Moxifloxacin | fluoroquinolone | 1 | 3 | 7 | 1 | 1 | 0 |
| 32 | Moxifloxacin | fluoroquinolone | 1 | 3 | 7 | 1 | 1 | 0 |
| 33 | Moxifloxacin | fluoroquinolone | 1 | 3 | 7 | 1 | 1 | 0 |
| 34 | Moxifloxacin | fluoroquinolone | 1 | 3 | 7 | 1 | 1 | 0 |
| 35 | Moxifloxacin | fluoroquinolone | 1 | 3 | 7 | 1 | 1 | 0 |
| 36 | Moxifloxacin | fluoroquinolone | 1 | 3 | 7 | 1 | 1 | 0 |
| 37 | Moxifloxacin | fluoroquinolone | 1 | 3 | 7 | 1 | 1 | 0 |
| 38 | Moxifloxacin | fluoroquinolone | 1 | 3 | 7 | 1 | 1 | 0 |
| 39 | Moxifloxacin | fluoroquinolone | 1 | 3 | 7 | 1 | 1 | 0 |
| 40 | Moxifloxacin | fluoroquinolone | 1 | 3 | 7 | 1 | 1 | 0 |
| 41 | Moxifloxacin | fluoroquinolone | 1 | 3 | 7 | 1 | 1 | 0 |
| 42 | Moxifloxacin | fluoroquinolone | 1 | 3 | 7 | 1 | 1 | 0 |
| 43 | Moxifloxacin | fluoroquinolone | 1 | 3 | 7 | 1 | 1 | 0 |
| 44 | Moxifloxacin | fluoroquinolone | 1 | 3 | 7 | 1 | 1 | 0 |
| 45 | Moxifloxacin | fluoroquinolone | 1 | 3 | 7 | 1 | 1 | 0 |
| 46 | Moxifloxacin | fluoroquinolone | 1 | 3 | 7 | 1 | 1 | 0 |
| 47 | Moxifloxacin | fluoroquinolone | 1 | 3 | 7 | 1 | 1 | 0 |
| 48 | Moxifloxacin | fluoroquinolone | 1 | 3 | 7 | 1 | 1 | 0 |
| 49 | Moxifloxacin | fluoroquinolone | 1 | 3 | 7 | 1 | 1 | 0 |
| 50 | Moxifloxacin | fluoroquinolone | 1 | 3 | 7 | 1 | 1 | 0 |

In Tables 4a-h, the annotations are as above in Example 1 for Tables 2a and 2b, with the following extra annotations in the columns:

best_pheno: Phenotype (drug) with smallest adjusted p-value best_pheno_class: drug class of best drug (if phenotypes are drugs)
best_pv: adj. p-value of best phenotype calculated using Fishers exact test and adjusted by FDR (Benjamini Hochberg method (Benjamini Hochberg, 1995))
sign_phenos: names of all phenotypes with significant adj. p-value separated by ";"
sign_phenos_class: drug classes of all significant drugs (if phenotypes are drugs)
Further, in Tables 4a-h, all SNP are again non-synonymous (1=yes, 0=no), and the SNPs lie within a coding region (are "OnProtein")

A genetic test for the combined pathogen identification and antimicrobial susceptibility testing direct from the patient sample can reduce the time-to actionable result significantly from several days to hours, thereby enabling targeted treatment. Furthermore, this approach will not be restricted to central labs, but point of care devices can be developed that allow for respective tests. Such technology along with the present methods and computer program products could revolutionize the care, e.g. in intense care units or for admissions to hospitals in general. Furthermore, even applications like real time outbreak monitoring can be achieved using the present methods.

Instead of using only single variants, a combination of several variant positions can improve the prediction accuracy and further reduce false positive findings that are influenced by other factors.

Compared to approaches using MALDI-TOF MS, the present approach has the advantage that it covers almost the complete genome and thus enables us to identify the potential genomic sites that might be related to resistance. While MALDI-TOF MS can also be used to identify point mutations in bacterial proteins, this technology only detects a subset of proteins and of these not all are equally well covered. In addition, the identification and differentiation of certain related strains is not always feasible.

The present method allows computing a best breakpoint for the separation of isolates into resistant and susceptible groups. The inventors designed a flexible software tool that allows to consider - besides the best breakpoints - also values defined by different guidelines (e.g. European and US guidelines), preparing for an application of the GAST in different countries.

The inventors demonstrate that the present approach is capable of identifying mutations in genes that are already known as drug targets, as well as detecting potential new target sites.

The current approach enables

a. Identification and validation of markers for genetic identification and susceptibility/resistance testing within one diagnostic test
b. Validation of known drug targets and modes of action
c. Detection of potentially novel resistance mechanisms leading to putative novel target / secondary target genes for new therapies

## Claims

1. A computer-implemented method of determining an antimicrobial drug, e.g. antibiotic, resistance profile for a microorganism, particularly a bacterial microorganism, comprising:

   obtaining or providing a first data set of gene sequences of a plurality of clinical isolates of the microorganism; wherein at least a part of the gene sequences of the first data set are assembled;
   analyzing the gene sequences of the first data set for genetic variants to obtain a third data set of genetic variants, wherein the genetic variants in the gene sequences of the first data set are detected alignment-free;
   providing a second data set of antimicrobial drug, e.g. antibiotic, resistance and/or susceptibility of the plurality of clinical isolates of the microorganism;
   correlating the third data set with the second data set and statistically analyzing the correlation; and
   determining the genetic sites in the genome of the microorganism with antimicrobial drug, e.g. antibiotic, resistance.

2. The method of claim 1, wherein the genetic variants in the gene sequences of the first data set are single nucleotide polymorphisms (SNPs).

**3.** The method of any one of the preceding claims, wherein the microorganism is a Staphylococcus species, particularly Staphylococcus aureus, optionally wherein the antimicrobial drug is methicillin.

**4.** A computer-implemented diagnostic method of determining an infection of a patient with a microorganism, particularly a bacterial microorganism potentially resistant to antimicrobial drug treatment, comprising determining the presence of at least one genetic variant in at least one position of at least one genetic sequence of the microorganism, particularly bacterial microorganism, contained or suspected of being contained in a sample obtained from a patient by performing the method of any one of claims 1 to 3, wherein the presence of the at least one genetic variant is indicative of an infection with an antimicrobial drug resistant microorganism in said patient.

**5.** A computer-implemented method of selecting a treatment of a patient suffering from an infection with a potentially resistant microorganism, particularly bacterial microorganism, comprising the steps of:

a) determining the presence of at least one genetic variant in at least one position of at least one genetic sequence of the microorganism, particularly bacterial microorganism, contained or suspected of being contained in a sample obtained from a patient by performing the method of any one of claims 1 to 3, wherein the presence of the at least one genetic variant is indicative of a resistance to one or more antimicrobial drugs;
b) identifying said at least one or more antimicrobial drugs; and
c) selecting one or more antimicrobial drugs different from the ones identified in step b) and being suitable for the treatment of the infection with the microorganism, particularly the bacterial microorganism.

**6.** A computer-implemented method of acquiring, respectively determining, an antimicrobial drug, e.g. antibiotic, resistance profile for a clinical isolate of a microorganism, particularly a bacterial microorganism, comprising:

obtaining or providing at least one gene sequence of the clinical isolate of the microorganism, particularly the bacterial microorganism; and
determining the presence of genetic variants in the at least one gene sequence of the clinical isolate of the microorganism, particularly bacterial microorganism by performing the method of any one of claim 1 to 3.

**7.** The method of claim 2, wherein the SNPs are annotated to a pan-genome of the microorganism and/or annotated to one or more reference genomes.

**8.** Computer-readable storage medium storing program code comprising computer executable instructions which, when executed, perform a method according to any one of claims 1 to 3.

**Patentansprüche**

**1.** Computer-implementiertes Verfahren zur Bestimmung eines Resistenzprofils für einen antimikrobiellen Wirkstoff, z.B. Antibiotikum, für einen Mikroorganismus, insbesondere einen bakteriellen Mikroorganismus, umfassend:

Erhalten oder Bereitstellen eines ersten Datensatzes von Gensequenzen einer Vielzahl von klinischen Isolaten des Mikroorganismus,
wobei mindestens ein Teil der Gensequenzen des ersten Datensatzes assembliert wird,
Analysieren der Gensequenzen des ersten Datensatzes auf genetische Varianten, um einen dritten Datensatz von genetischen Varianten zu erhalten, wobei die genetischen Varianten in den Gensequenzen des ersten Datensatzes alignmentfrei detektiert werden,
Bereitstellen eines zweiten Datensatzes der Resistenz und/oder der Empfindlichkeit für einen antimikrobiellen Wirkstoff, z.B. Antibiotikum, der Vielzahl von klinischen Isolaten des Mikroorganismus,
Korrelieren des dritten Datensatzes mit dem zweiten Datensatz und statistisches Analysieren der Korrelation und Bestimmen der genetischen Stellen in dem Genom des Mikroorganismus mit Resistenz für einen antimikrobiellen Wirkstoff, z.B. Antibiotikum,

**2.** Verfahren nach Anspruch 1, wobei die genetischen Varianten in den Gensequenzen des ersten Datensatzes Einzelnukleotid-Polymorphismen (SNPs) sind.

**3.** Verfahren nach einem der vorhergehenden Ansprüche, wobei der Mikroorganismus eine Staphylococcus-Spezies, insbesondere Staphylococcus aureus, ist, wobei der antimikrobielle Wirkstoff gegebenenfalls Methicillin ist.

**4.** Computer-implementiertes Diagnoseverfahren zur Bestimmung einer Infektion eines Patienten mit einem Mikroorganismus, insbesondere einem bakteriellen Mikroorganismus, der potentiell resistent gegen Behandlung mit einem antimikrobiellen Wirkstoff ist, umfassend die Bestimmung des Vorhandenseins mindestens einer genetischen Variante in mindestens einer Position mindestens einer genetischen Sequenz des Mikroorganismus, insbesondere bakteriellen Mikroorganismus, der in einer von einem Patienten erhaltenen Probe enthalten ist oder vermutet wird, indem das Verfahren nach einem der Ansprüche 1 bis 3 durchgeführt wird, wobei das Vorhandensein der mindestens einen genetischen Variante auf eine Infektion mit einem gegen einen antimikrobiellen Wirkstoff resistenten Mikroorganismus in dem Patienten hinweist.

**5.** Computer-implementiertes Verfahren zur Auswahl einer Behandlung eines Patienten, der an einer Infektion mit einem potentiell resistenten Mikroorganismus, insbesondere einem bakteriellen Mikroorganismus, leidet, umfassend die Schritte:

a) Bestimmen des Vorhandenseins mindestens einer genetischen Variante in mindestens einer Position mindestens einer genetischen Sequenz des Mikroorganismus, insbesondere bakteriellen Mikroorganismus, der in einer von einem Patienten erhaltenen Probe enthalten ist oder vermutet wird, indem das Verfahren nach einem der Ansprüche 1 bis 3 durchgeführt wird, wobei das Vorhandensein der mindestens einen genetischen Variante ein Hinweis auf eine Resistenz gegen einen oder mehrere antimikrobielle Wirkstoffe ist,
b) Identifizieren des mindestens einen oder der mehreren antimikrobiellen Wirkstoffe und
c) Auswählen eines oder mehrerer antimikrobieller Wirkstoffe, die sich von den in Schritt b) identifizierten unterscheiden und für die Behandlung der Infektion mit dem Mikroorganismus, insbesondere dem bakteriellen Mikroorganismus, geeignet sind.

**6.** Computer-implementiertes Verfahren zur Gewinnung bzw. Bestimmung eines Resistenzprofils für einen antimikrobiellen Wirkstoff, z.B. Antibiotikum, für ein klinisches Isolat eines Mikroorganismus, insbesondere eines bakteriellen Mikroorganismus, umfassend: Erhalten oder Bereitstellen mindestens einer Gensequenz des klinischen Isolats des Mikroorganismus, insbesondere des bakteriellen Mikroorganismus, und
Bestimmen des Vorhandenseins von genetischen Varianten in der mindestens einen Gensequenz des klinischen Isolats des Mikroorganismus, insbesondere bakteriellen Mikroorganismus, durch Durchführen des Verfahrens nach einem der Ansprüche 1 bis 3.

**7.** Verfahren nach Anspruch 2, wobei die SNPs mit einem Pan-Genom des Mikroorganismus und/oder mit einem oder mehreren Referenzgenomen annotiert werden.

**8.** Computerlesbares Speichermedium, das Programmcode speichert, der computerausführbare Befehle umfasst, die, wenn sie ausgeführt werden, ein Verfahren nach einem der Ansprüche 1 bis 3 durchführen.

**Revendications**

**1.** Procédé mis en oeuvre par ordinateur de détermination d'un profil de résistance à un médicament antimicrobien, par exemple un antibiotique, d'un microorganisme, en particulier un microorganisme bactérien, comprenant :

l'obtention ou la fourniture d'un premier ensemble de données de séquences géniques d'une pluralité d'isolat cliniques du microorganisme ;
dans lequel au moins une partie des séquences géniques du premier ensemble de données sont assemblées ;
l'analyse des séquences géniques du premier ensemble de données pour rechercher des variants génétiques pour obtenir un troisième ensemble de données de variants génétiques, dans lequel les variants génétiques dans les séquences géniques du premier ensemble de données sont détectés comme étant sans alignement ;
la fourniture d'un deuxième ensemble de données de résistance et/ou susceptibilité à un médicament antimicrobien, par exemple un antibiotique, de la pluralité d'isolats cliniques du microorganisme ;
la corrélation du troisième ensemble de données avec le deuxième ensemble de données et l'analyse statistique de la corrélation ; et
la détermination des sites génétiques dans le génome du microorganisme présentant une résistance à un médicament antimicrobien, par exemple un antibiotique.

**2.** Procédé selon la revendication 1, dans lequel les variants génétiques dans les séquences géniques du premier ensemble de données sont des polymorphismes d'un seul nucléotide (SNP).

3. Procédé selon l'une quelconque des revendications précédentes, dans lequel le microorganisme est une espèce de Staphylococcus, en particulier Staphylococcus aureus, facultativement dans lequel le médicament antimicrobien est la méthicilline.

4. Procédé de diagnostic mis en oeuvre par ordinateur de détermination d'une infection d'un patient avec un microorganisme, en particulier un microorganisme bactérien potentiellement résistant au traitement par un médicament antimicrobien, comprenant la détermination de la présence d'au moins un variant génétique à au moins une position d'au moins une séquence génétique du microorganisme, en particulier un microorganisme bactérien, contenu ou suspecté d'être contenu dans un échantillon obtenu à partir d'un patient en conduisant le procédé selon l'une quelconque des revendications 1 à 3, dans lequel la présence de l'au moins un variant génétique est indicatrice d'une infection par un microorganisme résistant à un médicament antimicrobien chez ledit patient.

5. Procédé mis en oeuvre par ordinateur de sélection d'un traitement d'un patient souffrant d'une infection par un microorganisme potentiellement résistant, en particulier un microorganisme bactérien, comprenant les étapes de :

a) détermination de la présence d'au moins un variant génétique à au moins une position d'au moins une séquence génétique du microorganisme, en particulier un microorganisme bactérien, contenu ou suspecté d'être contenu dans un échantillon obtenu à partir d'un patient en conduisant le procédé selon l'une quelconque des revendications 1 à 3, dans lequel la présence de l'au moins un variant génétique est indicatrice d'une résistance à un ou plusieurs médicaments antimicrobiens ;
b) l'identification desdits au moins un ou plusieurs médicaments antimicrobiens ; et
c) la sélection d'un ou plusieurs médicaments antimicrobiens différents de ceux identifiés dans l'étape b) et étant adaptés pour le traitement de l'infection par le microorganisme, en particulier le microorganisme bactérien.

6. Procédé mis en oeuvre par ordinateur d'acquisition, respectivement de détermination, d'un profil de résistance à un médicament antimicrobien, par exemple un antibiotique, pour un isolat clinique d'un microorganisme, en particulier un microorganisme bactérien, comprenant :

l'obtention ou la fourniture d'au moins une séquence génique de l'isolat clinique du microorganisme, en particulier le microorganisme bactérien ; et
la détermination de la présence de variants génétiques dans l'au moins une séquence génique de l'isolat clinique du microorganisme, en particulier un microorganisme bactérien en conduisant le procédé selon l'une quelconque des revendications 1 à 3.

7. Procédé selon la revendication 2, dans lequel les SNP sont annotés à un pangénome du microorganisme et/ou annotés à un ou plusieurs génomes de référence.

8. Support de stockage lisible par ordinateur stockant un code de programme comprenant des instructions exécutables par ordinateur qui, lorsqu'elles sont exécutées, conduisent un procédé selon l'une quelconque des revendications 1 à 3.

1 2 3 4 5

# Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **WOZNIAK et al.** *BMC Genomics,* 2012, vol. 13 (7), 23
- *J Antimicrob Chemother,* 2013, vol. 68, 2234-2244
- **CHEWAPREECHA et al.** Comprehensive Identification of single nucleotid polymorphisms associated with beta-lactam resistance within pneumococcal mosaic genes. *PLoS Genet,* 2014, vol. 10 (8), e1004547
- **GORDON et al.** Prediction of Staphylococcus aureus Antimicrobial Resistance by Whole-Genome Sequencing. *J. Clin.Microbiol.,* 2014, vol. 52 (4), 1182
- **DORDEL, J. ; KIM, C. et al.** Novel Determinants of Antibiotic Resistance: Identification of Mutated Loci in Highly Methicillin-Resistant Subpopulations of Methicillin-Resistant Staphylococcus aureus. *mBio,* 2014, vol. 5 (2), e01000-13
- **FORTH ; HENSCHLER ; RUMMEL.** Allgemeine und spezielle Pharmakologie und Toxikologie. 2005
- **REMINGTON.** The Science and Practice of Pharmacy. 2013
- *PLoS Pathog.,* 2010, vol. 6 (4), E1000855
- *BMC Microbiol.,* 2007, vol. 7, 99
- *Genome Announc,* 2013, vol. 1 (5
- *Genome Announc,* 2013, vol. 1 (4
- *J. Bacteriol.,* 2012, vol. 194 (6), 1625-1626
- *Proc. Natl. Acad. Sci. U.S.A.,* 2004, vol. 101 (26), 9786-9791
- *Genome Biol Evol,* 2010, vol. 2, 454-466
- *J. Bacteriol.,* 2012, vol. 194 (23), 6627-6628
- GRAM POSITIVE PATHOGENS. ASM Press, 2006
- *J. Bacteriol.,* 2012, vol. 194 (8), 2107-2108
- *PLoS ONE,* 2013, vol. 8 (8), E72803
- **BOLGER AM ; LOHSE M ; USADEL B.** Trimmomatic: a flexible trimmer for Illumina sequence data. *Bioinformatics.,* 2014, vol. 30 (15), 2114-2120
- **BANKEVICH A ; NURK S ; ANTIPOV D et al.** SPAdes: A New Genome Assembly Algorithm and Its Applications to Single-Cell Sequencing. *Journal of Computational Biology.,* 2012, vol. 19 (5), 455-477